(19) Europäisches Patentamt
European Patent Office
Office européen des brevets

(11) EP 1 250 317 B1

(12) # EUROPÄISCHE PATENTSCHRIFT

(45) Veröffentlichungstag und Bekanntmachung des Hinweises auf die Patenterteilung:
**14.09.2005 Patentblatt 2005/37**

(21) Anmeldenummer: **00987242.5**

(22) Anmeldetag: **14.11.2000**

(51) Int Cl.[7]: **C07C 259/18**, C07C 257/18, C07C 217/58, C07C 271/64, C07D 211/26, A61K 31/135, A61K 31/155, A61K 31/325, A61P 37/08

(86) Internationale Anmeldenummer:
**PCT/EP2000/011216**

(87) Internationale Veröffentlichungsnummer:
**WO 2001/036374 (25.05.2001 Gazette 2001/21)**

(54) **BIS-BASISCHE VERBINDUNGEN ALS TRYPTASE-INHIBITOREN, VERFAHREN ZU DEREN HERSTELLUNG SOWIE DEREN VERWENDUNG ALS ARZNEIMITTEL**

BIS-BASIC COMPOUNDS FOR USE AS TRYPTASE INHIBITORS, METHOD FOR PRODUCING THE SAME AND THEIR USE AS MEDICAMENTS

COMPOSES BIS-BASIQUES EN TANT QU'INHIBITEURS DE TRYPTASE, PROCEDE DE FABRICATION, ET UTILISATION EN TANT QUE MEDICAMENT

(84) Benannte Vertragsstaaten:
**AT BE CH CY DE DK ES FI FR GB GR IE IT LI LU MC NL PT SE TR**

(30) Priorität: **18.11.1999 DE 19955476**

(43) Veröffentlichungstag der Anmeldung:
**23.10.2002 Patentblatt 2002/43**

(73) Patentinhaber: **Boehringer Ingelheim Pharma GmbH & Co.KG**
**55218 Ingelheim am Rhein (DE)**

(72) Erfinder:
- **ANDERSKEWITZ, Ralf**
  **55411 Bingen (DE)**
- **BRAUN, Christine**
  **CH-6512 Giubiasco (CH)**
- **HAMM, Rainer**
  **55218 Ingelheim am Rhein (DE)**
- **DISSE, Bernd**
  **55127 Mainz (DE)**
- **JENNEWEIN, Hans, Michael**
  **65193 Wiesbaden (DE)**
- **SPECK, Georg**
  **55218 Ingelheim (DE)**

(56) Entgegenhaltungen:
**WO-A-99/24395**

- **BURGESS L.E. ET AL: 'Potent Selective Nonpeptidic Inhibitors of Human Lung Tryptase' PROC. NATL. ACAD. SCI. USA Bd. 96, 1999, Seiten 8348 - 8352**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft Bis-basische Verbindungen der allgemeinen Formel (IA)

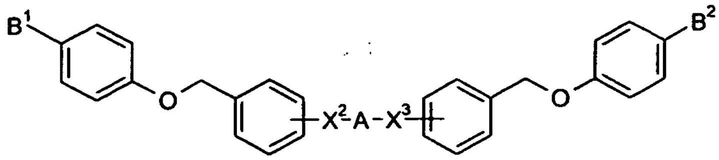

( IA )

in der die Reste $B^1$, $B^2$, $X^2$, $X^3$ und A die in der nachstehenden Beschreibung und in den Ansprüchen genannte Bedeutung haben können, Verfahren zu deren Herstellung und deren Verwendung als Arzneimittel, insbesondere als Arzneimittel mit Tryptase-inhibierender Wirkung.

Tryptase-Inhibitoren können bei der Herstellung von Arzneimitteln Verwendung finden, die zur Vorbeugung und/oder Behandlung entzündlicher und/oder allergischer Erkrankungen dienen. WO 99/24395 offenbart Verbindungen, welche die Tryptaseaktivität inhibieren können. Diese Verbindungen sind geeignet zur Behandlung und Vorbeugung von entzündlichen Erkrankungen insbesondere Erkrankungen, die mit der Aktivierung von Mastzellen zusammenhängen. Burgess et al. (Proc. Natl. Acad. Sci. USA, 96 (1999) 8348 untersuchen die Hypothese, dass symmetrische Tryptaseinhibitoren zwei aktive Zentren am Rezeptor inhibieren können.

Es ist dementsprechend Aufgabe der vorliegenden Erfindung, neue Verbindungen bereitzustellen, die eine Tryptase-inhibierende Wirkung aufweisen und zur Vorbeugung und Behandlung von Erkrankungen, in denen Tryptase-Inhibitoren einen therapeutischen Nutzen entfalten können, Verwendung finden können. Bei den erfindungsgemäßen Verbindungen handelt es sich um Bis-basische Verbindungen der allgemeinen Formel (IA)

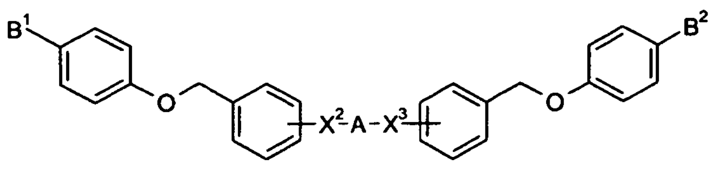

( IA )

worin

$B^1$ und $B^2$ gleich oder verschieden $-C(=NR^1)-NR^{1'}H$, $-CH_2NH_2$, $-CH_2CH_2NH_2$ oder $-NH-C(=NH)-NH_2$;

$R^1$ und $R^{1'}$ gleich oder verschieden Wasserstoff, OH, $-COR^2$oder $-COOR^2$;

$R^2$ Wasserstoff, $C_1$-$C_{18}$-Alkyl, Aryl oder Aryl-$C_1$-$C_6$-Alkyl;

$X^2$ und $X^3$ gleich oder verschieden, eine Brücke ausgewählt aus der Gruppe $-(CH_2)_n-$, $-(CH_2)_nO-$, $-(CH_2)_n-S-$, $-(CH_2)_nNR^3-$ und $-(CH_2)_nN^+R^3R^4-$ mit n=1 oder 2;

A ein Rest ausgewählt aus der Gruppe $C_2$-$C_{16}$-Alkylen und $C_2$-$C_{16}$-Alkenylen, in dem gegebenenfalls ein oder mehrere Wasserstoffatome durch einen oder mehrere Reste ausgewählt aus der Gruppe F, $R^3$, $OR^3$ und $COOR^3$ ersetzt sein können,
$C_2$-$C_{16}$-Alkinylen, oder

A $-(CH_2)_l-D-(CH_2)_m-$, wobei in den Alkylengruppen $-(CH_2)_l-$ und $-(CH_2)_m-$ ein oder zwei Wasserstoffatome gegebenenfalls durch $C_1$-$C_6$-Alkyl ersetzt sein können und wobei

D Aryl oder $C_3$-$C_{10}$-Cycloalkyl, in dem gegebenenfalls ein oder mehrere Wasserstoffatome durch einen oder mehrere Reste ausgewählt aus der Gruppe F, $R^3$ und $OR^3$ ersetzt sein können und l und m, gleich oder verschieden 0, 1, 2, 3 oder 4, oder

D -O-, -S- oder $-NR^3-$ und l und m, gleich oder verschieden 2, 3 oder 4;

oder

A $-G^1-(CH_2)_r-G^2-$, falls $X^2$ oder $X^3$ $-(CH_2)_n-$ bedeuten, auch $-E^1-(CH_2)_r-G^1-$ oder $-E^1-(CH_2)_r-E^2-$, wobei r eine Zahl 0, 1, 2, 3, 4, 5 oder 6,

$G^1$ und $G^2$ gleich oder verschieden eine Einfachbindung oder $C_3$-$C_{10}$-Cycloalkyl bedeuten, im Falle von r=0 oder r=1 $G^1$ und $G^2$ aber nicht gleichzeitig für eine Einfachbindung stehen können;
$E^1$ und $E^2$ gleich oder verschieden $C_3$-$C_{10}$-aza-Cycloalkyl, das ein oder zwei Stickstoffatome enthält, wobei mindestens ein N-Atom an $X^2$ oder $X^3$ = $(CH_2)_n$ gebunden ist,

bedeuten, gegebenenfalls in Form ihrer Racemate, ihrer Enantiomere, ihrer Diastereomere, Tautomere und ihrer Gemische, sowie gegebenenfalls ihrer pharmakologisch unbedenklichen Säureadditionssalze.

Bevorzugt sind Verbindungen der allgemeinen Formel (IA), worin

$B^1$ und $B^2$ gleich oder verschieden $-C(=NR^1)-NR^{1'}H$, $-CH_2NH_2$, $-CH_2CH_2NH_2$ oder $-NH-C(=NH)-NH_2$;

$R^1$ und $R^{1'}$ gleich oder verschieden Wasserstoff, OH, $-COR^2$oder $-COOR^2$;

$R^2$ Wasserstoff, $C_1$-$C_{14}$-Alkyl, Aryl oder Aryl-$C_1$-$C_6$-Alkyl;

$X^2$ und $X^3$ gleich oder verschieden, eine Brücke ausgewählt aus der Gruppe bestehend aus $-(CH_2)_n-$, $-(CH_2)_nO-$, $-(CH_2)_n-S-$, $-(CH_2)_nNR^3-$ und $-(CH_2)_nN^+R^3R^4-$ mit n=1 oder 2;

A ein Rest ausgewählt aus der Gruppe $C_2$-$C_{14}$-Alkylen und $C_2$-$C_{10}$-Alkenylen, in dem gegebenenfalls ein oder mehrere Wasserstoffatome durch einen oder mehrere Reste ausgewählt aus der Gruppe F, $R^3$, $OR^3$ und $COOR^3$ ersetzt sein können, $C_2$-$C_{10}$-Alkinylen, oder

A $-(CH_2)_l-D-(CH_2)_m-$, wobei in den Alkylengruppen $-(CH_2)_l-$ und $-(CH_2)_m-$ ein oder zwei Wasserstoffatome gegebenenfalls durch $C_1$-$C_6$-Alkyl ersetzt sein können und wobei

D Aryl oder $C_3$-$C_8$-Cycloalkyl, in dem gegebenenfalls ein oder mehrere Wasserstoffatome durch einen oder mehrere Reste ausgewählt aus der Gruppe F, $R^3$ und $OR^3$ ersetzt sein können und l und m, gleich oder verschieden 0, 1, 2, 3 oder 4, oder

D -O-, -S- oder $-NR^3-$ und l und m, gleich oder verschieden 2, 3 oder 4;

oder

A $-G^1-(CH_2)_r-G^2-$, falls $X^2$ oder $X^3$ $-(CH_2)_n-$ bedeuten auch $-E^1-(CH_2)_r-G^1-$ oder $-E^1-(CH_2)_r-E^2-$, wobei r eine Zahl 0, 1, 2, 3, 4, 5 oder 6,

$G^1$ und $G^2$ gleich oder verschieden eine Einfachbindung oder $C_3$-$C_8$-Cycloalkyl bedeuten, im Falle von r=0 oder r=1 $G^1$ und $G^2$ aber nicht gleichzeitig für eine Einfachbindung stehen können;
$E^1$ und $E^2$ gleich oder verschieden $C_3$-$C_8$-aza-Cycloalkyl, das ein oder zwei Stickstoffatome enthält, wobei mindestens ein N-Atom an $X^2$ oder $X^3$ = $(CH_2)_n$ gebunden ist,

bedeuten, gegebenenfalls in Form ihrer Racemate, ihrer Enantiomere, ihrer Diastereomere, Tautomere und ihrer Gemische, sowie gegebenenfalls ihrer pharmakologisch unbedenklichen Säureadditionssalze.

Besonders bevorzugt sind Verbindungen der allgemeinen Formel (I), worin

$B^1$ und $B^2$ gleich oder verschieden $-C(=NR^1)-NR^{1'}H$, $-CH_2NH_2$, $-CH_2CH_2NH_2$ oder $-NH-C(=NH)-NH_2$;

$R^1$ und $R^{1'}$ gleich oder verschieden Wasserstoff, OH, $-COR^2$oder $-COOR^2$;

$R^2$ Wasserstoff, $C_1$-$C_{10}$-Alkyl oder Aryl-$C_1$-$C_4$-Alkyl;

$X^2$ und $X^3$ gleich oder verschieden, eine Brücke ausgewählt aus der Gruppe bestehend aus $-(CH_2)_n-$, $-(CH_2)_nO-$, $-(CH_2)_n-S-$, $-(CH_2)_nNR^3-$ und $-(CH_2)_nN^+R^3R^4-$ mit n=1 oder 2, bevorzugt mit n=1;

A $C_2$-$C_{12}$-Alkylen, in dem gegebenenfalls ein oder mehrere Wasserstoffatome durch einen oder mehrere Reste ausgewählt aus der Gruppe F, $OR^3$ und $COOR^3$ ersetzt sein können, oder

A $-(CH_2)_l-D-(CH_2)_m-$, wobei in den Alkylengruppen $-(CH_2)_l-$ und $-(CH_2)_m-$ ein oder zwei Wasserstoffatome gegebenenfalls durch eine $C_1$-$C_4$-Gruppe ersetzt sein können und wobei

D ein Rest ausgewählt aus der Gruppe bestehend aus Phenyl, Cyclopentyl und Cyclohexyl, in dem gegebenenfalls ein oder mehrere Wasserstoffatome durch einen oder mehrere Reste ausgewählt aus der Gruppe F, $R^3$ und $OR^3$ ersetzt sein können
und l und m, gleich oder verschieden 0, 1, 2, 3 oder 4 bedeuten, oder

D -O- oder $-NR^3-$
und l und m, gleich oder verschieden 2, 3 oder 4;

oder

A $-G^1-(CH_2)_r-G^2-$,
falls $X^2$ oder $X^3$ $-(CH_2)_n-$ bedeuten auch
$-E^1-(CH_2)_r-G^1-$ oder $-E^1-(CH_2)_r-E^2-$,
wobei
r eine Zahl 0, 1, 2, 3 oder 4,

$G^1$ und $G^2$ gleich oder verschieden eine Einfachbindung, Cyclopentyl, Cyclohexyl oder Cycloheptyl bedeuten, im Falle von r=0 oder r=1 $G^1$ und $G^2$ aber nicht gleichzeitig für eine Einfachbindung stehen können;

$E^1$ und $E^2$ gleich oder verschieden ein Rest ausgewählt aus der Gruppe bestehend aus Pyrrolidin, Imidazolidin, Piperidin und Piperazin, wobei mindestens ein N-Atom an $X^2$ oder $X^3$ = $(CH_2)_n$ gebunden ist,

bedeuten, gegebenenfalls in Form ihrer Racemate, ihrer Enantiomere, ihrer Diastereomere, Tautomere und ihrer Gemische, sowie gegebenenfalls ihrer pharmakologisch unbedenklichen Säureadditionssalze.

Erfindungsgemäß von besonderer Bedeutung sind Verbindungen der allgemeinen Formel (I), worin

$B^1$ und $B^2$ gleich oder verschieden $-C(=NR^1)-NR^{1'}H$, $-CH_2NH_2$, $-CH_2CH_2NH_2$ oder $-NH-C(=NH)-NH_2$;

$R^1$ und $R^{1'}$ gleich oder verschieden Wasserstoff, OH, $-COR^2$oder $-COOR^2$, bevorzugt Wasserstoff oder OH,

$R^2$ Wasserstoff, $C_1$-$C_8$-Alkyl oder Benzyl;

$X^2$ und $X^3$ gleich oder verschieden, eine Brücke ausgewählt aus der Gruppe bestehend aus $-(CH_2)_n-$, $-(CH_2)_nO-$, $-(CH_2)_n-S-$, $-(CH_2)_nNR^3-$ und $-(CH_2)_nN^+R^3R^4-$ mit n=1 oder 2, bevorzugt mit n=1; oder

A $C_2$-$C_{12}$-Alkylen, in dem gegebenenfalls ein oder mehrere Wasserstoffatome durch einen oder mehrere

Reste ausgewählt aus der Gruppe F, $OR^3$ und $COOR^3$ ersetzt sein können, oder

A $-(CH_2)_l-D-(CH_2)_m-$, wobei in den Alkylengruppen $-(CH_2)_l-$ und $-(CH_2)_m-$ ein oder zwei Wasserstoffatome gegebenenfalls durch eine Methyl-Gruppe ersetzt sein können und wobei

D ein Rest ausgewählt aus der Gruppe bestehend aus Phenyl und Cyclohexyl, in dem gegebenenfalls ein oder mehrere Wasserstoffatome durch einen oder mehrere Reste ausgewählt aus der Gruppe F, $R^3$ und $OR^3$ ersetzt sein können
und l und m, gleich oder verschieden 0, 1, 2 oder 3 bedeuten,
oder

D für -O- und l und m, gleich oder verschieden für 2 oder 3 stehen;

oder

A $-G^1-(CH_2)_r-G^2-$,
falls $X^2$ oder $X^3$ $-(CH_2)_n-$ bedeuten auch
$-E^1-(CH_2)_r-G^1-$ oder $-E^1-(CH_2)_r-E^2-$,
wobei
r eine Zahl 0, 1, 2 oder 3,

$G^1$ und $G^2$ gleich oder verschieden eine Einfachbindung, Cyclopentyl oder Cyclohexyl bedeuten, im Falle von r=0 oder r=1 $G^1$ und $G^2$ aber nicht gleichzeitig für eine Einfachbindung stehen können;

$E^1$ und $E^2$ gleich oder verschieden Piperidin oder Piperazin, wobei mindestens ein N-Atom an $X^2$ oder $X^3 = (CH_2)_n$ gebunden ist,

bedeuten, gegebenenfalls in Form ihrer Racemate, ihrer Enantiomere, ihrer Diastereomere, Tautomere und ihrer Gemische, sowie gegebenenfalls ihrer pharmakologisch unbedenklichen Säureadditionssalze.

Erfindungsgemäß bedeutsam sind ferner Verbindungen der allgemeinen Formel (I), worin

$B^1$ und $B^2$ gleich oder verschieden $-C(=NR^1)-NH_2$, $-CH_2NH_2$ oder $-CH_2CH_2NH_2$;

$R^1$ Wasserstoff, OH, $-COR^2$ oder $-COOR^2$, bevorzugt Wasserstoff oder OH,

$R^2$ Wasserstoff, Methyl, Ethyl, Propyl, Butyl, Pentyl, Hexyl oder Benzyl;

$X^2$ und $X^3$ gleich oder verschieden, eine Brücke ausgewählt aus der Gruppe bestehend aus $-(CH_2)_n-$, $-(CH_2)_nO-$, $-(CH_2)_nNH-$, $-(CH_2)_nNMe-$, $-(CH_2)_nNEt-$, $-(CH_2)_nNProp-$, $-(CH_2)_nNCyclopropy-$, $-(CH_2)_nNBu-$ und $-(CH_2)_nN^+(Me)_2-$ mit n=1;

A $C_2$-$C_{12}$-Alkylen, das gegebenenfalls durch einen Rest ausgewählt aus der Gruppe OH, COOH und COOMe substituiert sein kann, oder
$-(CH_2)_l-D-(CH_2)_m-$, wobei in den Alkylengruppen $-(CH_2)_l-$ und $-(CH_2)_m-$ ein oder zwei Wasserstoffatome gegebenenfalls durch Methyl ersetzt sein können und wobei

D Phenyl oder Cyclohexyl, das gegebenenfalls durch Methyl substituiert sein kann und l und m, gleich oder verschieden 0, 1, 2 oder 3 bedeuten, oder

D für -O- und l und m, gleich oder verschieden für 2 oder 3 stehen;

oder

A $-G^1-(CH_2)_r-G^2-$,
falls $X^2$ oder $X^3$ $-(CH_2)_n-$ bedeuten auch
$-E^1-(CH_2)_r-G^1-$ oder $-E^1-(CH_2)_r-E^2-$,
wobei
r eine Zahl 0, 1, 2 oder 3,

$G^1$ und $G^2$ gleich oder verschieden eine Einfachbindung oder Cyclohexyl bedeuten, im Falle von r=0

oder r=1 $G^1$ und $G^2$ aber nicht gleichzeitig für eine Einfachbindung stehen können;

$E^1$ und $E^2$ gleich oder verschieden Piperidin oder Piperazin, wobei mindestens ein N-Atom an $X^2$ oder $X^3 = (CH_2)_n$ gebunden ist,

bedeuten, gegebenenfalls in Form ihrer Racemate, ihrer Enantiomere, ihrer Diastereomere, Tautomere und ihrer Gemische, sowie gegebenenfalls ihrer pharmakologisch unbedenklichen Säureadditionssalze.

Insbesondere bevorzugt sind die Verbindungen der allgemeinen Formel (IA1)

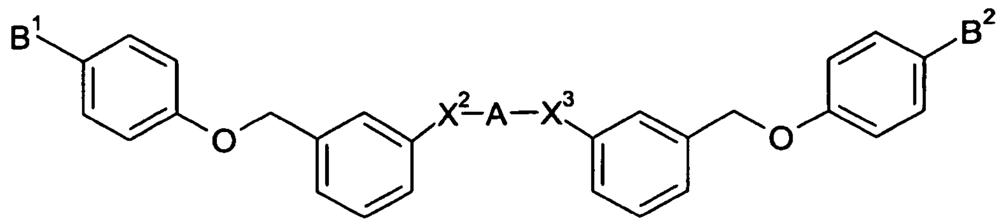

( IA1 )

worin $B^1$, $B^2$, A, $X^2$ und $X^3$ die vor- und nachstehend angegebenen Bedeutungen aufweisen, gegebenenfalls in Form ihrer Racemate, ihrer Enantiomere, ihrer Diastereomere, Tautomere und ihrer Gemische, sowie gegebenenfalls ihrer pharmakologisch unbedenklichen Säureadditionssalze.

Besonders bevorzugt sind die Verbindungen der allgemeinen Formel (IA) oder (IA1), worin $-X^2-A-X^3-$ eine Gruppe der Formel

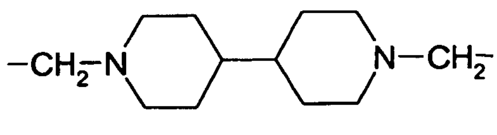

bedeutet.

Verbindungen der allgemeinen Formel (I) in denen $B^1$ und $B^2$ gleich oder verschieden $-C(=NR^1)-NR^{1'}H$ bedeuten, sind für den Fall daß weder $R^1$ noch $R^{1'}$ Wasserstoff bedeuten, sogenannte Prodrugs. Diese können aufgrund einer in vivo abspaltbaren Funktionalität nach ihrer Einnahme durch den Patienten vom Organismus in die therapeutisch wirksamen Verbindungen der allgemeinen Formel (I), in denen $B^1$ und $B^2$ gleich oder verschieden $-C(=NH)NH_2$ beudeten, umgewandelt werden.

Als Alkylgruppen (auch soweit sie Bestandteil anderer Reste sind) werden soweit nicht anders definiert verzweigte und unverzweigte Alkylgruppen mit 1 bis 18 Kohlenstoffatomen bevorzugt 1 - 14 Kohlenstoffatomen, besonders bevorzugt 1 - 10 Kohlenstoffatomen betrachtet. Beispielsweise werden genannt: Methyl, Ethyl, Propyl, Butyl, Pentyl, Hexyl, Heptyl, Octyl etc. Sofern nicht anders genannt, sind von den vorstehend genannten Bezeichnungen Propyl, Butyl, Pentyl, Hexyl, Heptyl, Octyl etc. sämtliche der möglichen isomeren Formen umfaßt. Beispielsweise umfaßt die Bezeichnung Propyl die beiden isomeren Reste n-Propyl und iso-Propyl, die Bezeichnung Butyl n-Butyl, iso-Butyl, sec. Butyl und tert.-Butyl, die Bezeichnung Pentyl, iso-Pentyl, Neopentyl etc. Gegebenfalls werden zur Bezeichnung der vorstehend genannten Alkylreste auch gängige Abkürzungen wie Me für Methyl, Et für Ethyl etc. verwendet.

Als Alkylengruppen werden verzweigte und unverzweigte Alkylenbrücken mit 1 bis 18 Kohlenstoffatomen bevorzugt 1 - 14 Kohlenstoffatomen, besonders bevorzugt 1 - 10 Kohlenstoffatomen betrachtet. Beispielsweise werden genannt: Methylen, Ethylen, Propylen, Butylen etc. Sofern nicht anders genannt, sind von den vorstehend genannten Bezeichnungen Propylen, Butylen etc. sämtliche der möglichen isomeren Formen umfaßt. Beispielsweise umfaßt die Bezeichnung Propylen die beiden isomeren Brücken n-Propylen und Dimethylmethylen, die Bezeichnung Butylen n-Butylen, 1-Methylpropylen, 2-Methylpropylen, 1.1-Dimethylethylen, 1.2-Dimethylethylen etc.

Als Alkenylgruppen (auch soweit sie Bestandteil anderer Reste sind) werden verzweigte und unverzweigte Alkenylgruppen mit 2 bis 16 Kohlenstoffatomen, bevorzugt 2 bis 10 Kohlenstoffatomen, besonders bevorzugt 2 bis 6 Kohlenstoffatomen, genannt, soweit sie mindestens eine Doppelbindung aufweisen, beispielsweise auch oben genannte Alkylgruppen bezeichnet, soweit sie mindestens eine Doppelbindung aufweisen, wie zum Beispiel Vinyl (soweit keine unbeständigen Enamine oder Enolether gebildet werden), Propenyl, iso-Propenyl, Butenyl, Pentenyl, Hexenyl.

Als Alkinylgruppen (auch soweit sie Bestandteil anderer Reste sind) werden verzweigte und unverzweigte Alkenylgruppen mit 2 bis 16 Kohlenstoffatomen, bevorzugt 2 bis 10 Kohlenstoffatomen, besonders bevorzugt 2 bis 6 Kohlenstoffatomen, genannt, soweit sie mindestens eine Dreifachbindung aufweisen, beispielsweise Ethinyl, Propargyl, Butinyl, Pentinyl, Hexinyl.

In den vorstehend genannten Alkylgruppen, Alkylengruppen und Alkenylgruppen können gegebenenfalls ein oder mehrere Wasserstoffatome durch die in den Definitionen angegebenen Reste substituiert sein. Unter mehreren substituierten Wasserstoffatomen ist die Substitution von mindestens 2 Wasserstoffatomen zu verstehen. Im Falle des Substituenten Fluor können gegebenenfalls auch alle Wasserstoffatome der Alkyl-, Alkylen- und Alkenylgruppen ersetzt sein.

Als Cycloalkylreste mit 3 - 10 Kohlenstoffatomen werden beispielsweise Cyclopropyl, Cyclobutyl, Cyclopentyl, Cyclohexyl, Cycloheptyl, Cyclooctyl, Cyclononyl, Cyclodecyl bezeichnet, die auch durch verzweigtes oder unverzweigtes Alkyl mit 1 bis 4 Kohlenstoffatomen, Hydroxy, und/oder Halogen oder wie zuvor definiert substituiert sein können.

In den vorstehend genannten Cycloalkylresten können gegebenenfalls ein oder mehrere Wasserstoffatome durch die in den Definitionen angegebenen Reste substituiert sein. Unter mehreren substituierten Wasserstoffatomen ist die Substitution von mindestens 2 Wasserstoffatomen zu verstehen. Im Falle des Substituenten Fluor können gegebenenfalls auch alle Wasserstoffatome der Cycloalkylreste ersetzt sein.

Als Halogen wird im allgemeinen Fluor, Chlor, Brom oder Jod bezeichnet.

Als $C_3$-$C_{10}$-aza-Cycloalkylreste werden 3- bis 10-gliedrige Cycloalkylreste bezeichnet, in denen ein oder zwei Stickstoffatome enthalten sind. Beispielsweise werden genannt Pyrrolidin, Imidazolidin, Piperidin, Piperazin, Azepan, Diazepane etc., die jeweils, falls nicht anders definiert, auch durch verzweigtes oder unverzweigtes Alkyl mit 1 bis 4 Kohlenstoffatomen, Hydroxy, und/oder Halogen oder wie zuvor definiert substituiert sein können.

Als 5-10-gliedrige mono- oder bicyclische Heteroarylringe in denen bis zu drei C-Atome durch ein oder mehrere Hetaroatome ausgewählt aus der Gruppe Sauerstoff, Stickstoff oder Schwefel ersetzt sein können werden beispielsweise Furan, Thiophen, Pyrrol, Pyrazol, Imidazol, Triazol, Pyridin, Pyridazin, Pyrimidin, Pyrazin, Triazin, Oxazol, Isoxazol, Thiazol, Thiadiazol, Oxadiazol genannt, wobei jeder der vorstehehend genannten Heterocyclen gegebenenfalls ferner an einen Benzolring ankondensiert sein kann und wobei diese Heterocyclen wie in den Definitionen angegeben substituiert sein können.

Der Begriff $C_6$-$C_{10}$-Aryl steht für ein aromatisches Ringsystem mit 6 bis 10 Kohlenstoffatomen, das, soweit nicht anders beschrieben, beispielsweise einen oder mehrere der nachfolgend genannten Substituenten tragen kann: $C_1$-$C_6$-Alkyl, $C_1$-$C_6$-Alkyloxy, Halogen, Hydroxy, Mercapto, Amino, Alkylamino, Dialkylamino, $CF_3$, Cyano, Nitro, -CHO, -COOH, -COO-$C_1$-$C_6$-Alkyl, -S-$C_1$-$C_6$-Alkyl. Bevorzugter Arylrest ist Phenyl.

"=O" bedeutet ein über eine Doppelbindung verknüpftes Sauerstoffatom.

In den vorstehehend genannten Definitionen sind, soweit nicht anders definiert, alle der für die Reste -$Ar^1$-, -$Ar^2$-, -$Ar^3$- und -$Ar^4$- angegebenen Definitionen als zweibindige Gruppen anzusehen, die in drei denkbaren Substitutionsmustern (ortho, meta und para) mit den beiden benachbarten Funktionen verknüpft sein können. Bevorzugt sind die meta- und para-Substitution.

In den vorstehehend genannten Definitionen sind, soweit nicht anders definiert, alle der für die Reste -$X^1$-, -$X^2$-, -A-, -$X^3$- und -$X^4$- angegebenen Definitionen als zweibindige Gruppen anzusehen, die in beiden denkbaren Ausrichtungen mit den beiden benachbarten Funktionen verknüpft sein können. Bevorzugt bedeuten:

-$X^1$- -O-$(CH_2)_n$-, -S-$(CH_2)_n$- oder -$NR^3$-$(CH_2)_n$- insbesondere -O-$CH_2$-; und
-$X^4$- -$(CH_2)_n$-O-, -$(CH_2)_n$-S- oder -$(CH_2)$-$NR^3$-$_n$, insbesondere -$CH_2$-O-.

Ein weiterer Aspekt der vorliegenden Erfindung zielt auf die Verwendung der vorstehend definierten Verbindungen der allgemeinen Formel (I) als Arzneimittel. Insbesondere zielt die vorliegende Erfindung auf die Verwendung der Verbindungen der allgemeinen Formel (I) zur Herstellung eines Arzneimittels zur Vorbeugung und/oder Behandlung von Erkrankungen, in denen Tryptase-Inhibitoren einen therapeutischen Nutzen entfalten können.

Erfindungsgemäß bevorzugt ist die vorstehend genannte Verwendung von Verbindungen der allgemeinen Formel (I) zur Herstellung eines Arzneimittels zur Vorbeugung und/oder Behandlung entzündlicher und/oder allergischer Erkrankungen.

Besonders bevorzugt ist die vorstehend genannte Verwendung der Verbindungen der allgemeinen Formel (I) zur Herstellung eines Arzneimittels zur Vorbeugung und/oder Behandlung von Asthma bronchiale, allergischer Rhinitis, allergischer Conjunctivitis, atopischer Dermatitis, Urticaria, allergischer Otitis, allergischer Magen-Darmerkrankungen, Morbus Crohn, Colitis ulcerosa, anaphylaktischer Schock, septischer Schock, Schocklunge (ARDS) und Arthritis.

Femer ist von Interesse die vorstehend genannte Verwendung der Verbindungen der allgemeinen Formel (I) zur Herstellung eines Arzneimittels zur Vorbeugung und/oder Behandlung von Fibrosen wie Lungenfibrose, fibrosierende Alveolitis und Narbenbildung, von Kollagneosen wie Lupus erythematodes und Sklerodermie sowie von Arteriosklerose, Psoriasis und Neoplasien.

Ein synthetischer Zugang zu den erfindungsgemäßen Verbindungen der allgemeinen Formel (IA) kann unter Anwendung unterschiedlicher Methoden, gegebenenfalls in Anlehnung an oder unter Verwendung von konventionellen chemischen Synthesemethoden wie nachfolgend detaillierter beschrieben erfolgen. Im Folgenden bedeuten $Ar^1$, $Ar^2$, $Ar^3$ und $Ar^4$ jeweils Phenyl, $X^1$- bedeutet $-O-CH_2-$ und $-X^4-$ bedeutet $-CH_2-O-$.

**Methode A**

Zur Herstellung von Verbindungen der allgemeinen Formel (IA)

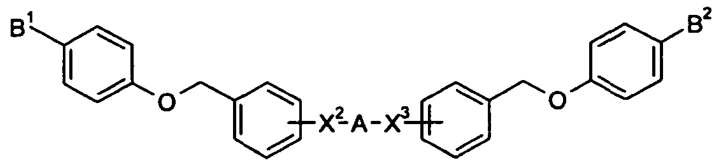

( IA )

in denen $B^1$ und $B^2$ $-C(=NH)-NH_2$ bedeuten, werden Imidoester der allgemeinen Formel (II)

$$RO-C(=NH)-Ar^1-X^1-Ar^2-X^2-A-X^3-Ar^3-X^4-Ar^4-C(=NH)-OR \quad (II)$$

in denen R für $C_1$-$C_6$-Alkyl steht, mit Ammoniak umgesetzt.

Die Umsetzung erfolgt zweckmäßig in einem organischen Lösungsmittel bei Temperaturen zwischen etwa 0°C und der Siedetemperatur des Reaktionsgemisches, vorzugsweise zwischen Raumtemperatur und etwa 100°C bzw. der Siedetemperatur des verwendeten Lösungsmittels, soweit diese niedriger ist. Geeignete Lösungsmittel sind polare organische Lösungsmittel, vorzugsweise Alkohole, besonders bevorzugt Methanol, Ethanol oder Propanole. Bei hinreichend säurestabilen Ausgangsstoffen kann die Umsetzung statt über die Imidoester auch über die entsprechenden Säureimidchloride erfolgen.

**Methode B1**

Zur Herstellung von Verbindungen der allgemeinen Formel (IA)

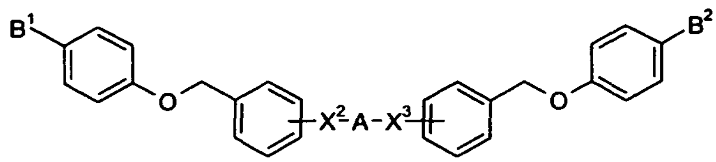

( IA )

in denen $B^1$ und $B^2$ $-C(=NOH)-NH_2$ bedeuten, werden Verbindungen der allgemeinen Formel (III)

$$NC-Ar^1-X^1-Ar^2-X^2-A-X^3-Ar^3-X^4-Ar^4-CN \quad (III)$$

mit Hydroxylamin in Gegenwart von Carbonaten oder Alkoholaten der Alkali- oder Erdalkalimetalle in Lösemitteln wie Methanol, Ethanol, n-Propanol oder Isopropanol eventuell im Gemisch mit Dioxan oder Tetrahydrofuran behandelt. Die Alkoholate können dargestellt werden aus den jeweiligen Alkalimetallen oder Metallhydriden und dem entsprechenden Alkohol. Die Reaktion wird vorzugsweise bei 20-100°C, besonders bevorzugt bei der Siedetemperatur des verwendeten Lösemittels durchgeführt.

**Methode B2**

Zur Herstellung von Verbindungen der allgemeinen Formel (I)

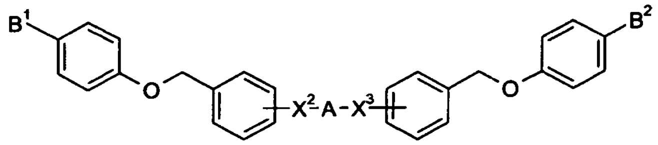

(IA)

in denen $B^1$ und $B^2$ -C(=NH)-$NH_2$ bedeuten, werden Amidoxime der allgemeinen Formel (IA) mit $B^1$ und $B^2$ -C(=NOH) -$NH_2$ reduziert.
Für die Stufe der Reduktion eignet sich die katalytische Hydrierung, insbesondere mit Raney-Nickel, Palladium oder Platin in einem niederen Alkohol, z.B. Methanol, Ethanol oder Propanole. Zweckmäßig wird das Amidoxim unter Zugabe der berechneten Menge derjenigen Säure, deren Salz als Endprodukt gewünscht wird, in einem polaren Solvens, z.B. Methanol, Ethanol, Propanole, Tetrahydrofuran oder Dimethylformamid gelöst und bei Raumtemperatur unter leichtem Druck ab 1 bar, z.B. bei 5 bar, bis zur beendeten Wasserstoffaufnahme hydriert.

**Methode C**

Zur Herstellung von Verbindungen der allgemeinen Formel (IA)

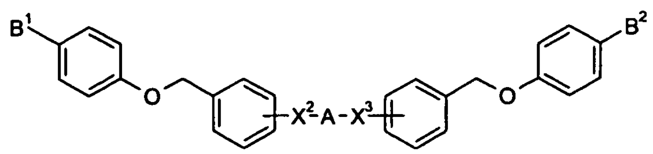

(IA)

in denen $B^1$ und $B^2$ -C(=NH)-$NH_2$ bedeuten, werden Verbindungen der allgemeinen Formel (III)

$$NC\text{-}Ar^1\text{-}X^1\text{-}Ar^2\text{-}X^2\text{-}A\text{-}X^3\text{-}Ar^3\text{-}X^4\text{-}Ar^4\text{-}CN \qquad (III)$$

mit Li-Hexamethyldisilazan umgesetzt. Für die Reaktion eignen sich unpolare und polare aprotische Lösungsmittel wie beispielsweise Toluol, Ether oder Tetrahydrofuran bei Temperaturen von -80°C bis 120°C. Zur Abspaltung der Silylgruppen werden anorganische und organische Säuren verwendet, wie HCl, HBr, $H_2SO_4$, Sulfonsäuren wie p-Toluolsulfonsäure Benzolsulfonsäure oder Methansulfonsäure, Carbonsäuren wie Ameisensäure, Essigsäure oder Trifluoressigsäure bei Temperaturen von 0°C bis 100°C.

**Methode D**

Zur Herstellung von Verbindungen der allgemeinen Formel (IA)

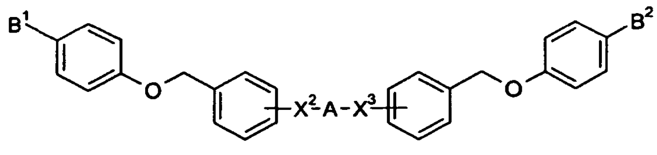

( IA )

in denen $B^1$ und $B^2$ die eingangs genannten Bedeutungen haben können, werden Verbindungen der allgemeinen Formel (IV und (V)

$$PG\text{-}B^1\text{-}Ar^1\text{-}X^1\text{-}Ar^2\text{-}CHO \quad \text{(IV)}$$

$$OHC\text{-}Ar^3\text{-}X^4\text{-}Ar^4\text{-}B^2\text{-}PG \quad \text{(V)}$$

in denen PG eine zum Schutz von Aminen geeignete Schutzgruppe darstellen kann, die auch zweimal vorhanden sein kann, mit einem Diamin unter anschließender Reduktion der dadurch gebildeten C=N-Doppelbindungen umgesetzt. Als geeignete Aminoschutzgruppen PG seien an dieser Stelle beispielsweise genannt Alkoxycarbonyl, insbesondere tert-Butyloxycarbonyl, Benzyloxycarbonyl, 2-Trimethylsilylethyloxycarbonyl, 2,2,2-Trichlorethyloxycarbonyl etc.

Zur Reaktion werden Aldehyde der Formel (IV) und (V) mit Diaminen in aprotischen Solventien wie Toluol, Dichlormethan, Essigsäureethylester, Ether, Tetrahydrofuran etc. bei Temperaturen von -80°C bis 120°C zur Reaktion gebracht. Die anschließende Reduktion kann mit komplexen Hydriden wie beispielsweise $LiAlH_4$, Li-Alkoxyhydriden, $NaBH_4$, $NaBHCN_3$, $NaBH(OAc)_3$, etc. erfolgen.

Für die Reaktion mit primären Aminen verwendet man vorzugsweise $NaBH_4$, für sekundäre Amine $NaBH(OAc)_3$. Als Lösungsmittel können polare Lösungsmittel wie DMF, Alkohole wie Methanol, Ethanol, Propanole etc., Wasser Verwendung finden. Die Temperatur wird dabei in einem Bereich von -30°C bis 100°C gehalten. Für die Spaltung der Hydrid-Komplexe verwendet man organische und anorganische Säuren wie HCl, HBr, $H_2SO_4$, Ameisensäure, Essigsäure, Sulfonsäuren wie p-Toluolsulfonsäure, Benzolsulfonsäure oder Methansulfonsäure in polaren Solventien wie Essigsäureethylester, Methanol, Ethanol, Propanole, Wasser, DMF, Acetonitril.

Abschließend erfolgt die Abspaltung der Schutzgruppen insbesondere mit anorganischen oder organischen Säuren oder auf dem Wege der Hydrogenolyse oder mit anderen aus dem Stand der Technik bekannten Verfahren, die üblicherweise zur Abspaltung spezifischer Schutzgruppen verwendet werden.

**Methode E**

Zur Herstellung von Verbindungen der allgemeinen Formel (IA)

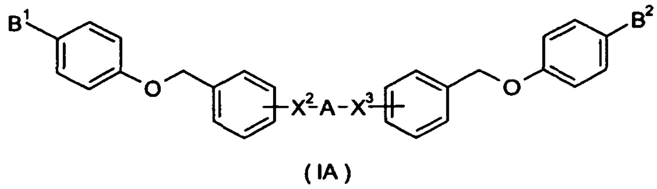

( IA )

in denen $B^1$ und $B^2$ die eingangs genannten Bedeutungen haben können, werden Verbindungen der allgemeinen Formel (VI) und (VII)

$$PG\text{-}B^1\text{-}Ar^1\text{-}X^1\text{-}Ar^2\text{-}CH_2Y \quad (VI)$$

$$YCH_2\text{-}Ar^3\text{-}X^4\text{-}Ar^4\text{-}B^2\text{-}PG \quad (VII)$$

in denen PG eine zum Schutz von Aminen geeignete Schutzgruppe darstellen kann, die auch zweimal vorhanden sein kann und

Y Fluor, Chlor, Brom oder Jod oder einen $C_1$-$C_4$-Alkyl- oder einen Arylsulfonatrest bedeuten

mit einem Diamin oder einem Dialkohol umgesetzt. Als geeignete Aminoschutzgruppen PG seien an dieser Stelle beispielsweise genannt Alkoxycarbonyl, insbesondere tert-Butyloxycarbonyl, Benzyloxycarbonyl, 2-Trimethylsilylethyloxycarbonyl, 2,2,2-Trichlorethyloxycarbonyl etc.

Die Umsetzung erfolgt mit basischen Hilfsmitteln wie beispielsweise Alkali- oder Erdalkalihydroxiden, Alkali- oder Erdalkalicarbonaten, $C_1$-$C_4$-Alkalialkoholaten in unter den gewählten Reaktionsbedingungen inerten Lösungsmitteln wie Formamiden - bevorzugt Dimethylformamid (DMF) -, $C_1$-$C_4$-Alkylestern von Carbonsäuren - bevorzugt Essigsäureethylester oder Ameisensäureethylester -, aromatischen oder aliphatischen Kohlenwasserstoffen - bevorzugt Toluol - oder in verzweigten oder unverzweigten $C_1$-$C_4$-Alkoholen.

Im abschließenden Reaktionsschritt erfolgt die Abspaltung der Schutzgruppen insbesondere mit anorganischen oder organischen Säuren oder auf dem Wege der Hydrogenolyse oder mit anderen aus dem Stand der Technik bekannten Verfahren, die üblicherweise zur Abspaltung spezifischer Schutzgruppen verwendet werden.

**Methode F**

Zur Herstellung von Verbindungen der allgemeinen Formel (IA)

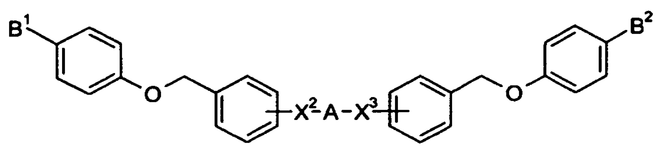

( IA )

in denen $B^1$ und $B^2$ -C(=NR$^1$)-NH$_2$ bedeuten mit $R^1 \neq H$, werden Verbindungen der allgemeinen Formel (I), in denen $B^1$ und $B^2$ -C(=NH)-NH$_2$ bedeuten, mit Chlorameisensäureestem oder Acylhalogeniden bzw. entsprechenden Anhydriden umgesetzt. Dazu werden die Bis-benzamidine in Lösungsmitteln wie Toluol, Ether, Dichlormethan, DMF, Essigsäurethylester, Wasser bei Temperaturen von 0°C bis 120°C mit Acylhalogeniden oder Säureanhydriden zusammengegeben unter Zugabe eines basischen Stoffes wie Triethylamin, cyclischen Aminen wie DBU, oder Pyridin. Die Amine können auch als Lösungsmittel verwendet werden. Auch Zweiphasengemische wie z.B. Wasser/Toluol oder Wasser/Dichlormethan sind für die Reaktion geeignet.

Die Verbindungen der Formel (I) in denen $B^1$ und $B^2$ -C(=NR$^1$)-NH$_2$ bedeuten (mit $R^1 \neq H$) können auch aus den acylierten Amidinen (IV) und (V) im Sinne von Methode D und E hergestellt werden. In diesem Fall fungiert als Schutzgruppe PG der Rest $R^1$. Hier ist deine Abspaltung der in Methode D und E aufgeführten Schutzgruppe PG nicht erforderlich.

**Methode G**

Zur Herstellung von Verbindungen der allgemeinen Formel (IA)

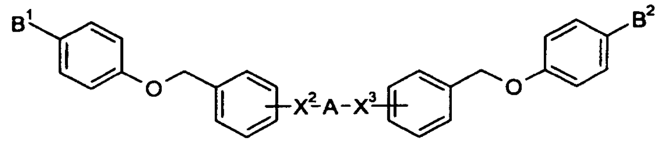

( IA )

in denen $B^1$ und $B^2$ $-CH_2-NH_2$ bedeuten, werden die entsprechenden Nitril-Verbindungen der allgemeinen Formel (III)

$$NC-Ar^1-X^1-Ar^2-X^2-A-X^3-Ar^3-X^4-Ar^4-CN \qquad (III)$$

reduziert, entweder durch katalytische Hydrierung in Solventien wie Methanol, Ethanol, höheren Alkoholen, DMF, Wasser mit Katalysatoren wie Raney-Nickel, Pd/C, Platin, oder mit Hydrid-Reagenzien, wie $NaBH_4$, $Ca(BH_4)_2$, $LiAlH_4$ und anderen Al- oder B-Hydriden bei Temperaturen von 0-100°C und Drücken von 760 Torr oder mehr.

Die vorstehend ausgeführten Methoden A-G sind sowohl zur Synthese symmetrischer als auch unsymmetrischer Verbindungen der allgemeinen Formel (I) geeignet.

Im Folgenden werden exemplarische Vorgehensweisen zur Herstellung der erfindungsgemäßen Verbindungen der allgemeinen Formel (I) detaillierter beschrieben. Die nachfolgenden Beispiele dienen ausschließlich der detaillierteren Erläuterung, ohne den Gegenstand der Erfindung zu beschränken.

Beispiel 1 (Methode B1):

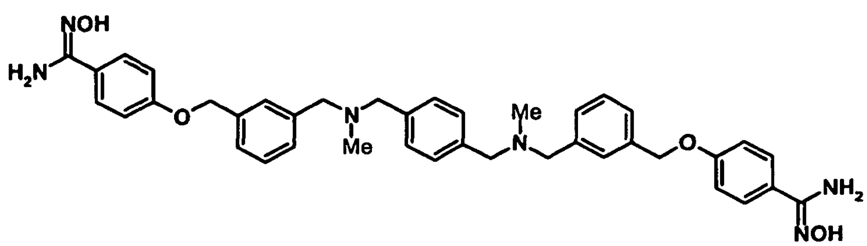

Zu 2,3 g des entsprechenden Dinitrils in 80 ml Ethanol wurden 1,4 g Natriumcarbonat und 1,83 g Hydroxylamin x HCl in 10 ml $H_2O$ getropft und 3h unter Rückfluß erhitzt. Die entstandene Suspension wurde abgesaugt und mit Ethanol gewaschen. Die Kristalle wurden über Kieselgel 60 mit Acetonitril/Dichlormethan/Ameisensäure/$H_2O$ 70:20:15:10 chromatographiert. Nach Überführen in die Base mit 2N NaOH und Extrahieren mit Essigsäureethylester wurde die Substanz in Methanol suspendiert und mit verdünnter Methansulfonsäure versetzt. Die Lösung wurde konzentriert und mit Ether langsam kristallisiert. Man erhielt 0,7 g als Tri-Methansulfonat. Fp. 210-212°C.
$^1$H-NMR(250 MHz, DMSO-d6):δ=9.66(2H, s, OH); 7.84-7.07 (2OH, m, aryl-H); 5.75 (4H, s, $NH_2$), 5.12 (4H, S, $OCH_2$-); 3.57; 3.55 (8H, 2,s,N-$CH_2$-); 2.36 (6H, s, N-$CH_3$); 2.11 (9H, s, $CH_3$-C=O).

Beispiel 2 (Methode C):

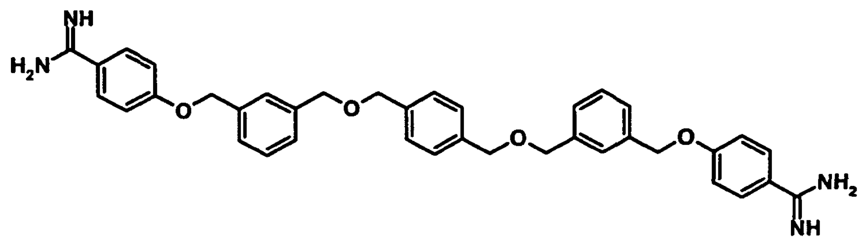

1,74 g des entsprechenden Dinitrils werden in 75 THF gelöst und unter Stickstoff 20 ml Lithium-hexamethyldisilazan (1M) in THF zugetropft. Nach Zugabe von weiteren 25 ml THF und Erhitzen auf 45°C wurde die Lösung 12 h bei Raumtemp. Gerührt. Unter Kühlen wurden bei 0°C 18 ml 4N Salzsäure langsam zugetropft. Das THF wurde abdestilliert, Wasser zugegeben und die Kristalle abgesaugt und mit Wasser gewaschen. Das Dihydrochlorid wurde mit 2N NaOH in DMF in die Base überführt. Diese wurde über 160 g Kieselgel 60 chromatographiert (Acetonitril/ Chloroform/ Eisessig/Wasser: 75:20:10:7,5). Ausbeute: 0,73 als Diacetat. Fp.:221°C.
$^1$H-NMR(250 MHz, DMSO-d6):δ=9.92 (8H, breit, -C(=$NH_2^+$)$NH_2$); 8.03-7.25 (20H, m, aryl-H); 5.33 (4H, s, $OCH_2$-); 4.64; 4.63 (8H, 2s, -$CH_2$-O-$CH_2$-); 1.74 (6H, s, $CH_3$-C=O).

Beispiel 3 (Methode D):

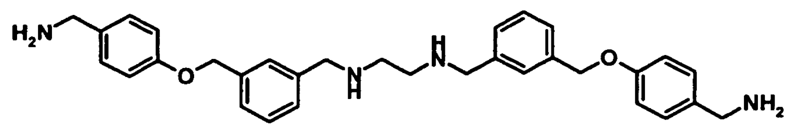

0,49 g Diaminoethan in 2 ml Dichlormethan wurden vorgelegt und 0,8 g 3-[4-(N-Boc-aminomethyl)-phenyloxymethyl]-benzaldehyd (70 %) zugegeben. Nach Erwärmen auf 80°C und Abkühlen wurden 10 ml Ethanol zugegeben und die Lösung bei 0-5°C mit 100 mg $NaBH_4$ versetzt. Nach 12 h bei Raumtemp. wurde bei 0-5°C mit 1 ml 2N HCl angesäuert und nach 2 h die ausgefallenen Kristalle abgesaugt. Nach Lösung in HCl/Essigsäureethylester, DMF und Methanol wurde erwärmt und nach 12 h eingeengt. Der Rückstand wurde in Methanol aufgenommen und die Kristalle abgesaugt. Ausbeute: 0,33 g.
$^1$H-NMR(250 MHz, $CD_3OD$):δ=7.75-7.10 (16H, m, aryl-H); 5.19 (4H, s, $OCH_2$-); 4.32(4H, s, N-$CH_2$-phenyl); 4.09 (4H, s, $CH_2NH_2$); 3.47 (4H, s, N-$CH_2CH_2$-N).

Beispiel 4 (Methode E):

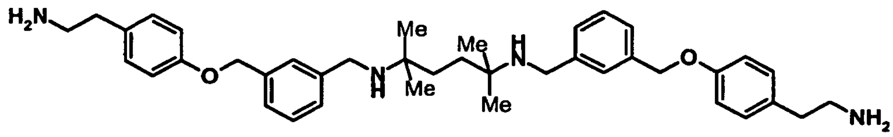

3,75 g N-Boc-2-[4-(3-chlormethyl-benzyloxy-)phenyl]ethylamin, 0,72 g 2,5-Diamino-2,5-dimethyl-hexan, 1,4 g Kaliumcarbonat und 0,1 g Kaliumjodid in 15 ml DMF wurden 6 h bei 75°C Innentemperatur und weitere 7 h bei 140°C gerührt. Die Suspension wurde eingedampft, der Rückstand in Wasser aufgenommen und mit Essigsäureethylester

extrahiert. Die über $Na_2SO_4$ getrocknete organische Phase wurde eingedampft und der Rückstand über Kieselgel 60 mit Dichlormethan/Methanol/konz. Ammoniak 65:35:5 chromatographiert. Die Boc-geschützte Verbindung wurde in 10 ml Essigsäureethylester gelöst und mit 10 ml 3M HCl in Essigsäureethylester versetzt und 24 h gerührt. Die ausgefallenen Kristalle wurden aus 50 ml Ethanol umkristallisiert. Ausbeute: 0,22 g als Tetrahydrochlorid. Fp.: 270°C (Zersetzung).
$^1$H-NMR(250 MHz, $CD_3OD$):δ=7.85-6.98 (16H, m, aryl-H); 5.16 (4H, s, $OCH_2$-); 4.30 (4H, s, N-$CH_2$-phenyl); 3.15; 2.90 (8H, 2m, N-$CH_2$-$CH_2$-phenyl); 1.95 (4H, s, C-$CH_2CH_2$-); 1.52 (12H, s, $CH_3$-C-$CH_3$).

Beispiel 5 (Methode F):

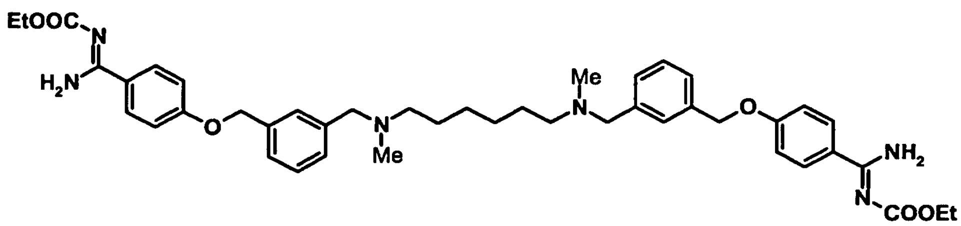

0,3 g des entsprechenden Bis-benzamidins wurden als Base in wenig abs. Ethanol angelöst. Dazu wurden 50 ml Dichlormethan und 0,115 g Chlorameisensäureethylester gegeben und bei Raumtemp. 1 ml Triethylamin zugetropft. Nach 2 h wurde zweimal mit je 50 ml Wasser extrahiert. Die organischen Phasen wurden eingeengt und über Kieselgel 60 chromatographiert (Acetonitril/Dichlormethan/Ameisensäure/$H_2O$ 70:20:15:10). Das Produkt wurde in Wasser aufgenommen, mit Natronlauge versetzt, mit 100 ml Essigsäureethylester extrahiert, getrocknet und eingedampft. Ausbeute: 85 mg als farbloses Öl.
$^1$H-NMR(250 MHz, $CDCl_3$):δ=8.50 (4H, s, $NH_2$); 8.02-6.96 (16H, m, aryl-H); 5.18 (4H, s, $OCH_2$-); 4.25 (4H, qu, J=7.0 HZ, $OCH_2$-$CH_3$); 4.10 (4H, s, N-$CH_2$-phenyl); 2.80-1.07 (12H, m, N-$CH_2(CH_2)_4$-$CH_2$-N); 2.55 (6H, s, N-$CH_3$); 1.28 (6H, t, J=7.0 HZ, $OCH_2$-$CH_3$).

Beispiel 6 (Methode G):

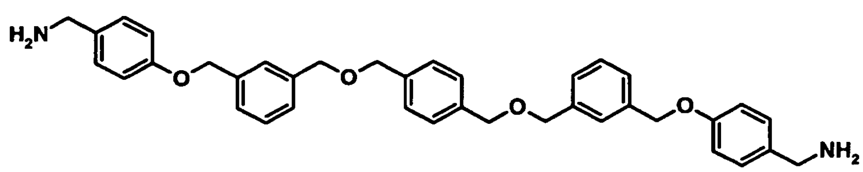

1,16 g des entsprechenden Dinitrils in 60 ml DMF wurden unter Zugabe von methanolischer Ammoniaklösung und Raney-Nickel 6 h bei 5 bar und 60°C hydriert. Der Katalysator wurde abgesaugt und das Lösungsmittel entfernt. Der Rückstand wurde in 100 ml DMF heiß gelöst und nach dem Abkühlen abgesaugt. Das Filtrat wurde in 50 ml DMF gelöst, die berechnete Menge etherische Salzsäure zugegeben, das Lösungsmittel abdestilliert und mit Ethanol verrührt. Der Rückstand wurde mit konz. Ammoniak in die Base überführt und über 70 g Kieselgel 60 chromatographiert (Acetonitril/Chloroform/Eisessig/Wasser 75:20:10:7,5). Ausbeute: 0,24 g als Diacetat. Fp.: 148°C.
$^1$H-NMR(250 MHz, DMSO-d6):δ=7.44-6.86 (2OH, m, aryl-H); 5.08 (4H-$OCH_2$-); 4.60 (6H, s, $CH_3$); 4.52; 4.51 (8H, 2s, -$CH_2$-O-$CH_2$-); 3.69 (4H, s, $CH_2$-$NH_2$); 1.81 (6H, s, $CH_3$-C=O).

In Analogie zu den vorstehend beschriebenen Synthesebeispielen und gemäß der Synthesemethoden A-G wurden ferner u.a. die folgenden Verbindungen erhalten:

Beispiel 7 (Methode G):

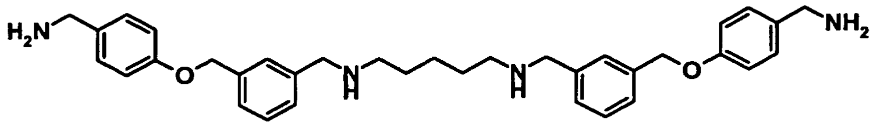

$^1$H-NMR(250 MHz, $CD_3OD$):δ=7.72-7.07 (16H, m, aryl-H); 5.20 (4H, s, $OCH_2$); 4.25 (4H, s, N-$CH_2$-phenyl); 4.10 (4H, s, $CH_2$-$NH_2$); 3.09 (4H, m, N-$CH_2(CH_2)_3$-$CH_2$-N); 1.89-1.40 (6H, m, N-$CH_2(CH_2)$-).

Beispiel 8 (Methode D):

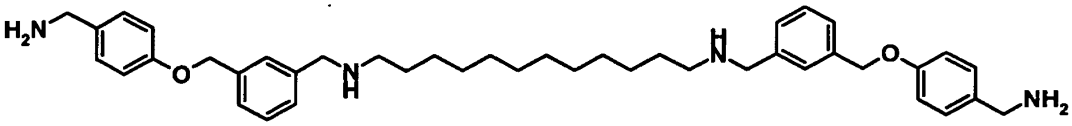

$^1$H-NMR(250 MHz, $CD_3OD$):δ=7.62-7.02 (16H, m, aryl-H); 5.14 (4H, s, $OCH_2$-); 4.20 (4H, s, H-$CH_2$-phenyl); 4.06 (4H, s, $CH_2$-$NH_2$); 2.99 (4H, m, N-$CH_2$-$(CH_2)_{10}$-$CH_2$-N); 1.82-1.17 (20H, m, N-$(CH_2)_{10}$-).

Beispiel 9 (Methode D):

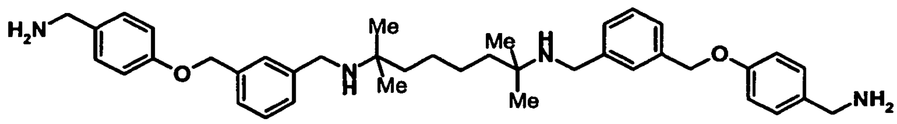

$^1$H-NMR(250 MHz, $CD_3OD$):δ=7.93-7.19 (16H, m, aryl-H); 5.28 (4H, s, $OCH_2$-); 4.33 (4H, s, N-$CH_2$-phenyl); 4.13 (4H, s, $CH_2$-$NH_2$); 2.03-1.38 (8H, m, C-$(CH_2)_4$-C); 1.48 (12H, s, C-$(CH_3)_2$).

Beispiel 10 (Methode D):

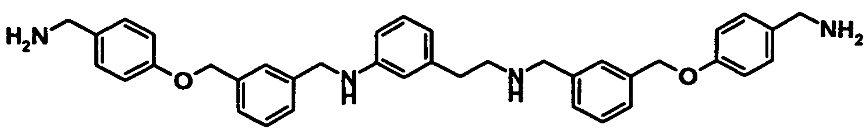

$^1$H-NMR(250 MHz, $CD_3OD$):δ=7.63-6.85 (20H, m, aryl-H); 5.10; 5.04(4H, 2s, $OCH_2$); 4.40; 4.18; 4.01 (8H, 3s, N-$CH_2$); 3.20; 2.95 (4H, 2m, H-$CH_2CH_2$-N).

Beispiel 11 (Methode D):

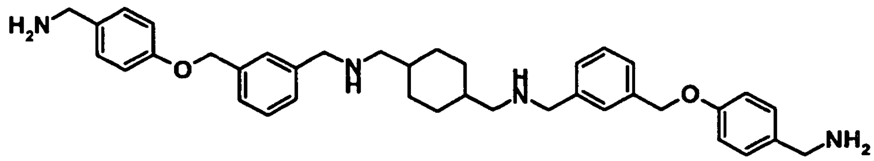

$^1$H-NMR(250 MHz, $CD_3OD$):δ=7.70-7.03 (16H, m, aryl-H); 5.15 (4H, s, $OCH_2$-); 4.20 (4H, s, N-$CH_2$-phenyl); 4.05 (4H, s, $CH_2$-$NH_2$); 2.82 (4H, d, J=5.8 Hz, $CH_2$-CH); 2.00-0.95 (10H, m, cyclohexyl-H).

Beispiel 12 (Methode D):

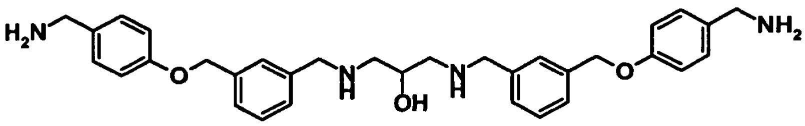

$^1$H-NMR(250 MHz, $CD_3OD$):δ=7.76-7.07 (16H, m, aryl-H); 5.19 (4H, s, $OCH_2$); 4.37 (1H, m, CH-OH); 4.29 (4H, s, N-$CH_2$-phenyl); 4.09 (4H, s, $CH_2$-$MH_2$); 3.16 (4H, m,-$\underline{CH_2}$-CHOH-$\underline{CH_2}$-).

Beispiel 13 (Methode D):

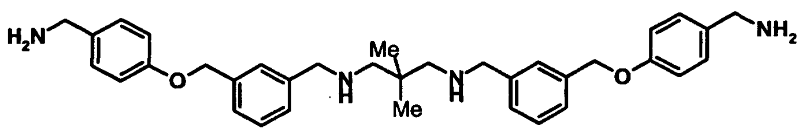

$^1$H-NMR(250 MHz, $CD_3OD$):δ=7.70-7.05 (16H, m, aryl-H); 5.15 (4H, s, $OCH_2$); 4.23 (4H, s, N-$CH_2$-phenyl); 4.07 (4H, s, $CH_2$-$NH_2$); 3.02 (4H, S, -$CH_2$-C-$CH_2$-); 1.11 (6H, s, $CH_3$-C-$CH_3$).

Beispiel 14 (Methode D):

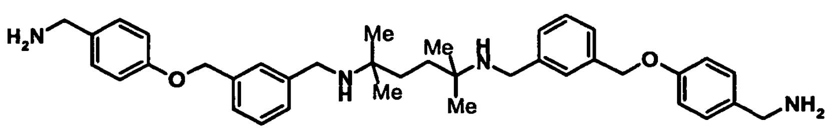

$^1$H-NMR(250 MHz, $CD_3OD$):δ=7.88-7.17 (16H, m, aryl-H); 5.27 (4H, s, $OCH_2$); 4.31 (4H, s, N-$CH_2$-phenyl); 4.13 (4H, s, $CH_2$-$NH_2$); 1.93 (4H, s, C-$CH_2CH_2$-C); 1.51 (12H, s, $CH_3$-C-$CH_3$).

Beispiel 15 (Methode D):

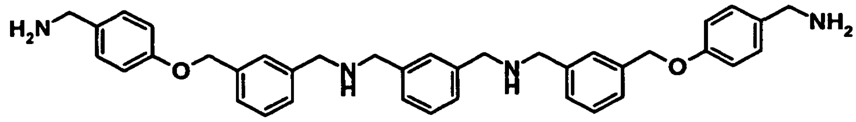

$^1$H-NMR(250 MHz, $CD_3OD$):δ=7.79-7.13 (2OH, m, aryl-H; 5.22 (4H, s, $OCH_2$); 4.28 (8H, s, N-$CH_2$-phenyl); 4.11 (4H, s, $CH_2$-$NH_2$):

Beispiel 16 (Methode D):

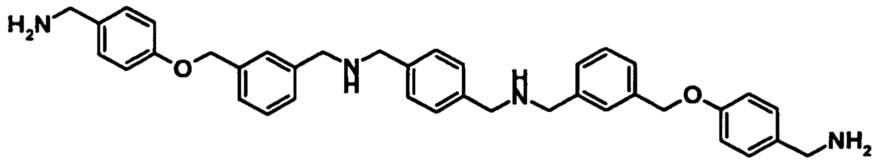

$^1$H-NMR(250 MHz, $CD_3OD$):δ=7.60-7.00 (2OH, m, aryl-H); 5.14 (4H, s, $OCH_2$); 4.15 (8H, s, N-$CH_2$-phenyl); 4.04 (4H, s, $NH_2$-$CH_2$).

Beispiel 17 (Methode D):

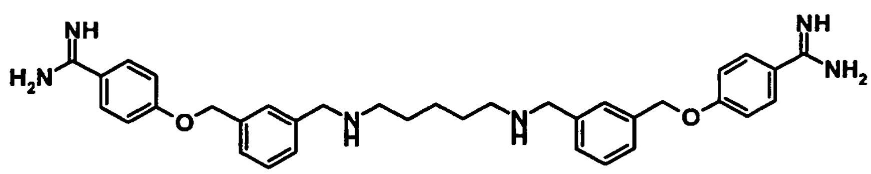

$^1$H-NMR(250 MHz, DMSO-d6):δ=9.54 (4H, s, $NH_2^+$); 9.23; 9.09 (8H, 2s, -C(=$NH_2^+$)$NH_2$); 7.90-7.15 (16H, m, aryl-H); 5.22 (4H, s, $OCH_2$); 4.11 (4H, N-$CH_2$-phenyl); 2.83 (4H, m, N-$CH_2(CH_2)_3$-$CH_2$-N); 1.54-0.90 (6H, m, N-$CH_2$-$(CH_2)_3$-).

Beispiel 18 (Methode D):

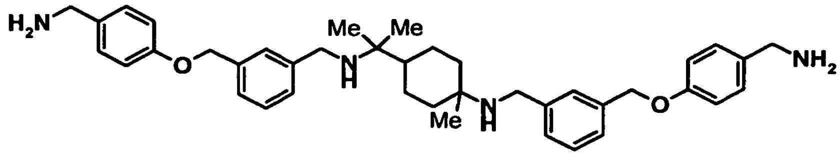

$^1$H-NMR(250 MHz, $CD_3OD$):δ=7.89-7.20(16H, m, aryl-H); 5.28 (4H, s, $OCH_2$); 4.39; 4.33 (4H, 2s, N-$CH_2$-phenyl); 4.15 (4H, s, $CH_2$-$NH_2$); 2.43-1.30 (9H, m, cyclohexyl); 1.56 (3H, s, C-$CH_3$); 1.50 (6H, s, $CH_3$-C-$CH_3$).

Beispiel 19 (Methode D):

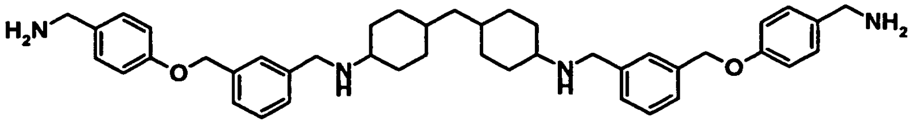

$^1$H-NMR(250 MHz, $CD_3OD$):δ=7.68-6.93 (16H, m, aryl-H); 5.08 (4H, s, $OCH_2$); 4.19 (4H, s, N-$CH_2$-phenyl); 4.02 (4H, s, $CH_2$-$NH_2$); 3.08 (2H, m, N-CH-cyclohexyl); 2.30-0.88 (20H, cyclohex-$CH_2$-cyclohex-H).

Beispiel 20 (Methode D):

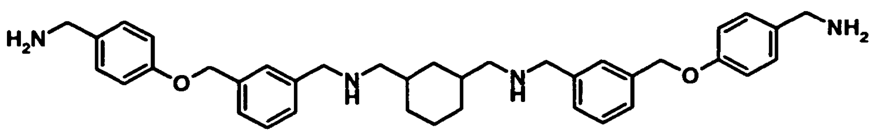

$^1$H-NMR(250 MHz, $CD_3OD$):δ=7.85-7.13 (16H, m, aryl-H); 5.23 (4H, s, $OCH_2$); 4.31 (4H, s, N-$CH_2$-phenyl); 4.11 (4H, s, $CH_2$-$NH_2$); 3.12 (4H, m, N-$CH_2$-cyclohexyl); 2.33-0.73 (10H, m, cyclohexyl-H).

Beispiel 21 (Methode D):

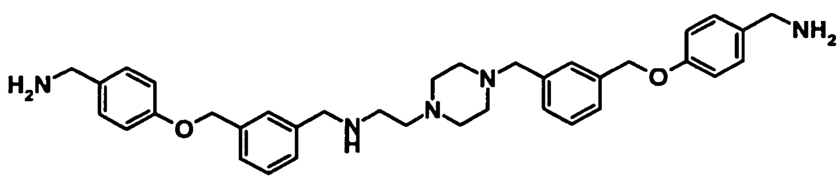

$^1$H-NMR(250 MHz, $CD_3OD$):δ=7.93-7.15 (16H, m, aryl-H); 5.28 (4H, s, $OCH_2$); 4.35 (4H, s, N-$CH_2$-phenyl); 4.14 (4H, m, $CH_2$-$NH_2$); 3.76-3.46 (12H, m, $NCH_2CH_2$-pip.).

Beispiel 22 (Methode D):

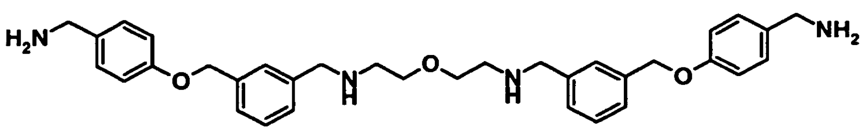

$^1$H-NMR(250 MHz, DMSO-d6):δ=9.54 (4H, s, $NH_2$); 9.23; 9.09 (8H, 2s, $C(=NH_2^+)NH_2$); 7.90-7.15 (16H, m, aryl-H); 5.22 (4H, s, $OCH_2$); 4.11 (4H, N-$CH_2$-phenyl); 2.83 (4H, m, N-$CH_2(CH_2)_3$-$CH_2$-N); 1.54-0.90 (6H, m, N-$CH_2$-$(CH_2)_3$-).

Beispiel 23 (Methode D):

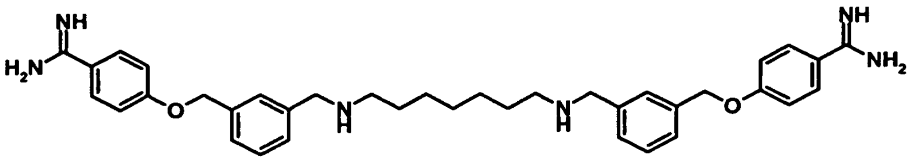

$^1$H-NMR(250 MHz, $CD_3OD$):δ=7.88-7.16 (16H, m, aryl-H); 5.27 (4H, s, $OCH_2$); 4.24 (4H, s, N-$CH_2$-phenyl); 3.05 (4H, m, N-$CH_2$-$(CH_2)_5$-$CH_2$-N-); 1.89-1.27 (1OH, m, N-$CH_2(CH_2)_5$-);

Beispiel 24 (Methode D):

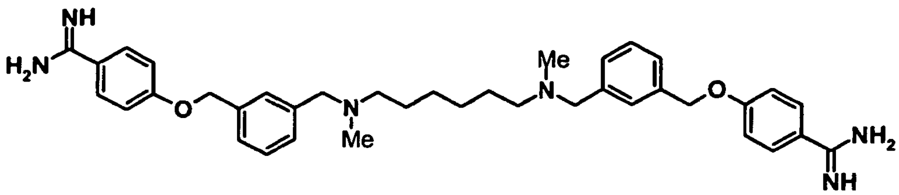

$^1$H-NMR(250 MHz, $CD_3OD$):δ=7.85-7.15 (16H, m, aryl-H); 5.27 (4H, s, $OCH_2$-); 4.51; 4.29 (4H, m, N-$CH_2$-phenyl); 3.20 (4H, m, N-$CH_2$-$(CH_2)_4$-$CH_2$-N); 2.81 (6H, s, N-$CH_3$); 2.03-1.35 (8H, m, N-$CH_2$-$(CH_2)_4$-).

Beispiel 25 (Methode D):

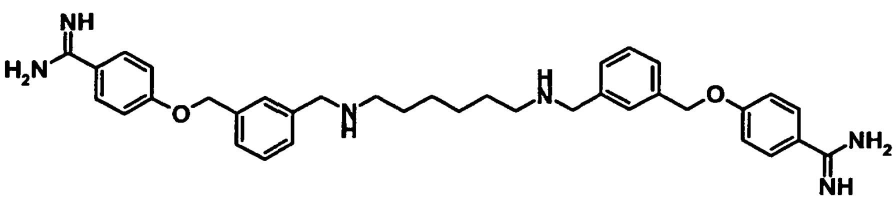

$^1$H-NMR(250 MHz, $CD_3OD$):δ=7.98-7.26 (16H, m, aryl-H); 5.32 (4H, s, $OCH_2$-); 4.28 (4H, s, N-$CH_2$-phenyl); 3.08 (4H, m, N-$CH_2$-$(CH_2)_4$-$CH_2$-N); 1.89-1.32 (8H, m, N-$CH_2$-$(CH_2)_4$-).

Beispiel 26 (Methode D):

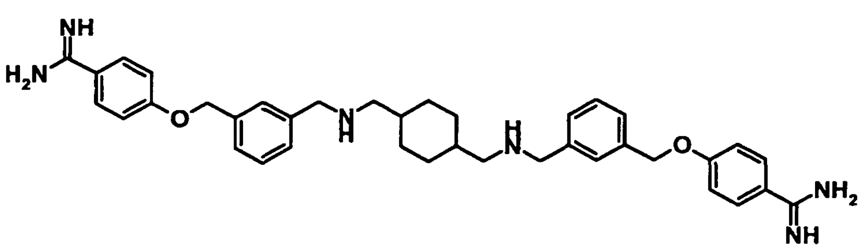

$^1$H-NMR(250 MHz, $CD_3OD$):δ=7.80-7.13 (16H, m, aryl-H); 5.22 (4H, s, $OCH_2$); 4.22 (4H, s, N-$CH_2$-phenyl); 2.90 (4H,

d, J=7.4 Hz, N-$CH_2$-cyclohexyl); 2.13-0.93 (10H, m, cyclohexyl-H).

Beispiel 27 (Methode D):

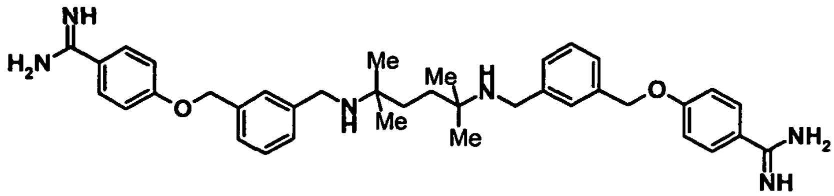

$^1$H-NMR(250 MHz, DMSO-$d_6$):δ=10.15; 9.20; 8.37 (12H, 3s, $NH_2$); 7.99-7.11 (16H, m, aryl-H); 5.22 (4H, s, $OCH_2$-); 4.00 (4H, s, N-$CH_2$-phenyl); 1.91-1.12 (4H, m, -C-$CH_2CH_2$-C); 1.30 (12H, s, -$C(CH_3)_2$-$CH_2$-$CH_2$-$C(CH_3)_2$-).

Beispiel 28 (Methode D):

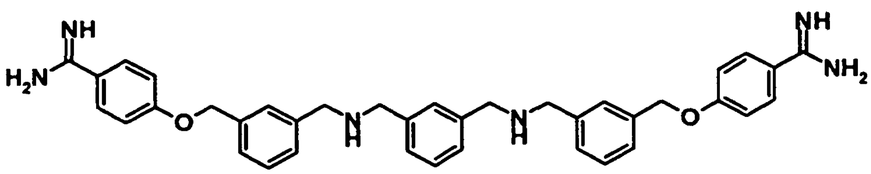

$^1$H-NMR(250 MHz, $CD_3OD$):δ=8.03-7.23 (2OH, m, aryl-H); 5.33 (4H, s, $OCH_2$); 4.31 (8H, s, N-$CH_2$-phenyl).

Beispiel 29 (Methode D):

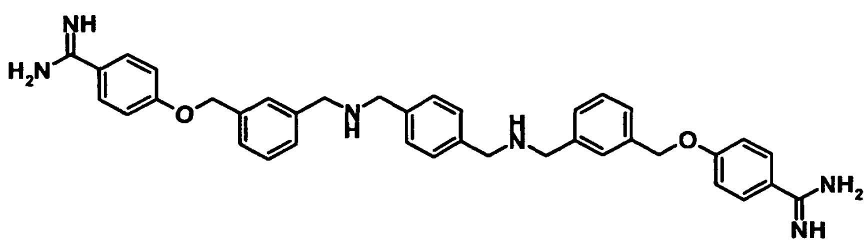

$^1$H-NMR(250 MHz, $CD_3OD$):δ=8.03-7.29 (2OH, m, aryl-H); 5.37 (4H, s, $OCH_2$); 4.40 (8H, s, N-$CH_2$-phenyl).

Beispiel 30 (Methode D):

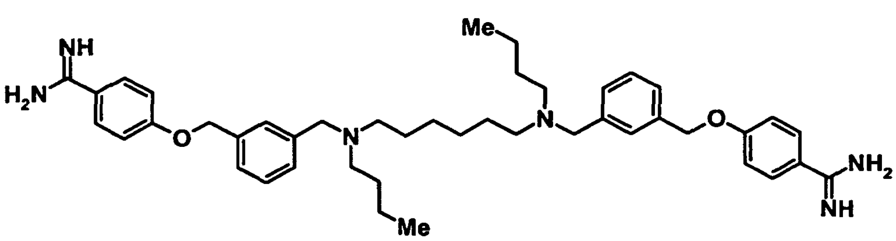

$^1$H-NMR(250 MHz, $CD_3OD$):δ=7.96-7.19 (16H, m, aryl-H); 5.32 (4H, s, $OCH_2$-); 4.43; 4.41 (4H, m, N-$CH_2$-phenyl); 3.15 (8H, m, N-$CH_2$-$(CH_2$-$)_4$-$CH_2$-N; N-$CH_2$-$CH_2CH_2CH_3$); 2.03-1.22 (16H, m, N-$CH_2$-$(CH_2)_4$; N-$CH_2CH_2CH_2CH_3$); 0.95 (6H, m, N-$(CH_2)_3$-$CH_3$).

Beispiel 31 (Methode D):

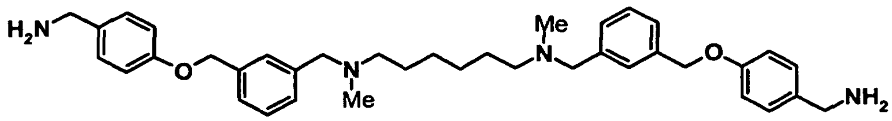

$^1$H-NMR(250 MHz, $CD_3OD$):δ=7.74-7.00 (16H, m, aryl-H); 5.17 (4H, s, $OCH_2$-); 4.40 (4H, s, N-$CH_2$-phenyl); 4.05 (4H, s, $CH_2$-$NH_2$); 3.18 (4H, m, N-$CH_2(CH_2)_4$-$CH_2$-N); 2.77 (6H, s, N-$CH_3$); 1.95-1.36 (8H, m, N-$CH_2(CH_2)_4$).

Beispiel 32 (Methode D):

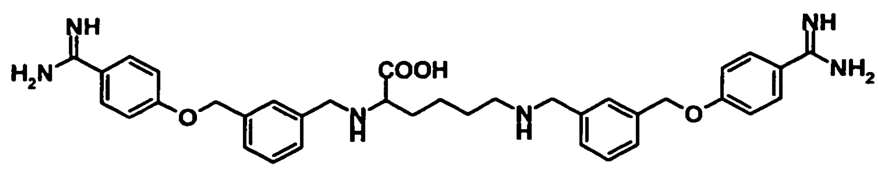

$^1$H-NMR(250 MHz, $CD_3OD$):δ=7.91-7.10 (16H, m, aryl-H); 5.23 (4H, s, $OCH_2$-); 4.24 (4H, s, N-$CH_2$-phenyl); 2.86 (3H, m, NH-CH-; N-$CH_2$-$CH_2CH_2$); 2.05-1.24 (6H, m, N-$CH_2$-$CH_2CH_2$);

Beispiel 33 (Methode D):

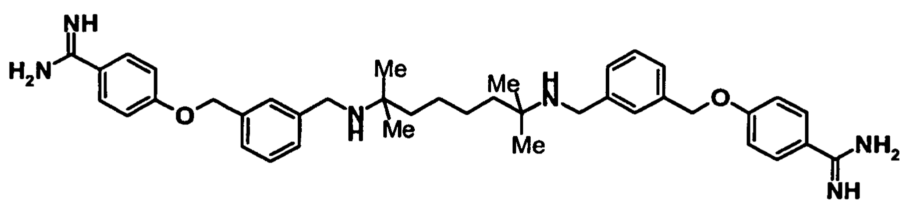

$^1$H-NMR(250 MHz, DMSO-d6):δ=9.61; 9.56; 9.38 (12H, 3, s, $NH_2$; C(=$NH_2^+$)$NH_2$); 8.22-7.43 (16H, m, aryl-H); 5.34 (4H, s, $OCH_2$-); 4.28 (4H, s, N-$CH_2$-phenyl); 2.05-1.27 (8H, m, C-$CH_2CH_2$-$CH_2CH_2$-C); 1.49 (12H, s, $CH_3$-C-$CH_3$).

Beispiel 34 (Methode D):

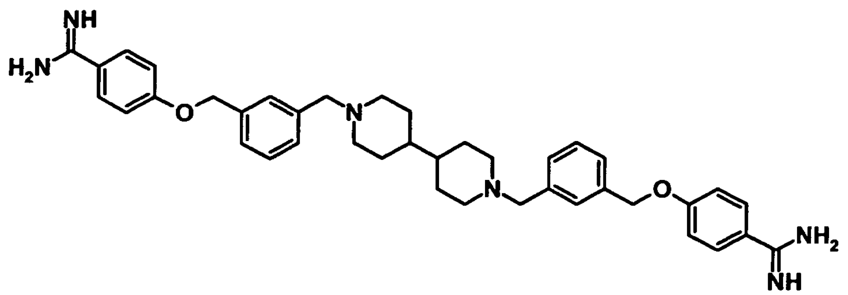

$^1$H-NMR(250 MHz, $CD_3OD$):δ=7.80-7.06 (16H, m, aryl-H); 5.18 (4H, s, $OCH_2$-); 3.48 (4H, s, N-$CH_2$-phenyl); 2.97-0.92 (18H, m, pip-H).

Beispiel 35 (Methode E):

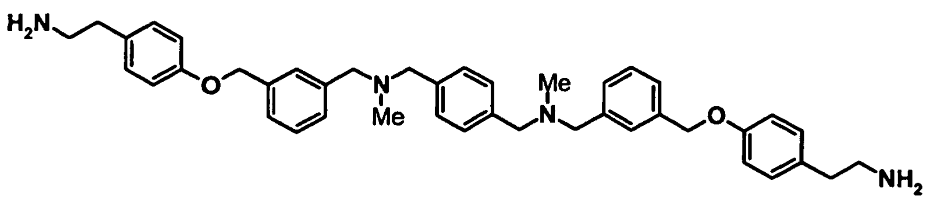

$^1$H-NMR(250 MHz, $CD_3OD$):δ=7.88-7.02 (2OH, m, aryl-H); 5.21 (4H, s, $OCH_2$-); 4.55; 4.43 (8H, 2s, N-$CH_2$-phenyl); 3.16; 2.93 (8H, 2m, N-$CH_2CH_2$-phenyl); 2.75 (6H, s, N-$CH_3$).

Beispiel 36 (Methode B1):

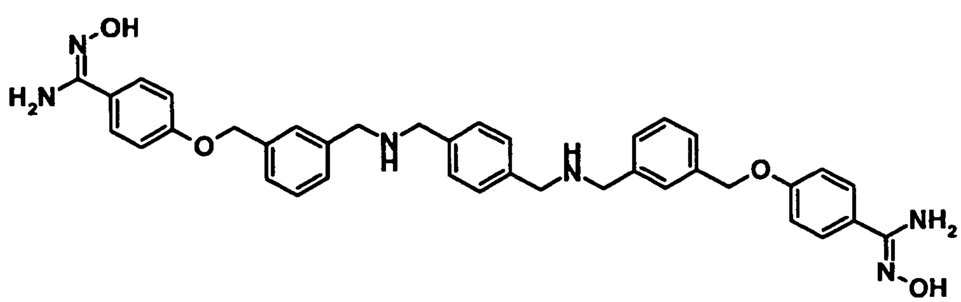

$^1$H-NMR(250 MHz, DMSO-d6):δ=13.16 (2H, s, C=N-OH); 11.33 (4H, s, $NH_2$); 10.26 (4H, s, $NH_2$); 7.86-7.22 (20H, m, aryl-H); 5.31 (4H, s, $OCH_2$-); 4.21 (8H, s, N-$CH_2$-phenyl).

Beispiel 37 (Methode F):

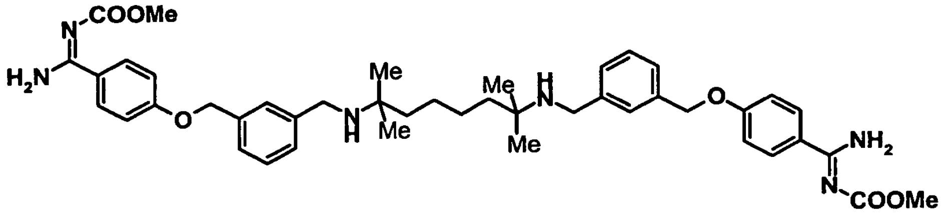

$^1$H-NMR(250 MHz, DMSO-d6):δ=9.14 (8H, s, $NH_2$); 8.11-7.04 (16H, m, aryl-H); 5.19 (4H, s, $OCH_2$-); 4.11 (4H, s, N-$CH_2$-phenyl); 3.59 (6H, s, $OCH_3$); 1.60-1.15 (8H, m, C-$(CH_2)_4$; 1.31 (18H, s, $CH_2$-C-$CH_3$);

Beispiel 38 (Methode G):

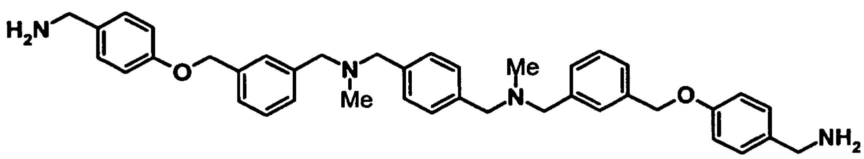

$^1$H-NMR(250 MHz, $CD_3OD$):δ=7.76-7.06 (2OH, m, aryl-H); 5.19 (4H, s, $OCH_2$-); 4.51; 4.38 (8H, 2s, N-$CH_2$-phenyl); 4.05 (4H, s, $CH_2$-$NH_2$); 2.72 (6H, s, N-$CH_3$).

Beispiel 39 (Methode D):

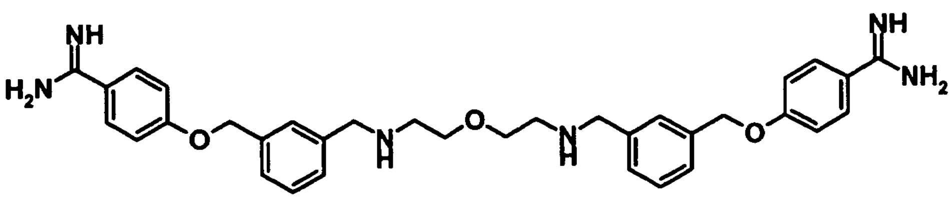

$^1$H-NMR(250 MHz, DMSO-d6):δ=9.80; 9.42; 9.19 (12H, 3s, $NH_2^+$, C(=$NH_2^+$)$NH_2$); 7.97-7.13 (16H, m, aryl-H); 5.22 (4H, s, $OCH_2$-); 4.20 (4H, s, N-$CH_2$-phenyl); 3.75; 3.06 (8H, 2m, N-$CH_2$-$CH_2$-O).

Beispiel 40 (Methode D):

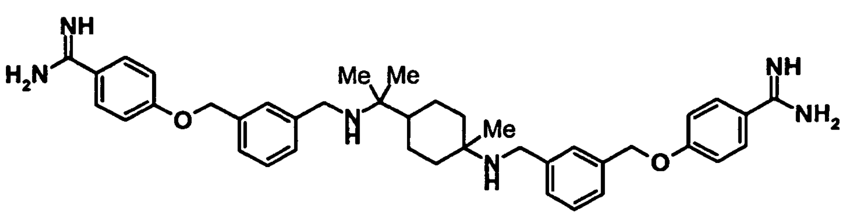

$^1$H-NMR(250 MHz, DMSO-d6):δ=9.31; 9.11; 9.09 (12H, 3s, $NH_2^+$, $C(=NH_2^+)NH_2$); 8.00-7.15 (16H, m, aryl-H); 5.23 (4H, s, $OCH_2$); 4.11 (4H, s, N-$CH_2$-phenyl); 2.37-0.83 (9H, m, pip-H); 1.38 (6H, s, $C(CH_3)_2$); 1.29 (3H, s, C-$CH_3$).

Beispiel 41 (Methode D):

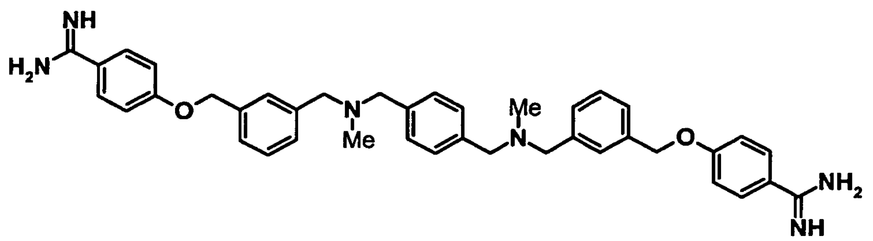

$^1$H-NMR(250 MHz, $CD_3OD$):δ=7.80-7.06 (16H, m, aryl-H); 5.18 (4H, s, $OCH_2$-); 3.48 (4H, s, N-$CH_2$-phenyl); 2.97-0.92 (18H, m, pip-H).

Beispiel 42 (Methode E):

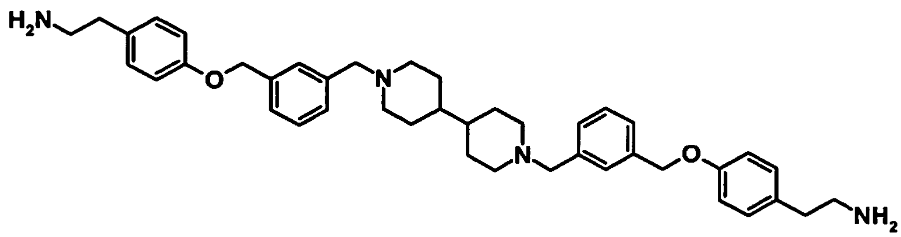

$^1$H-NMR(250 MHz, $CD_3OD$):δ=7.43-6.85 (16H, m, aryl-H); 5.15 (4H, s, $OCH_2$); 3.49 (4H, s, N-$CH_2$-phenyl); 2.88-0.93 (26H, m, $CH_2$-$CH_2$-$NH_2$; pip.-H).

Beispiel 43 (Methode D):

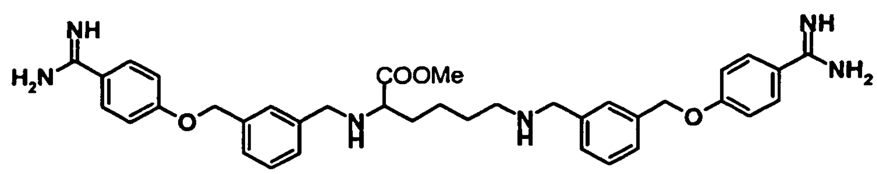

Beispiel 44 (Methode D):

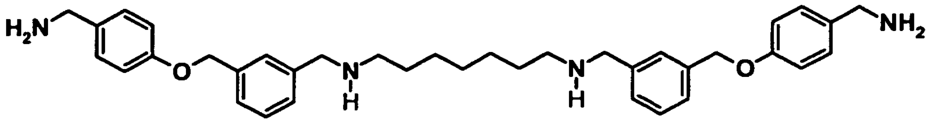

Beispiel 45 (Methode D):

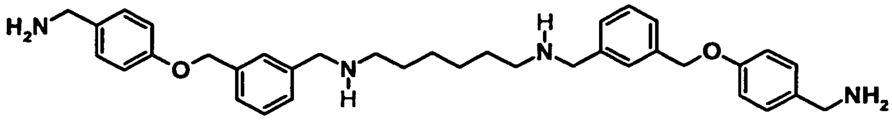

Beispiel 46 (Methode E):

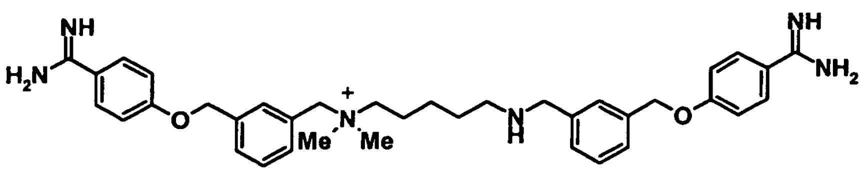

Beispiel 47 (Methode D):

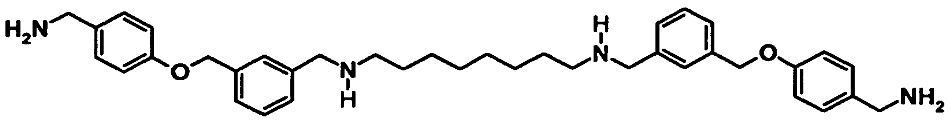

Beispiel 48 (Methode D):

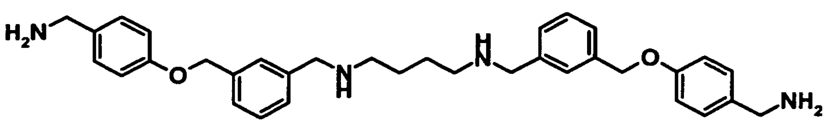

Beispiel 49 (Methode D):

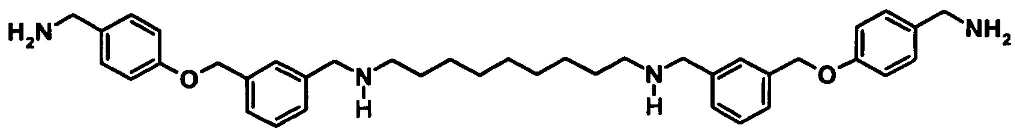

Beispiel 50 (Methode D):

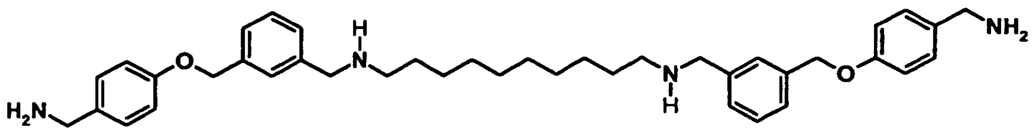

Beispiel 51 (Methode D):

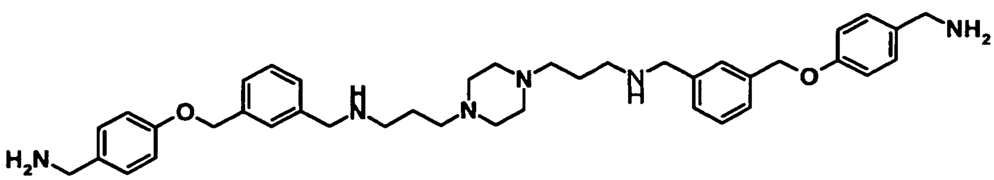

Beispiel 52 (Methode D):

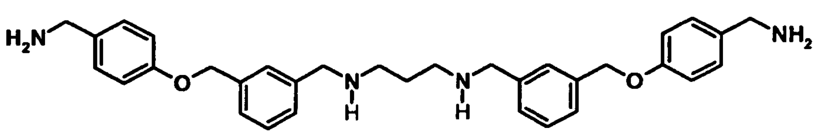

Beispiel 53 (Methode B1):

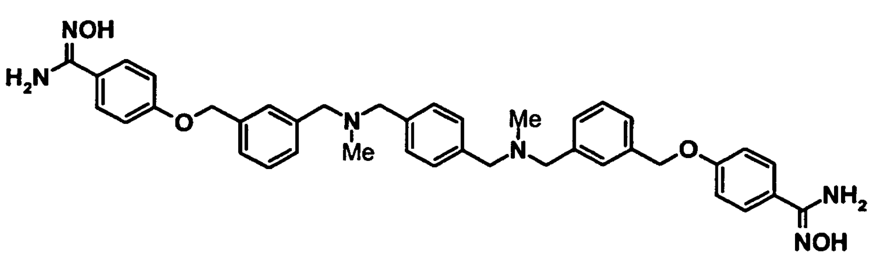

Beispiel 54 (Methode G):

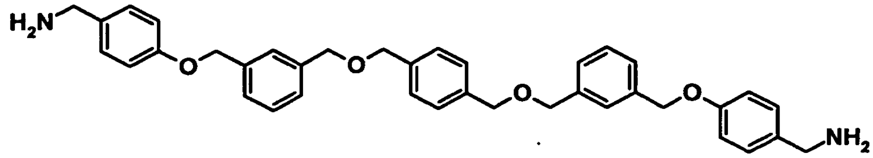

Beispiel 55 (Methode F):

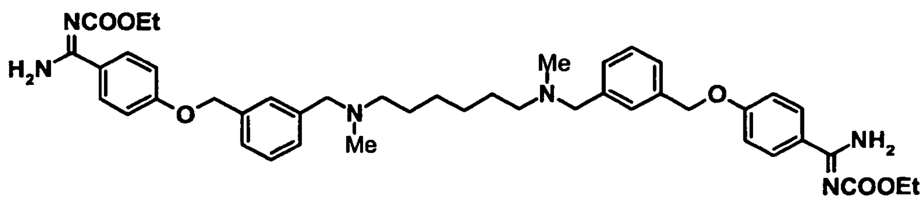

Beispiel 56 (Methode B1/B2):

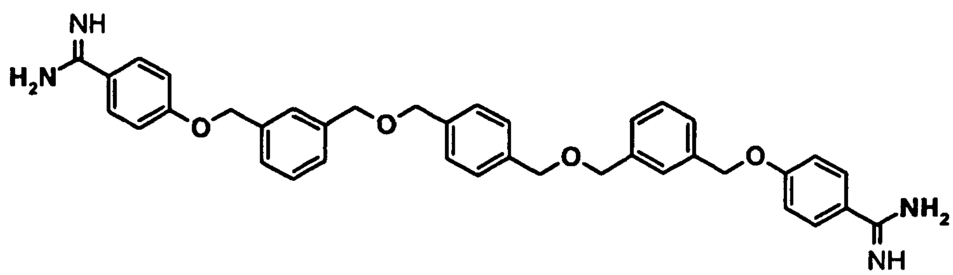

Beispiel 57 (Methode F):

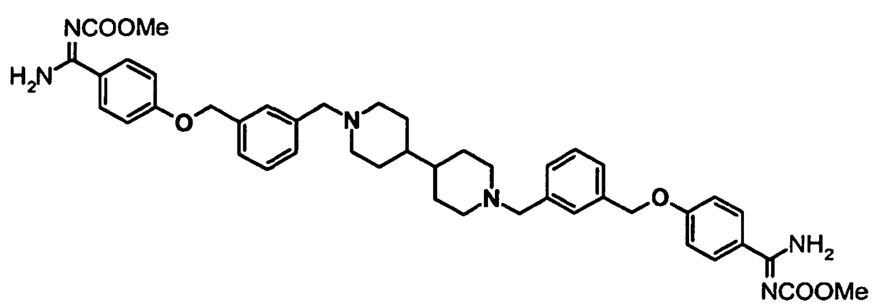

Beispiel 58 (Methode F):

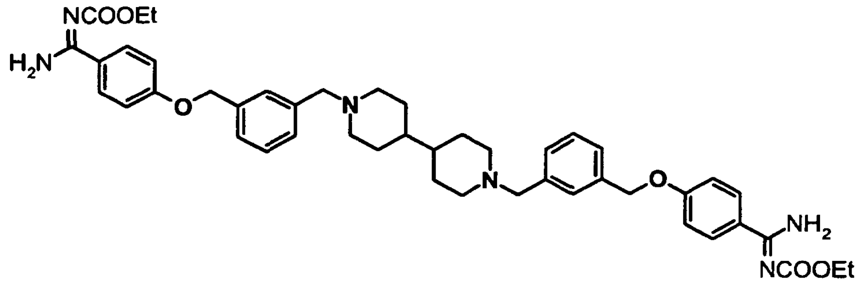

Beispiel 59 (Methode F):

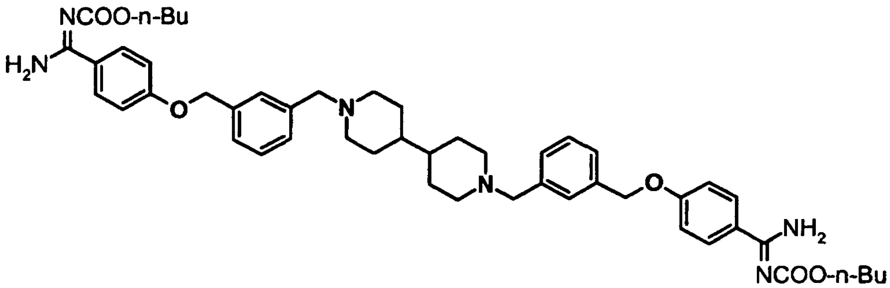

Beispiel 60 (Methode F):

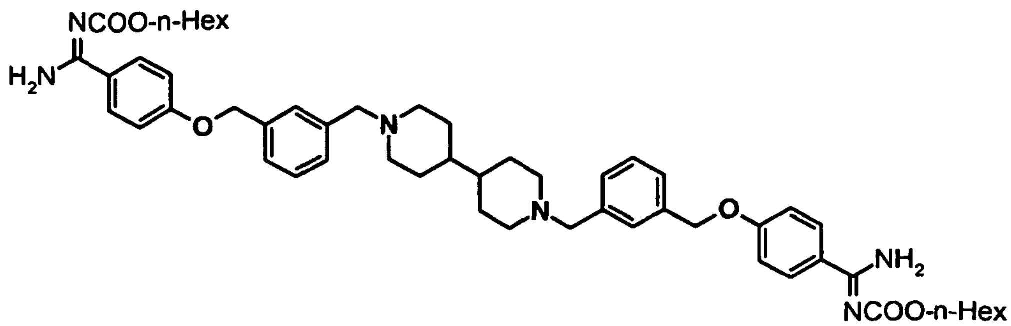

Beispiel 61 (Methode F):

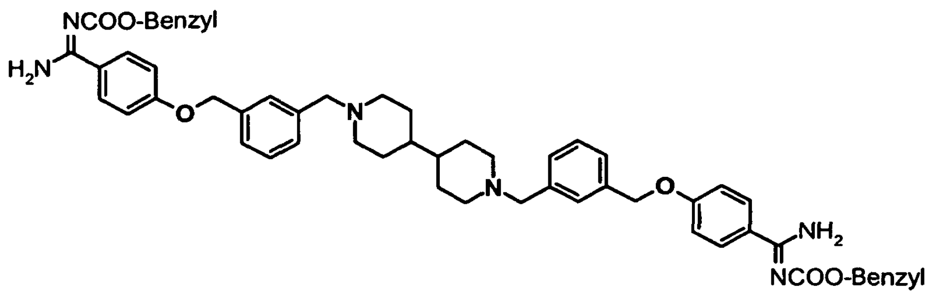

Beispiel 62 (Methode D):

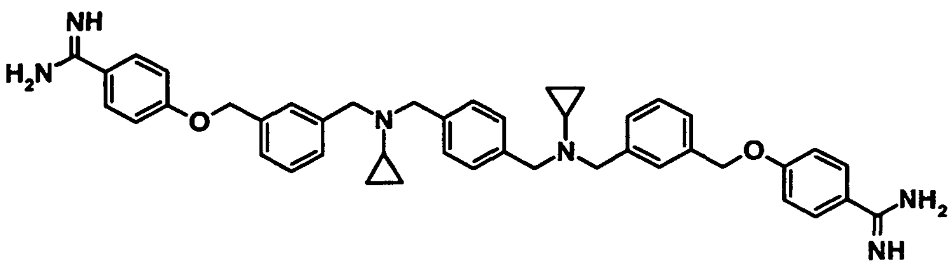

Beispiel 63 (Methode D):

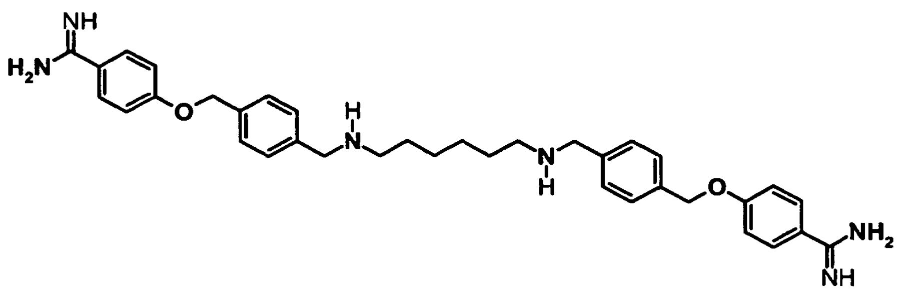

Beispiel 64 (Methode D):

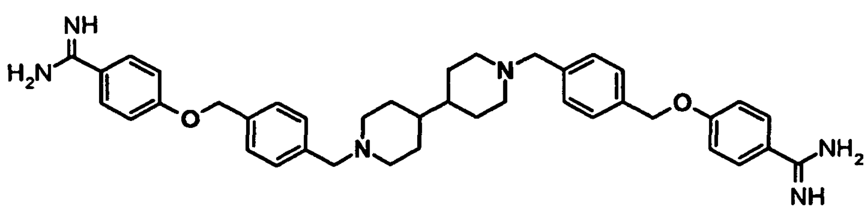

Beispiel 65 (Methode D):

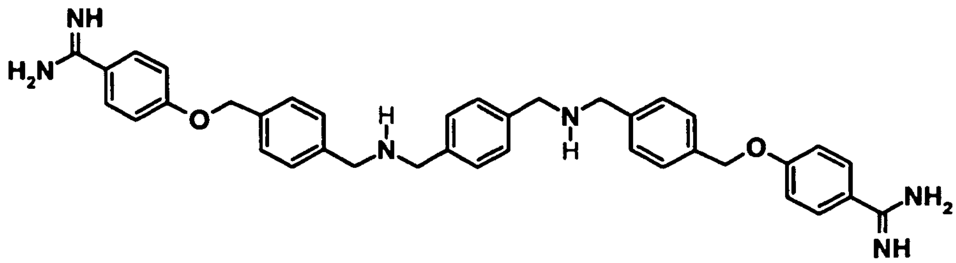

Beispiel 66 (Methode B1):

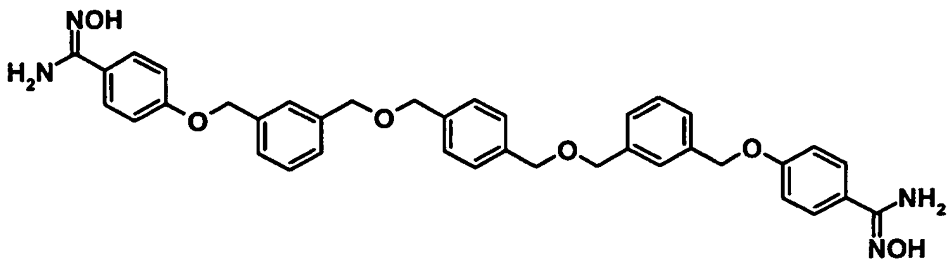

Beispiel 67 (Methode B1):

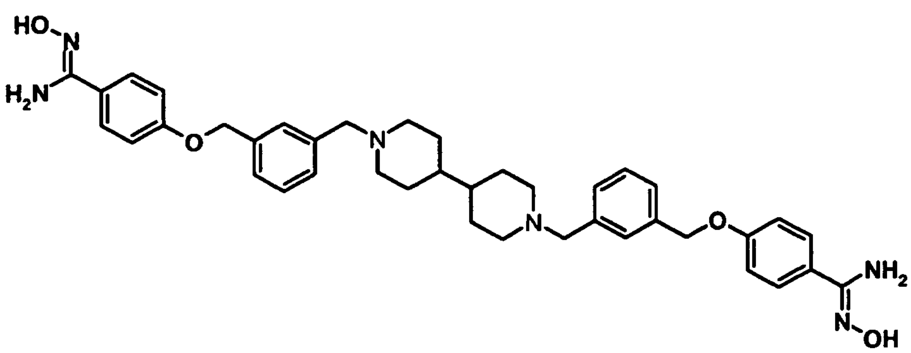

Beispiel 68 (Methode G):

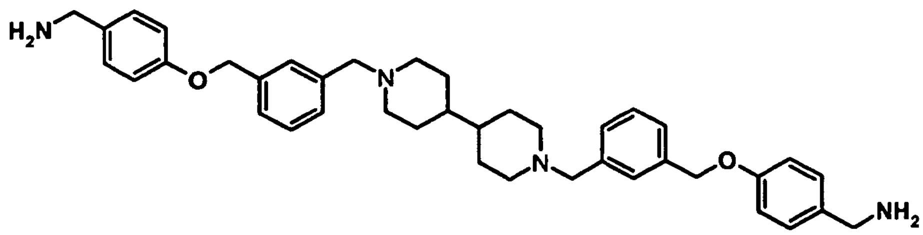

Die erfindungsgemäßen Verbindungen zeichnen sich durch ihre Tryptase-inhibierende Wirksamkeit aus. Besagte Fähigkeit, die Tryptase zu inhibieren, wurde gemäß der nachfolgenden Testbeschreibung untersucht.
Die Bestimmung wird in Tris HCl Puffer (100 mM), der zusätzlich Calcium (5 mM) und Heparin (100 mg/ml) enthält, bei pH 7.4 durchgeführt. Als Standard wird rh beta Tryptase eingesetzt, die beispielsweise von Promega käuflich zu erwerben ist. Als Substrat dient N-p-Tosyl-Gly-Pro-Lys-para-nitroanilin in einer Konzentration von 0.6 mM. Das Substrat wird durch Tryptase verdaut wobei p-Nitroanilin entsteht, das bei 405 nm gemessen werden kann. Üblicherweise wird eine Inkubationszeit von 5 Minuten und eine Inkubationstemperatur von 37°C gewählt. Als Enzymaktivität werden 0.91 U/ml eingesetzt. Die Bestimmung erfolgt in einem Autoanalyser (Cobas Bio) der Firma Hofmann LaRoche. Die potentiellen Hemmsubstanzen werden in Konzentrationen von 10 µM im Screening eingesetzt, wobei die Hemmung der Tryptase in Prozent angegeben wird. Bei über 70 % Hemmung wird die $IC_{50}$ bestimmt (Konzentration bei der 50% der Enzymaktivität gehemmt ist). Nach 5-minütiger Vorinkubation der potentiellen Hemmsubstanzen, wird das Substrat zum Starten der Reaktion zugegeben, wobei die Bildung von p-Nitroanilin nach 5 Minuten, nach Testung der Linearität, als Maß für die Enzymaktivität genommen wird.

Die sich für die erfindungsgemäßen Verbindungen ergebenden IC50-Werte sind Tabelle 1 zu entnehmen.

Tabelle 1:

| Beispiel | Salzform | $IC_{50}$-Wert [nM] |
|---|---|---|
| 2 | Diacetat | 19 |
| 7 | Tetrachlorid | 4,3 |
| 10 | Tetrachlorid | 35 |
| 11 | Tetrachlorid | 4 |
| 14 | Tetrachlorid | 13 |
| 15 | Tetrachlorid | 23 |
| 16 | Tetrachlorid | 12 |
| 17 | Tetrachlorid | 1,4 |
| 18 | Tetrachlorid | 10,7 |
| 19 | Tetrachlorid | 10 |
| 20 | Tetrachlorid | 7,1 |
| 22 | Tetrachlorid | 27,4 |
| 23 | Tetrachlorid | 1,1 |
| 24 | Tetrachlorid | 3,1 |
| 25 | Tetrachlorid | 1,2 |
| 26 | Tetrachlorid | 0,8 |

Tabelle 1: (fortgesetzt)

| Beispiel | Salzform | $IC_{50}$-Wert [nM] |
|---|---|---|
| 27 | Tetrachlorid | 1,7 |
| 28 | Tetrachlorid | 5,3 |
| 29 | Tetrachlorid | 1,7 |
| 30 | Tetrachlorid | 6,8 |
| 31 | Tetrachlorid | 13 |
| 32 | Tetrachlorid | 32,4 |
| 33 | Tetrachlorid | 2,5 |
| 34 | Tetrachlorid | 0,8 |
| 35 | Tetrachlorid | 31,6 |
| 39 | Tetrachlorid | 12 |
| 40 | Tetrachlorid | 5 |
| 41 | Tetrachlorid | 4,6 |
| 42 | Tetrachlorid | 30,9 |
| 56 | Diacetat | 19 |
| 62 | Tetrachlorid | 41 |
| 63 | Tetrachlorid | 0,74 |
| 64 | Tetrachlorid | 1,1 |
| 65 | Tetrachlorid | 0,59 |
| 68 | Tetrachlorid | 2,5 |

Die erfindungsgemäßen Tryptase-Inhibitoren können oral, transdermal, inhalativ oder parenteral verabreicht werden. Die erfindungsgemäßen Verbindungen liegen hierbei als aktive Bestandteile in üblichen Darreichungsformen vor, beispielsweise in Zusammensetzungen, die im wesentlichen aus einem inerten pharmazeutischen Träger und einer effektiven Dosis des Wirkstoffs bestehen, wie beispielsweise Tabletten, Dragees, Kapseln, Oblaten, Pulver, Lösungen, Suspensionen, Emulsionen, Sirupe, Suppositorien, transdermale Systeme etc.. Eine wirksame Dosis der erfindungsgemäßen Verbindungen liegt bei einer oralen Anwendung zwischen 1 und 100, vorzugsweise zwischen 1 und 50, besonders bevorzugt zwischen 5-30 mg/Dosis, bei intravenöser oder intramuskulärer Anwendung zwischen 0,001 und 50, vorzugsweise zwischen 0,1 und 10 mg/Dosis. Für die Inhalation sind erfindungsgemäß Lösungen geeignet, die 0,01 bis 1,0, vorzugsweise 0,1 bis 0,5 % Wirkstoff enthalten. Für die inhalative Applikation ist die Verwendung von Pulvern bevorzugt. Gleichfalls ist es möglich, die erfindungsgemäßen Verbindungen als Infusionslösung, vorzugsweise in einer physiologischen Kochsalzlösung oder Nährsalzlösung einzusetzen.

Die erfindungsgemäßen Verbindungen können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemittein, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Eine therapeutisch wirksame Tagesdosis beträgt zwischen 1 und 800 mg, bevorzugt 10 - 300 mg pro Erwachsener.

Die nachfolgenden Beispiele illustrieren die vorliegende Erfindung ohne sie jedoch in ihrem Umfang zu beschränken:

Pharmazeutische Formulierungsbeispiele

| A) | Tabletten | pro Tablette |
|---|---|---|
| | Wirkstoff | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) | Tabletten | pro Tablette |
|---|---|---|
| | Wirkstoff | 80 mg |
| | Maisstärke | 190 mg |
| | Milchzucker | 55 mg |
| | Mikrokristalline Cellulose | 35 mg |
| | Polyvinylpyrrolidon | 15 mg |
| | Natrium-carboxymethylstärke | 23 mg |
| | Magnesiumstearat | 2 mg |
| | | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natrium-carboxymethylstärke und das Magnesiumstearat, vermischt und verpreßt das Gemisch zu Tabletten geeigneter Größe.

| C) | Dragées | pro Dragée |
|---|---|---|
| | Wirkstoff | 5 mg |
| | Maisstärke | 41,5 mg |
| | Milchzucker | 30 mg |

(fortgesetzt)

| C) | Dragées | pro Dragée |
|---|---|---|
| | Polyvinylpyrrolidon<br>Magnesiumstearat | 3 mg<br>0,5 mg<br>80 mg |

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45°C und schlägt das Granulat anschließend durch dasselbe Sieb. Nach dem Zumischen von Magnesiumstearat werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichen aus Zucker und Talkum besteht. Die fertigen Dragees werden mit Wachs poliert.

| D) | Kapseln | pro Kapsel |
|---|---|---|
| | Wirkstoff<br>Maisstärke<br>Magnesiumstearat | 50 mg<br>268,5 mg<br>1,5 mg<br>320 mg |

Substanz und Maisstärke werden gemischt und mit Wasser befeuchtet. Die feuchte Masse wird gesiebt und getrocknet. Das trockene Granulat wird gesiebt und mit Magensiumstearat gemischt. Die Endmischung wird in Hartgelatinekapseln Größe 1 abgefüllt.

| E) | Ampullenlösung | |
|---|---|---|
| | Wirkstoff<br>Natriumchlorid<br>Aqua pro inj. | 50 mg<br>50 mg<br>5 ml |

Der Wirkstoff wird bei Eigen-pH oder gegebenenfalls bei pH 5,5 bis 6,5 in Wasser gelöst und mit Natriumchlorid als Isotonans versetzt, die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

| F) | Suppositorien | |
|---|---|---|
| | Wirkstoff<br>Adeps solidus | 50 mg<br>1650 mg<br>1700 mg |

Das Hartfett wird geschmolzen. Bei 40°C wird die gemahlene Wirksubstanz homogen dispergiert. Es wird auf 38°C abgekühlt und in schwach vorgekühlte Suppositorienformen ausgegossen.

**Patentansprüche**

**1.** Verbindungen der allgemeinen Formel (IA)

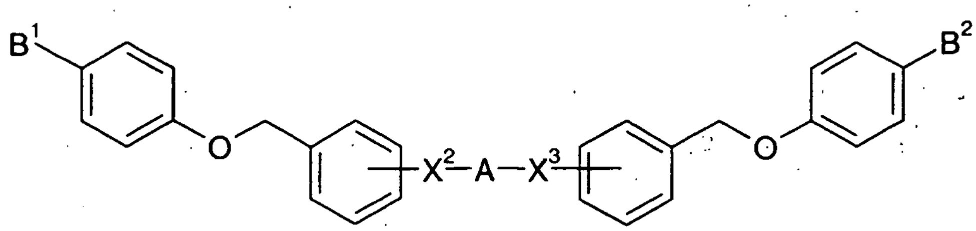

( IA )

worin

B1 und B2 gleich oder verschieden $-C(=NR^1)-NR^{1'}H$, $-CH_2NH_2$, $-CH_2CH_2NH_2$ oder $-NH-C(=NH)-NH_2$;

$R^1$ und $R^{1'}$ gleich oder verschieden Wasserstoff, OH, $-COR^2$ oder $-COOR^2$;

$R^2$ Wasserstoff, $C_1$-$C_{18}$-Alkyl, Aryl oder Aryl-$C_1$-$C_6$-Alkyl;

$X^2$ und $X^3$ gleich oder verschieden, eine Brücke ausgewählt aus der Gruppe $-(CH_2)_n-$, $-(CH_2)_nO-$, $-(CH_2)_n-S-$, $-(CH_2)_nNR^3-$ und $-(CH_2)_nN^+R^3R^4-$ mit n=1 oder 2;

A ein Rest ausgewählt aus der Gruppe $C_2$-$C_{16}$-Alkylen und $C_2$-$C_{16}$-Alkenylen, in dem gegebenenfalls ein oder mehrere Wasserstoffatome durch einen oder mehrere Reste ausgewählt aus der Gruppe F, $R^3$, $OR^3$ und $COOR^3$ ersetzt sein können,
$C_2$-$C_{16}$-Alkinylen, oder

A $-(CH_2)_l-D-(CH_2)_m-$, wobei in den Alkylengruppen $-(CH_2)_l-$ und $-(CH_2)_m-$ ein oder zwei Wasserstoffatome gegebenenfalls durch $C_1$-$C_6$-Alkyl ersetzt sein können und wobei

D Aryl oder $C_3$-$C_{10}$-Cycloalkyl, in dem gegebenenfalls ein oder mehrere Wasserstoffatome durch einen oder mehrere Reste ausgewählt aus der Gruppe F, $R^3$ und $OR^3$ ersetzt sein können und l und m, gleich oder verschieden 0, 1, 2, 3 oder 4, oder

D -O-, -S- oder $-NR^3-$
und l und m, gleich oder verschieden 2, 3 oder 4;

oder

A $-G^1-(CH_2)_r-G^2-$, falls $X^2$ oder $X^3$ $-(CH_2)_n-$ bedeuten, auch
$-E^1-(CH_2)_r-G^1-$ oder $-E^1-(CH_2)_r-E^2-$,
wobei
r eine Zahl 0, 1, 2, 3, 4, 5 oder 6,

$G^1$ und $G^2$ gleich oder verschieden eine Einfachbindung oder $C_3$-$C_{10}$-Cycloalkyl bedeuten, im Falle von r=0 oder r=1 $G^1$ und $G^2$ aber nicht gleichzeitig für eine Einfachbindung stehen können;

$E^1$ und $E^2$ gleich oder verschieden $C_3$-$C_{10}$-aza-Cycloalkyl, das ein oder zwei Stickstoffatome enthält, wobei mindestens ein N-Atom an $X^2$ oder $X^3$ = $(CH_2)_n$ gebunden ist,

bedeuten, gegebenenfalls in Form ihrer Racemate, ihrer Enantiomere, ihrer Diastereomere, Tautomere und ihrer Gemische, sowie gegebenenfalls ihrer pharmakologisch unbedenklichen Säureadditionssalze.

**2.** Verbindungen der allgemeinen Formel (IA) gemäß Anspruch 1, worin

$B^1$ und $B^2$ gleich oder verschieden $-C(=NR^1)-NR^{1'}H$, $-CH_2NH_2$, $-CH_2CH_2NH_2$ oder $-NH-C(=NH)-NH_2$;

$R^1$ und $R^{1'}$ gleich oder verschieden Wasserstoff, OH, $-COR^2$ oder $-COOR^2$;

$R^2$ Wasserstoff, $C_1$-$C_{14}$-Alkyl, Aryl oder Aryl-$C_1$-$C_6$-Alkyl;

$X^2$ und $X^3$ gleich oder verschieden, eine Brücke ausgewählt aus der Gruppe bestehend aus $-(CH_2)_n-$, $-(CH_2)_nO-$, $-(CH_2)_n-S-$, $-(CH_2)_nNR^3-$ und $-(CH_2)_nN^+R^3R^4-$ mit n=1 oder 2;

A ein Rest ausgewählt aus der Gruppe $C_2$-$C_{14}$-Alkylen und $C_2$-$C_{10}$-Alkenylen, in dem gegebenenfalls ein oder mehrere Wasserstoffatome durch einen oder mehrere Reste ausgewählt aus der Gruppe F, $R^3$, $OR^3$ und $COOR^3$ ersetzt sein können, $C_2$-$C_{10}$-Alkinylen, oder

A $-(CH_2)_l-D-(CH_2)_m-$, wobei in den Alkylengruppen $-(CH_2)_l-$ und $-(CH_2)_m-$ ein oder zwei Wasserstoffatome gegebenenfalls durch $C_1$-$C_6$-Alkyl ersetzt sein können und wobei

D Aryl oder $C_3$-$C_8$-Cycloalkyl, in dem gegebenenfalls ein oder mehrere Wasserstoffatome durch einen oder mehrere Reste ausgewählt aus der Gruppe F, $R^3$ und $OR^3$ ersetzt sein können und l und m, gleich oder verschieden 0, 1, 2, 3 oder 4, oder

D -O-, -S- oder $-NR^3-$ und l und m, gleich oder verschieden 2, 3 oder 4;

oder

A $-G^1-(CH_2)_r-G^2-$,
falls $X^2$ oder $X^3$ $-(CH_2)_n-$ bedeuten auch
$-E^1-(CH_2)_r-G^1-$ oder $-E^1-(CH_2)_r-E^2-$,
wobei
r eine Zahl 0, 1, 2, 3, 4, 5 oder 6,

$G^1$ und $G^2$ gleich oder verschieden eine Einfachbindung oder $C_3$-$C_8$-Cycloalkyl bedeuten, im Falle von r=0 oder r=1 $G^1$ und $G^2$ aber nicht gleichzeitig für eine Eirifachbindurig stehen können;

$E^1$ und $E^2$ gleich oder verschieden $C_3$-$C_8$-aza-Cycloalkyl, das ein oder zwei Stickstoffatome enthält, wobei mindestens ein N-Atom an $X^2$ oder $X^3 = (CH_2)_n$ gebunden ist,

bedeuten, gegebenenfalls in Form ihrer Racemate, ihrer Enantiomere, ihrer Diastereomere, Tautomere und ihrer Gemische, sowie gegebenenfalls ihrer pharmakologisch unbedenklichen Säureadditionssalze.

**3.** Verbindungen der allgemeinen Formel (IA) gemäß einem der Ansprüche 1 oder 2, worin

$B^1$ und $B^2$ gleich oder verschieden $-C(=NR^1)-NR^{1'}H$, $-CH_2NH_2$, $-CH_2CH_2NH_2$ oder $-NH-C(=NH)-NH_2$;

$R^1$ und $R^{1'}$ gleich oder verschieden Wasserstoff, OH, $-COR^2$ oder $-COOR^2$;

$R^2$ Wasserstoff, $C_1$-$C_{10}$-Alkyl oder Aryl-$C_1$-$C_4$-Alkyl;

$X^2$ und $X^3$ gleich oder verschieden, eine Brücke ausgewählt aus der Gruppe bestehend aus $-(CH_2)_n-$, $-(CH_2)_nO-$, $-(CH_2)_n-S-$, $-(CH_2)_nNR^3-$ und $-(CH_2)_nN^+R^3R^4-$ mit n=1 oder 2;

A $C_2$-$C_{12}$-Alkylen, in dem gegebenenfalls ein oder mehrere Wasserstoffatome durch einen oder mehrere Reste ausgewählt aus der Gruppe F, $OR^3$ und $COOR^3$ ersetzt sein können, oder

A $-(CH_2)_l-D-(CH_2)_m-$, wobei in den Alkylengruppen $-(CH_2)_l-$ und $-(CH_2)_m-$ ein oder zwei Wasserstoffatome gegebenenfalls durch eine $C_1$-$C_4$-Gruppe erstetzt sein können und wobei

D ein Rest ausgewählt aus der Gruppe bestehend aus Phenyl, Cyclopentyl und Cyclohexyl, in dem gegebenenfalls ein oder mehrere Wasserstoffatome durch einen oder mehrere Reste ausgewählt aus der Gruppe F, $R^3$ und $OR^3$ ersetzt sein können und l und m, gleich oder verschieden 0, 1, 2, 3 oder 4 bedeuten, oder

D -O- oder $-NR^3-$
und l und m, gleich oder verschieden 2, 3 oder 4;

oder

A $-G^1-(CH_2)_r-G^2-$,
falls $X^2$ oder $X^3$ $-(CH_2)_n-$ bedeuten auch
$-E^1-(CH_2)_r-G^1-$ oder $-E^1-(CH_2)_r-E^2-$,
wobei
r eine Zahl 0, 1, 2, 3 oder 4,

$G^1$ und $G^2$ gleich oder verschieden eine Einfachbindung, Cyclopentyl, Cyclohexyl oder Cycloheptyl bedeuten, im Falle von r=0 oder r=1 $G^1$ und $G^2$ aber nicht gleichzeitig für eine Einfachbindung stehen können;
$E^1$ und $E^2$ gleich oder verschieden ein Rest ausgewählt aus der Gruppe bestehend aus Pyrrolidin, Imidazolidin, Piperidin und Piperazin,

wobei mindestens ein N-Atom an $X^2$ oder $X^3$ = $(CH_2)_n$ gebunden ist, bedeuten, gegebenenfalls in Form ihrer Racemate, ihrer Enantiomere, ihrer Diastereomere, Tautomere und ihrer Gemische, sowie gegebenenfalls ihrer pharmakologisch unbedenklichen Säureadditionssalze.

**4.** Verbindungen der allgemeinen Formel (IA) gemäß einem der Ansprüche 1, 2 oder 3, worin

$B^1$ und $B^2$ gleich oder verschieden $-C(=NR^1)-NR^{1'}H$, $-CH_2NH_2$, $-CH_2CH_2NH_2$ oder $-NH-C(=NH)-NH_2$;

$R^1$ und $R^{1'}$ gleich oder verschieden Wasserstoff, OH, $-COR^2$ oder $-COOR^2$,

$R^2$ Wasserstoff, $C_1$-$C_6$-Alkyl oder Benzyl;

$X^2$ und $X^3$ gleich oder verschieden, eine Brücke ausgewählt aus der Gruppe bestehend aus $-(CH_2)_n-$, $-(CH_2)_nO-$, $-(CH_2)_n-S-$, $-(CH_2)_nNR^3-$ und $-(CH_2)_nN^+R^3R^4-$ mit n=1 oder 2;

A $C_2$-$C_{12}$-Alkylen, in dem gegebenenfalls ein oder mehrere Wasserstoffatome durch einen oder mehrere Reste ausgewählt aus der Gruppe F, $OR^3$ und $COOR^3$ ersetzt sein können, oder

A $-(CH_2)_l-D-(CH_2)_m-$, wobei in den Alkylengruppen $-(CH_2)_l-$ und $-(CH_2)_m-$ ein oder zwei Wasserstoffatome gegebenenfalls durch eine Methyl-Gruppe erstetzt sein können und wobei

D ein Rest ausgewählt aus der Gruppe bestehend aus Phenyl und Cyclohexyl, in dem gegebenenfalls ein oder mehrere Wasserstoffatome durch einen oder mehrere Reste ausgewählt aus der Gruppe F, $R^3$ und $OR^3$ ersetzt sein können
und l und m, gleich oder verschieden 0, 1, 2 oder 3 bedeuten, oder
D für -O und l und m, gleich oder verschieden für 2 oder 3 stehen;

oder

A $-G^1-(CH_2)_r-G^2-$,
falls $X^2$ oder $X^3$ $-(CH_2)_n-$ bedeuten auch
$-E^1-(CH_2)_r-G^1-$ oder $-E^1-(CH_2)_r-E^2-$,
wobei
r eine Zahl 0, 1, 2 oder 3,

$G^1$ und $G^2$ gleich oder verschieden eine Einfachbindung, Cyclopentyl oder Cyclohexyl bedeuten, im Falle von r=0 oder r=1 $G^1$ und $G^2$ aber nicht gleichzeitig für eine Einfachbindung stehen können;
$E^1$ und $E^2$ gleich oder verschieden Piperidin oder Piperazin, wobei mindestens ein N-Atom an $X^2$ oder $X^3$ = $(CH_2)_n$ gebunden ist,

bedeuten, gegebenenfalls in Form ihrer Racemate, ihrer Enantiomere, ihrer Diastereomere, Tautomere und ihrer Gemische, sowie gegebenenfalls ihrer pharmakologisch unbedenklichen Säureadditionssalze.

**5.** Verbindungen der allgemeinen Formel (IA) gemäß einem der Ansprüche 1 bis 4, worin

$B^1$ und $B^2$ gleich oder verschieden $-C(=NR^1)-NH_2$, $-CH_2NH_2$ oder $-CH_2CH_2NH_2$;

$R^1$ Wasserstoff, OH, $-COR^2$ oder $-COOR^2$,

$R^2$ Wasserstoff, Methyl, Ethyl, Propyl, Butyl, Pentyl, Hexyl oder Benzyl;

$X^2$ und $X^3$ $X^4$ gleich oder verschieden, eine Brücke ausgewählt aus der Gruppe bestehend aus $-(CH_2)_n-$, $-(CH_2)_nO-$, $-(CH_2)_nNH-$, $-(CH_2)_nNMe-$, $-(CH_2)_nNEt-$, $-(CH_2)_nNProp-$, $-(CH_2)_nNCyclopropy-$, $-(CH_2)_nNBu-$ und $-(CH_2)_nN^+(Me)_2-$ mit n=1;

A $C_2$-$C_{12}$-Alkylen, das gegebenenfalls durch einen Rest ausgewählt aus der Gruppe OH, COOH und COOMe substituiert sein kann, oder
$-(CH_2)_l-D-(CH_2)_m-$, wobei in den Alkylengruppen $-(CH_2)_l-$ und $-(CH_2)_m-$ ein oder zwei Wasserstoffatome gegebenenfalls durch Methyl erstetzt sein können und wobei

D Phenyl oder Cyclohexyl, das gegebenenfalls durch Methyl substituiert sein kann und I und m, gleich oder verschieden 0, 1, 2 oder 3 bedeuten,
oder
D für -O- und I und m, gleich oder verschieden für 2 oder 3 stehen;

oder

A $-G^1-(CH_2)_r-G^2-$,
falls $X^2$ oder $X^3$ $-(CH_2)_n-$ bedeuten auch
$-E^1-(CH_2)_r-G^1-$ oder $-E^1-(CH_2)_r-E^2-$,
wobei
r eine Zahl 0, 1, 2 oder 3,

$G^1$ und $G^2$ gleich oder verschieden eine Einfachbindung oder Cyclohexyl bedeuten, im Falle von r=0 oder r=1 $G^1$ und $G^2$ aber nicht gleichzeitig für eine Einfachbindung stehen können;

$E^1$ und $E^2$ gleich oder verschieden Piperidin oder Piperazin, wobei mindestens ein N-Atom an $X^2$ oder $X^3$ = $(CH_2)_n$ gebunden ist,

bedeuten, gegebenenfalls in Form ihrer Racemate, ihrer Enantiomere, ihrer Diastereomere, Tautomere und ihrer Gemische, sowie gegebenenfalls ihrer pharmakologisch unbedenklichen Säureadditionssalze.

**6.** Verbindungen der allgemeinen Formel (IA) gemäß einem der Ansprüche 1 bis 5, worin die Gruppierung

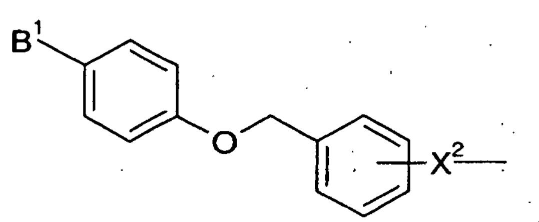

für einen Rest ausgewählt aus der Gruppe bestehend aus

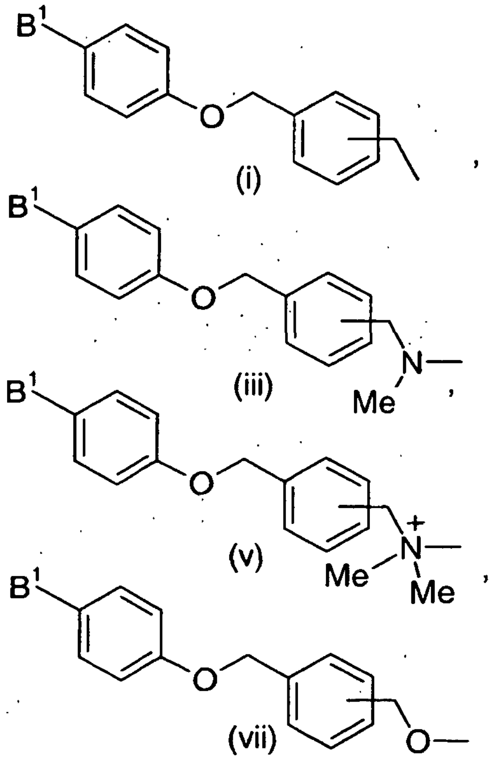

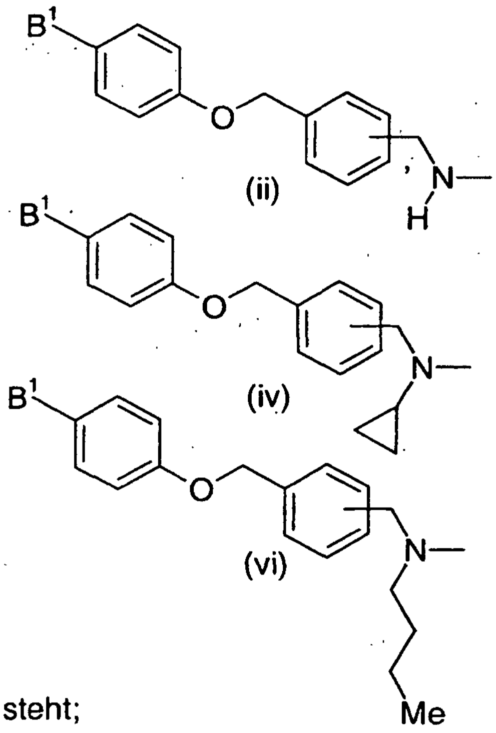

und

steht;

die Gruppierung -

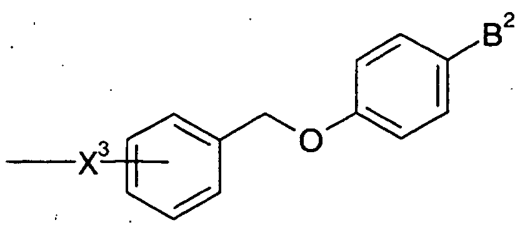

für einen Rest ausgewählt aus der Gruppe bestehend aus

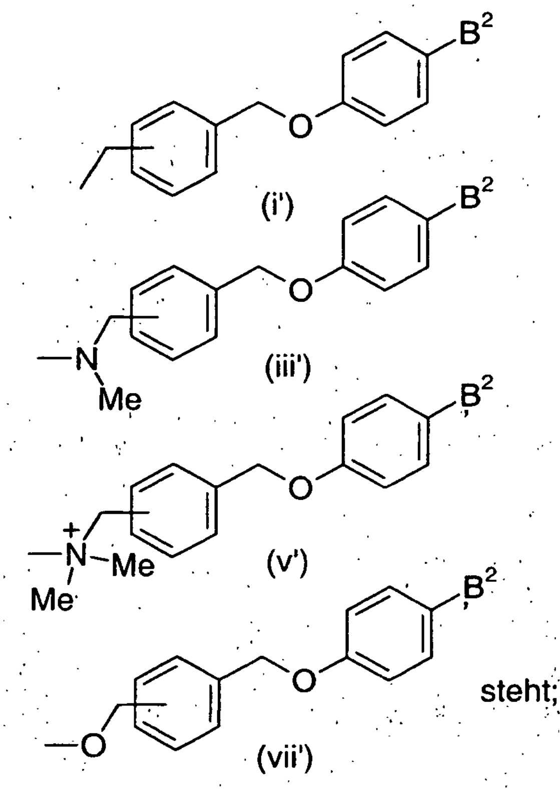

(i'), (iii'), (v'), (vii') steht;

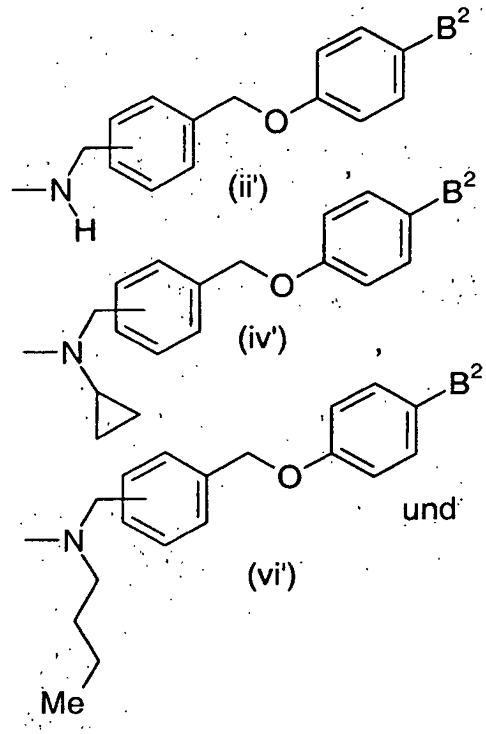

(ii'), (iv'), (vi') und

wobei

B1 und B2 gleich oder verschieden $-C(=NR^1)-NR^{1'}H$, $-CH_2NH_2$, $-CH_2CH_2NH_2$ oder $-NH-C(=NH)-NH_2$;

R1 und R1' gleich oder verschieden Wasserstoff oder OH;

A für einen verbrückenden Rest steht, der ausgewählt ist aus der Gruppe bestehend aus (viii) $C_4$-$C_{10}$-Alkylen, das gegebenenfalls durch COOH substituiert sein kann,

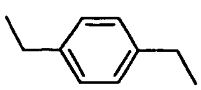

(ix),

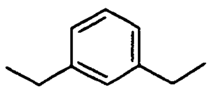

(x),

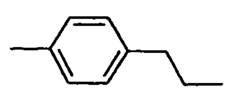

(xi),

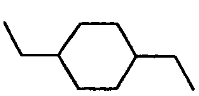

(xii),

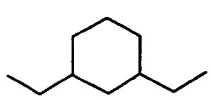

(xiii),

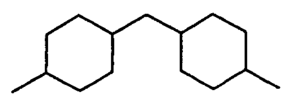

(xiv),

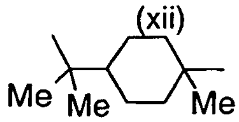

(xv),

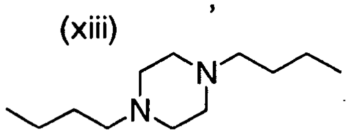

(xvi) und

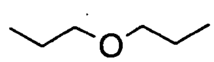

(xvii);

oder

A ferner

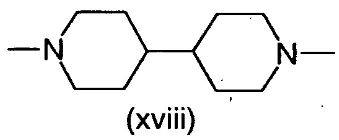

(xviii)

bedeutet, wenn die Gruppierung

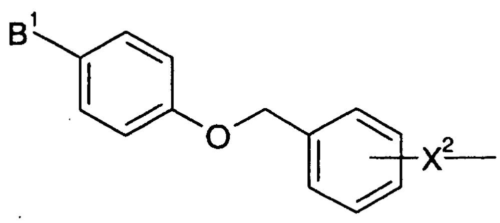

für die Gruppe

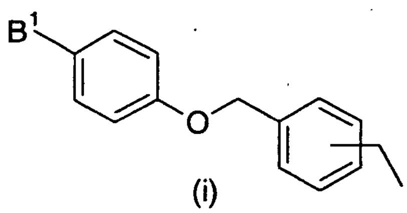

(i)

und die Gruppierung

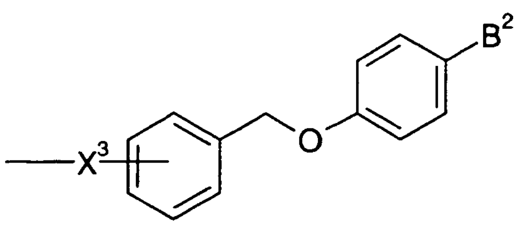

für den Rest

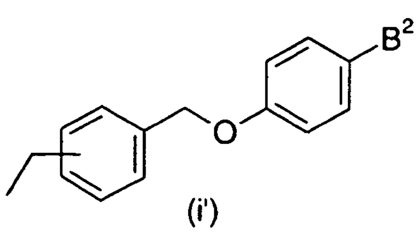

(i')

steht, oder

A ferner

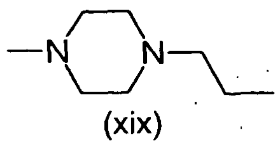

(xix)

sein kann, wenn die Gruppierung der beiden Reste

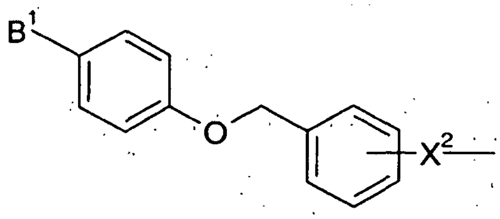

und

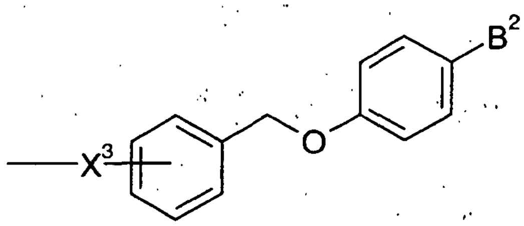

für den Rest (i) bzw. (i') steht, die direkt an den Piperazin-Stickstoff der Gruppe A angebunden ist,

bedeuten, gegebenenfalls in Form ihrer Racemate, ihrer Enantiomere, ihrer Diastereomere, Tautomere und ihrer Gemische, sowie gegebenenfalls ihrer pharmakologisch unbedenklichen Säureadditionssalze.

**7.** Verbindungen der allgemeinen Formel (IA) gemäß einem der Ansprüche 1 bis 6, worin die Gruppierung -

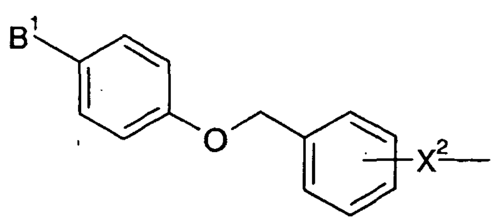

für einen Rest ausgewählt aus der Gruppe bestehend aus

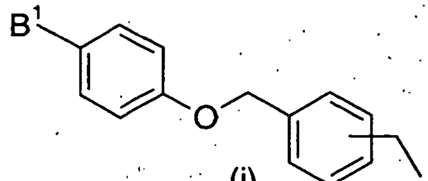

(i) ,

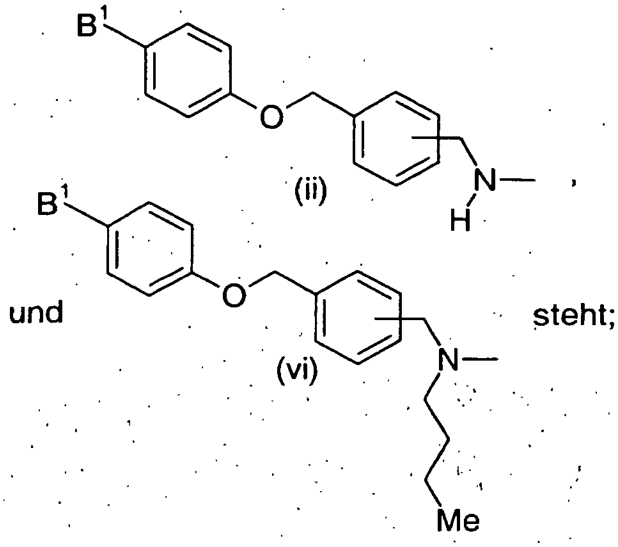

(ii) ,

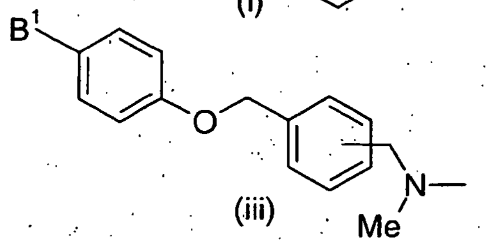

(iii) und (vi) steht;

die Gruppierung

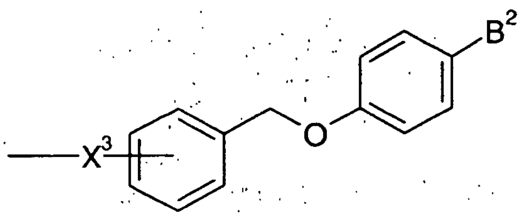

für einen Rest ausgewählt aus der Gruppe bestehend aus

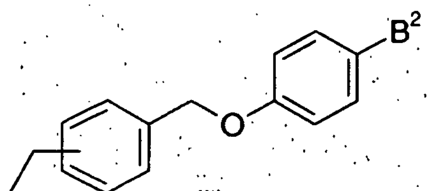

(i'),

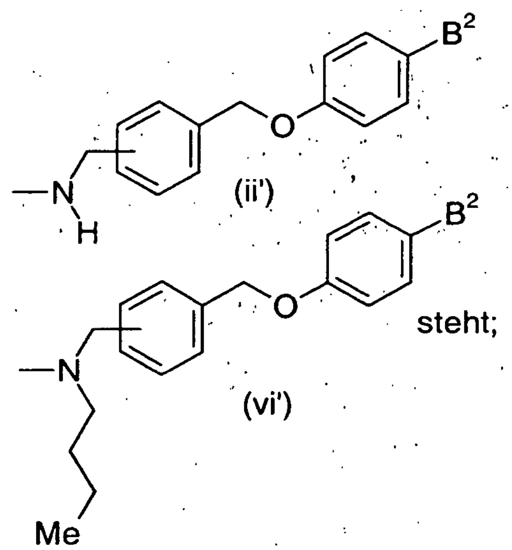

(ii') ,

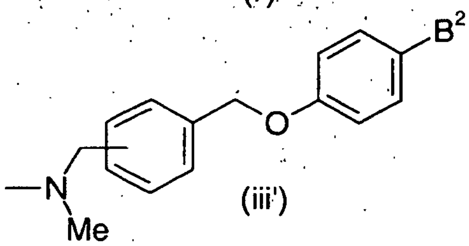

(iii') und (vi') steht;

wobei

$B^1$ und $B^2$ gleich oder verschieden $-C(=NR^1)-NR^{1'}H$ oder $-CH_2NH_2$,

$R^1$ und $R^{1'}$ gleich oder verschieden Wasserstoff oder OH;

A für einen verbrückenden Rest steht, der ausgewählt ist aus der Gruppe bestehend aus

(viii) $C_4$-$C_{10}$-Alkylen,

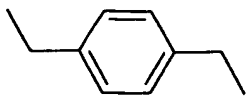 ,

(ix)

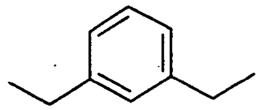 ,

(x)

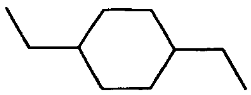 ,

(xii)

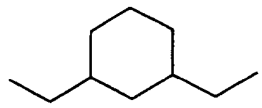

(xiii)

und

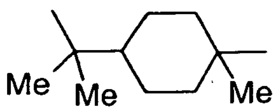

(xv)

oder

A ferner

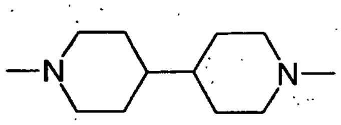

(xviii)

bedeutet, wenn die Gruppierung

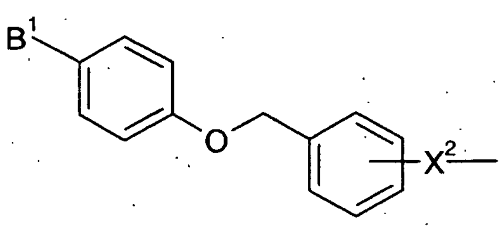

für die Gruppe (i)

und die Gruppierung

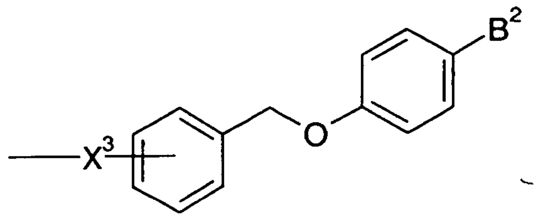

für den Rest (i') steht,
bedeuten, gegebenenfalls in Form ihrer Racemate, ihrer Enantiomere, ihrer Diastereomere, Tautomere und ihrer Gemische, sowie gegebenenfalls ihrer pharmakologisch unbedenklichen Säureadditionssalze.

**8.** Verbindungen der allgemeinen Formel (IA1)

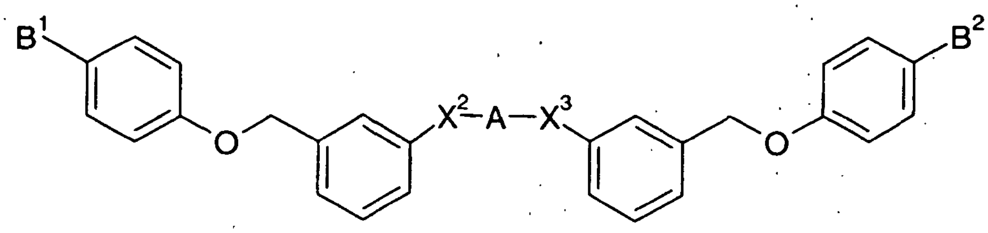

(IA1)

worin $B^1$, $B^2$, A, $X^2$ und $X^3$ die in den Ansprüchen 1 bis 7 angegebenen Bedeutungen aufweisen, gegebenenfalls in Form ihrer Racemate, ihrer Enantiomere, ihrer Diastereomere, Tautomere und ihrer Gemische, sowie gegebenenfalls ihrer pharmakologisch unbedenklichen Säureadditionssalze.

**9.** Verbindungen der allgemeinen Formel (IA) oder (IA1) nach einem der Ansprüche 1 bis 8, worin $-X^2-A-X^3-$ eine Gruppe ausgewählt aus den Formeln

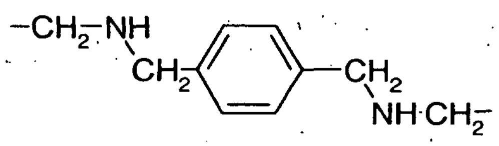

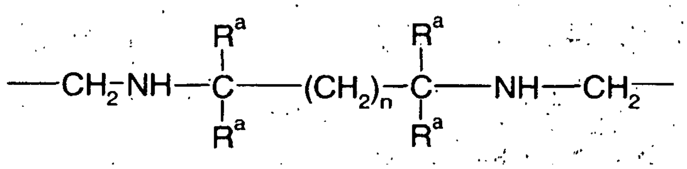

worin Ra für Wasserstoff oder Methyl steht und n 3, 4, 5 oder 6 ist,

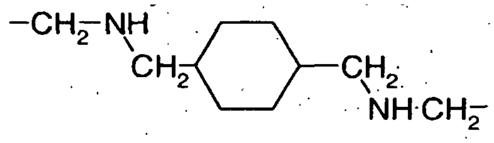

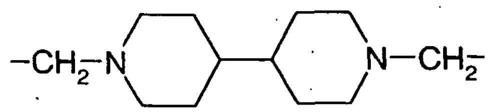

bedeutet.

**10.** Verbindungen der allgemeinen Formel (IA) oder (IA1) nach einem der Ansprüche 1 bis 9 ausgewählt aus der Gruppe bestehend aus:

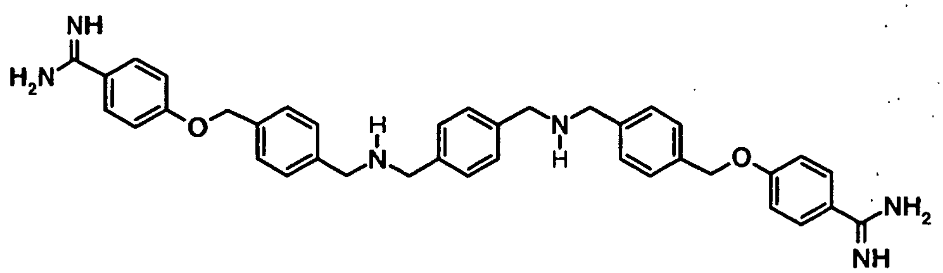

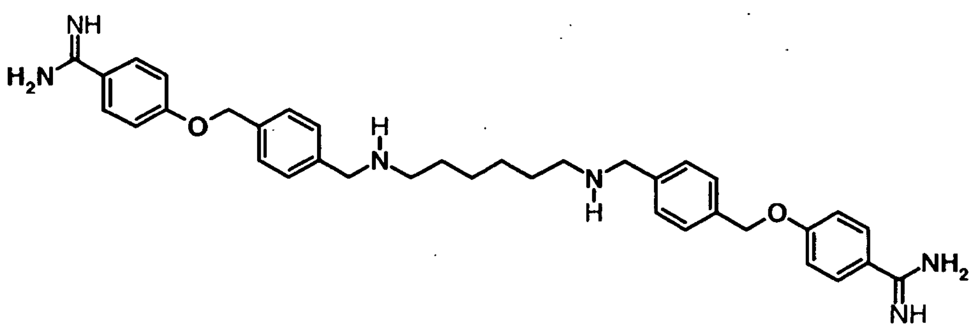

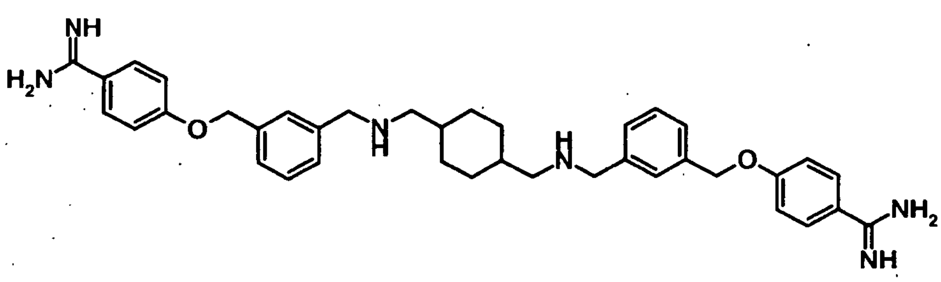

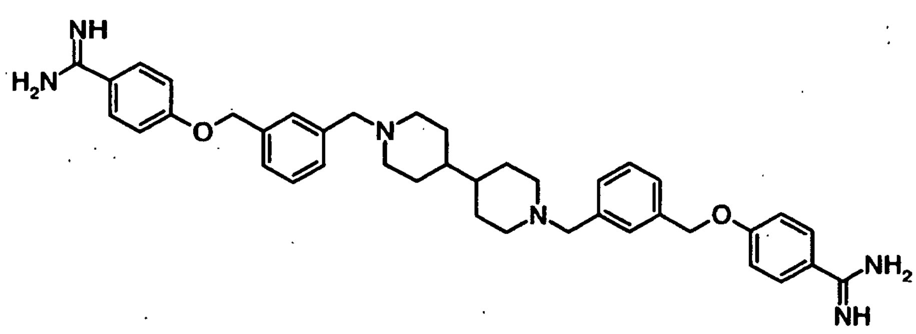

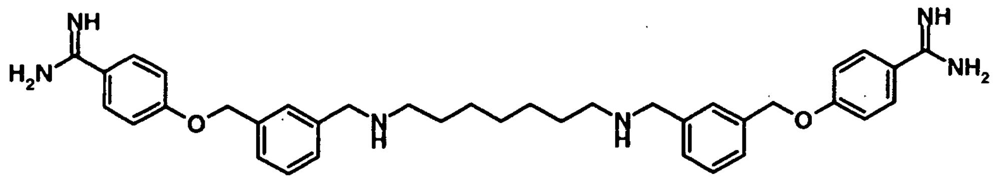

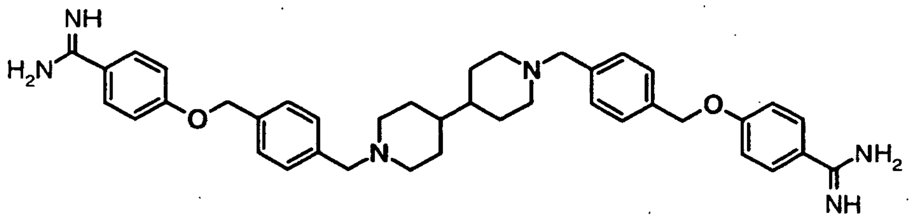

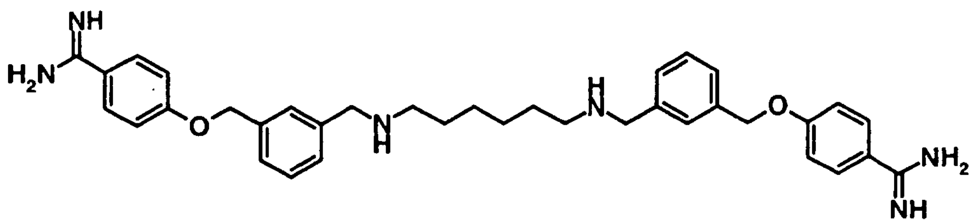

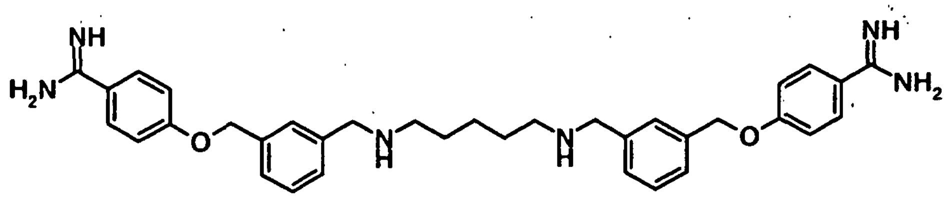

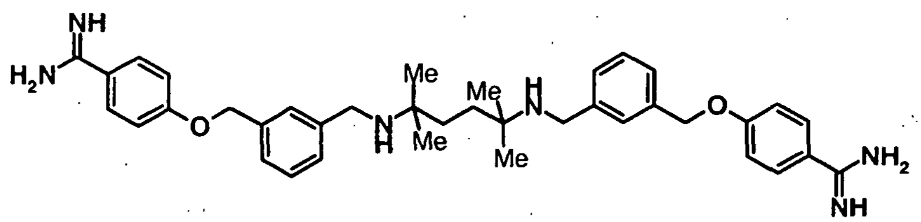

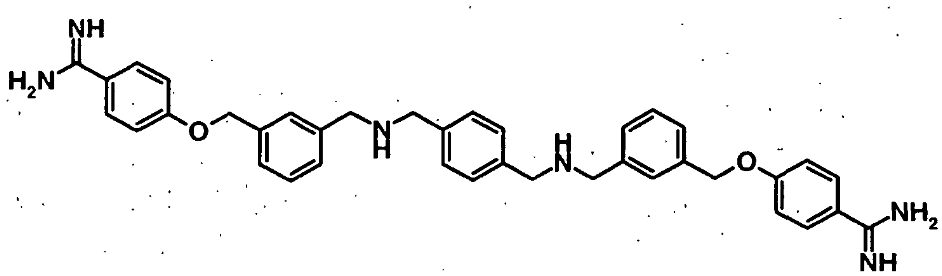

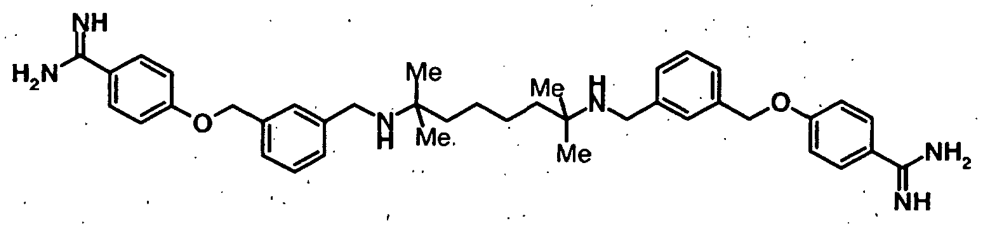

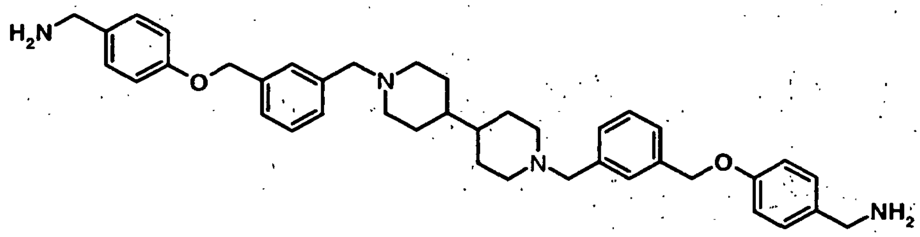

**11.** Verwendung von Verbindungen der allgemeinen Formel (IA) oder (IA1) gemäß einem der Ansprüche 1-10 zur Herstellung eines Arzneimittels zur Vorbeugung und/oder Behandlung von Krankheiten, in denen Tryptase-Inhibitoren einen therapeutischen Nutzen entfalten können.

**12.** Pharmazeutische Zusammensetzung **gekennzeichnet durch** einen Gehalt einer oder mehrer Verbindungen gemäß einem der Ansprüche 1-10.

**13.** Verfahren zur Herstellung der Verbindungen der allgemeinen Formel (IA)

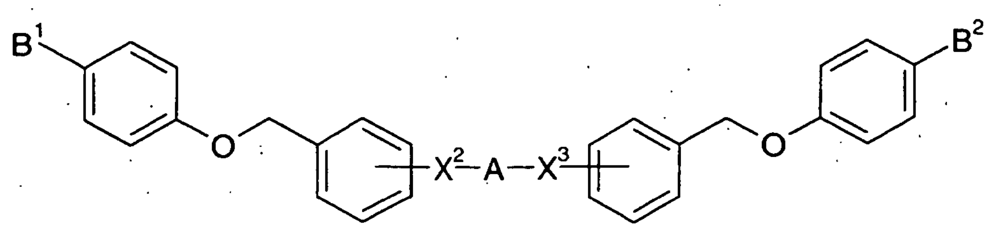

(IA)

in denen $B^1$ und $B^2$ $-C(=NH)-NH_2$ bedeuten und die Gruppen $X^2$, A, $X^3$, die in den Ansprüchen 1-10 genannten Bedeutungen aufweisen können, **dadurch gekennzeichnet**,

(a) daß man Imidoester der allgerrieinen Formel (IIA)

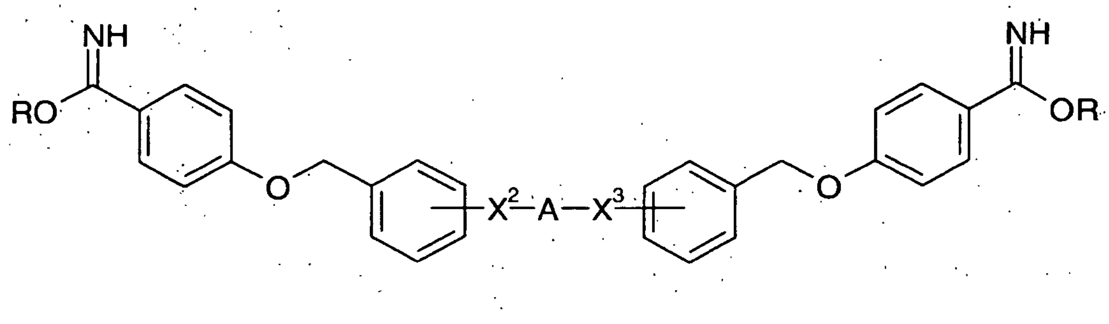

( IIA )

in denen R für $C_1$-$C_6$-Alkyl steht und die Gruppen $X^2$, A; $X^3$, die in den Ansprüchen 1-10 genannten Bedeutungen aufweisen können, mit Ammoniak umsetzt; oder
(b) daß man Verbindungen der allgemeinen Formel (IA) in denen $B^1$ und $B^2$ -C(=NOH)-$NH_2$ und die Gruppen $X^2$, A, $X^3$, die in den Ansprüchen 1-10 genannten Bedeutungen aufweisen können, reduziert.

**14.** Verfahren zur Herstellung von Verbindungen der allgemeinen Formel (IA)

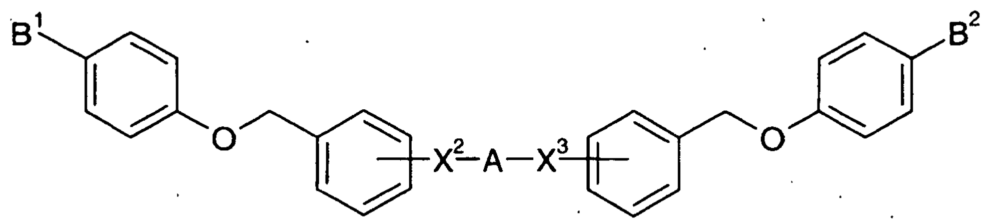

( IA )

in denen $B^1$ und $B^2$ -C(=NOH)-$NH_2$ bedeuten und die Gruppen $X^2$, A, $X^3$ die in den Ansprüchen 1-10 genannten Bedeutungen aufweisen können, **dadurch gekennzeichnet,**
**daß** Verbindungen der allgemeinen Formel (IIIA)

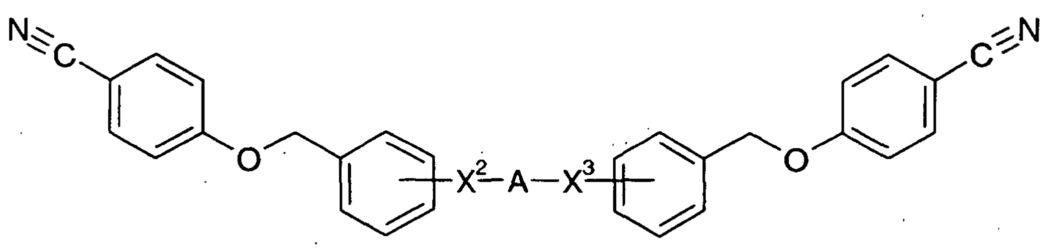

( IIIA )

in denen die Gruppen $X^2$, A, $X^3$ die in den Ansprüchen 1-10 genannten Bedeutungen aufweisen können, mit Hydroxylamin behandelt werden.

**15.** Verfahren zur Herstellung der Verbindungen der allgemeinen Formel (IA)

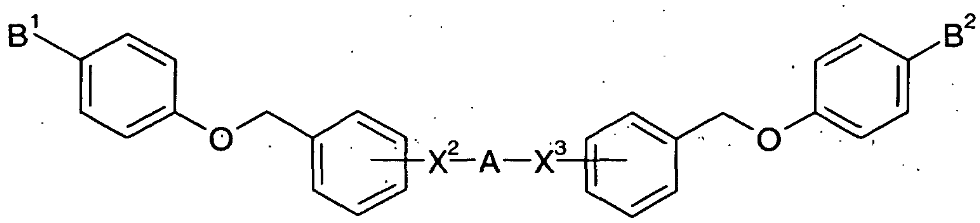

( IA )

(a) in denen $B^1$ und $B^2$ und die Gruppen $X^2$, A, $X^3$ die in den Ansprüchen 1-10 genannten Bedeutungen haben können, **dadurch gekennzeichnet, daß** man Verbindungen der allgemeinen Formeln (IVA) und (VA)

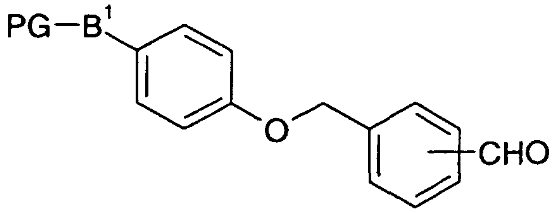

( IVA )

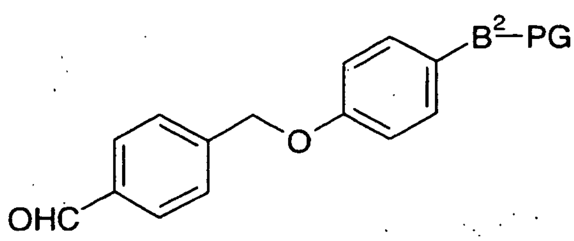

( VA )

in denen PG eine zum Schutz von Aminen geeignete Schutzgruppe darstellen kann, mit einem Diamin unter anschließender Reduktion der **dadurch** gebildeten C=N-Doppelbindungen umsetzt und abschließend die Schutzgruppen PG auf üblichem Wege absoaltet; oder

(b) daß man Verbindungen der allgemeinen Formel (VIA) und (VIIA)

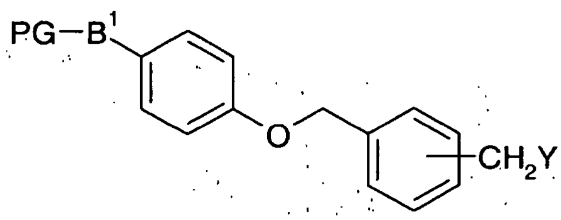

( VIA )

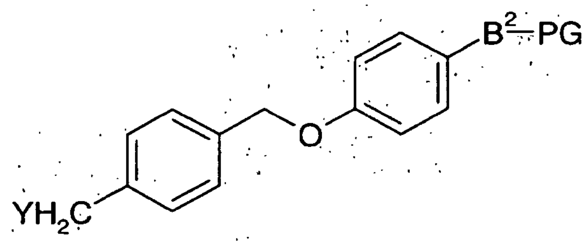

( VIIA )

in denen PG jeweils unabhängig voneinander eine zum Schutz von Aminen geeignete Schutzgruppe darstellen kann, und Y jeweils unabhängig voneinander für einen Rest ausgewählt aus der Gruppe bestehend aus Fluor, Chlor, Brom, Jod, $C_1$-$C_4$-Alkyl und Arylsulfonat steht, mit einem Diamin oder einem Dialkohol umgesetzt und abschließend die Schutzgruppen PG auf üblichem Wege abspaltet.

**16.** Verfahren zur Herstellung der Verbindungen der allgemeinen Formel (IA)

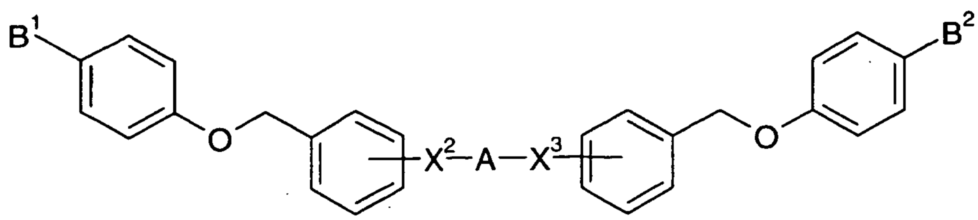

( IA )

in denen $B^1$ und $B^2$ -C(=$NR^1$)-$NH_2$ bedeuten mit $R^1 \neq H$ und die Gruppen $X^2$, A, $X^3$ die in den Ansprüchen 1-10 genannten Bedeutungen haben können, **dadurch gekennzeichnet,**
**daß** man Verbindungen der allgemeinen Formel (IA), in denen $B^1$ und $B^2$ -C(=NH)-$NH_2$ bedeuten, mit Chlorameisensäureestern oder Acylhalogeniden bzw. entsprechenden Anhydriden umsetzt.

**17.** Verfahren zur Herstellung der Verbindungen der allgemeinen Formel (IA)

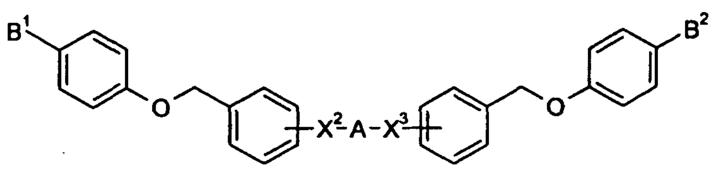

( IA )

in denen $B^1$ und $B^2$ -$CH_2$-$NH_2$ bedeuten und die Gruppen $X^2$, A, $X^3$ die in den Ansprüchen 1-10 genannten Bedeutungen haben können, **dadurch gekennzeichnet,**
**daß** man die entsprechenden Nitril-Verbindungen der allgemeinen Formel (III)

$$NC-Ar^1-X^1-Ar^2-X^2-A-X^3-Ar^3-X^4-Ar^4-CN \quad (III)$$

worin

| | |
|---|---|
| $Ar^1$, $Ar^2$, $Ar^3$ und $Ar^4$ | Phenyl; |
| $-X^1-$ | $-O-CH_2-$; |
| $-X^4-$ | $-CH_2-O-$bedeuten, |

reduziert.

## Claims

**1.** Compounds of general formula (IA)

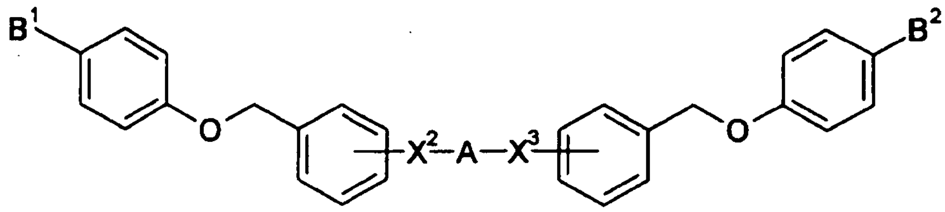

( IA )

wherein

$B^1$ and $B^2$ which may be identical or different denote $-C(=NR^1)-NR^{1'}H$, $-CH_2NH_2$, $-CH_2CH_2NH_2$ or $-NH-C(=NH)-NH_2$;

$R^1$ and $R^{1'}$ which may be identical or different denote hydrogen, OH, $-COR^2$ or $-COOR^2$;

$R^2$ denotes hydrogen, $C_1$-$C_{18}$-alkyl, aryl or aryl-$C_1$-$C_6$-alkyl;

$X^2$ and $X^3$ which may be identical or different denote a bridge selected from among $-(CH_2)_n-$, $-(CH_2)_nO-$, $-(CH_2)_n-S-$, $-(CH_2)_nNR^3-$ and $-(CH_2)_nN^+R^3R^4-$ where n=1 or 2;

A denotes a group selected from among $C_2$-$C_{16}$-alkylene and $C_2$-$C_{16}$-alkenylene, wherein optionally one or more hydrogen atoms may be replaced by one or more groups selected from among F, $R^3$, $OR^3$ and $COOR^3$, or A denotes $C_2$-$C_{16}$-alkynylene, or

A denotes $-(CH_2)_l-D-(CH_2)_m-$, whilst in the alkylene groups $-(CH_2)_l-$ and $-(CH_2)_m-$ one or two hydrogen atoms may optionally be replaced by $C_1$-$C_6$-alkyl and wherein

D denotes aryl or $C_3$-$C_{10}$-cycloalkyl wherein one or more hydrogen atoms may optionally be replaced by one or more groups selected from among F, $R^3$ and $OR^3$ and l and m, which may be identical or different, denote 0, 1, 2, 3 or 4, or

D denotes -O-, -S- or $-NR^3-$ and l and m, which may be identical or different, denote 2, 3 or 4;

or

A denotes $-G^1-(CH_2)_r-G^2-$, if $X^2$ or $X^3$ denote $-(CH_2)_n-$, A may also denote $-E^1-(CH_2)_r-G^1-$ or $-E^1-(CH_2)_r-E^2-$,
wherein
r denotes the number 0, 1, 2, 3, 4, 5 or 6,

$G^1$ and $G^2$ which may be identical or different denote a single bond or $C_3$-$C_{10}$-cycloalkyl, but if r=0 or r=1 $G^1$ and $G^2$ cannot simultaneously represent a single bond;

$E^1$ and $E^2$ which may be identical or different denote $C_3$-$C_{10}$-aza-cycloalkyl which contains one or two nitrogen atoms, wherein at least one N-atom is bound to $X^2$ or $X^3$ = $(CH_2)_n$,

optionally in the form of their racemates, enantiomers, diastereomers, tautomers and mixtures thereof, and optionally the pharmacologically acceptable acid addition salts thereof.

**2.** Compounds of general formula (IA) according to claim 1, wherein

$B^1$ and $B^2$ which may be identical or different denote $-C(=NR^1)-NR^{1'}H$, $-CH_2NH_2$, $-CH_2CH_2NH_2$ or $-NH-C(=NH)-NH_2$;

$R^1$ and $R^{1'}$ which may be identical or different denote hydrogen, OH, $-COR^2$ or $-COOR^2$;

$R^2$ denotes hydrogen, $C_1$-$C_{14}$-alkyl, aryl or aryl-$C_1$-$C_6$-alkyl;

$X^2$ and $X^3$ which may be identical or different denote a bridge selected from among $-(CH_2)_n-$, $-(CH_2)_nO-$, $-(CH_2)_n-S-$, $-(CH_2)_nNR^3-$ and $-(CH_2)_nN^+R^3R^4-$ where n=1 or 2;

A denotes a group selected from among $C_2$-$C_{14}$-alkylene and $C_2$-$C_{10}$-alkenylene, wherein optionally one or more hydrogen atoms may be replaced by one or more groups selected from among F, $R^3$, $OR^3$ and $COOR^3$, or A denotes $C_2$-$C_{10}$-alkynylene, or

A denotes $-(CH_2)_l-D-(CH_2)_m-$, whilst in the alkylene groups $-(CH_2)_l-$ and $-(CH_2)_m-$ one or two hydrogen atoms may optionally be replaced by $C_1$-$C_6$-alkyl and wherein

D denotes aryl or $C_3$-$C_8$-cycloalkyl wherein one or more hydrogen atoms may optionally be replaced by one or more groups selected from among F, $R^3$ and $OR^3$
and l and m, which may be identical or different, denote 0, 1, 2, 3 or 4, or

D denotes -O-, -S- or $NR^3$-
and l and m, which may be identical or different, denote 2, 3 or 4;

or

A denotes $-G^1-(CH_2)_r-G^2-$,
if $X^2$ or $X^3$ represents $-(CH_2)_n-$ it also denotes
$-E^1-(CH_2)_r-G^1-$ or $-E^1-(CH_2)_r-E^2-$,
wherein
r denotes the number 0, 1, 2, 3, 4, 5 or 6,

$G^1$ and $G^2$ which may be identical or different denote a single bond or $C_3$-$C_8$-cycloalkyl, but if r=0 or r=1 $G^1$ and $G^2$ cannot simultaneously represent a single bond;
$E^1$ and $E^2$ which may be identical or different denote $C_3$-$C_8$-aza-cycloalkyl which contains one or two nitrogen atoms, wherein at least one N-atom is bound to $X^2$ or $X^3$ = $(CH_2)_n$,

optionally in the form of their racemates, enantiomers, diastereomers, tautomers and mixtures thereof, and optionally the pharmacologically acceptable acid addition salts thereof.

**3.** Compounds of general formula (IA) according to one of claims 1 or 2, wherein

$B^1$ and $B^2$ which may be identical or different denote $-C(=NR^1)-NR^{1'}H$, $-CH_2NH_2$, $-CH_2CH_2NH_2$ or $-NH-C(=NH)-NH_2$;

$R^1$ and $R^{1'}$ which may be identical or different denote hydrogen, OH, $-COR^2$ or $-COOR^2$;

$R^2$ denotes hydrogen, $C_1$-$C_{10}$-alkyl or aryl-$C_1$-$C_4$-alkyl;

$X^2$ and $X^3$ which may be identical or different denote a bridge selected from among $-(CH_2)_n-$, $-(CH_2)_nO-$,

$-(CH_2)_n-S-$, $-(CH_2)_nNR^3-$ and $-(CH_2)_nN^+R^3R^4-$ where n=1 or 2;

A denotes $C_2$-$C_{12}$-alkylene wherein optionally one or more hydrogen atoms may be replaced by one or more groups selected from among F, $OR^3$ and $COOR^3$, or

A denotes $-(CH_2)_l-D-(CH_2)_m-$, whilst in the alkylene groups $-(CH_2)_l-$ and $-(CH_2)_m-$ one or two hydrogen atoms may optionally be replaced by a $C_1$-$C_4$ group and wherein

D denotes a group selected from among phenyl, cyclopentyl and cyclohexyl wherein one or more hydrogen atoms may optionally be replaced by one or more groups selected from among F, $R^3$ and $OR^3$
and l and m, which may be identical or different, denote 0, 1, 2, 3 or 4,
or

D denotes -O- or $-NR^3-$
and l and m, which may be identical or different, denote 2, 3 or 4;

or

A denotes $-G^1-(CH_2)_r-G^2-$,
if $X^2$ or $X^3$ is $-(CH_2)_n-$, A also denotes
$-E^1-(CH_2)_r-G^1-$ or $-E^1-(CH_2)_r-E^2-$,
wherein
r denotes the number 0, 1, 2, 3 or 4,

$G^1$ and $G^2$ which may be identical or different denote a single bond, cyclopentyl, cyclohexyl or cycloheptyl, but if r=0 or r=1 $G^1$ and $G^2$ cannot simultaneously represent a single bond;
$E^1$ and $E^2$ which may be identical or different denote a group selected from among pyrrolidine, imidazolidine, piperidine and piperazine, wherein at least one N-atom is bound to $X^2$ or $X^3$ = $(CH_2)_n$,

optionally in the form of their racemates, enantiomers, diastereomers, tautomers and mixtures thereof, and optionally the pharmacologically acceptable acid addition salts thereof.

**4.** Compounds of general formula (IA) according to one of claims 1, 2 or 3, wherein

$B^1$ and $B^2$ which may be identical or different denote $-C(=NR^1)-NR^{1'}H$, $-CH_2NH_2$, $-CH_2CH_2NH_2$ or $-NH-C(=NH)-NH_2$;

$R^1$ and $R^{1'}$ which may be identical or different denote hydrogen, OH, $-COR^2$ or $-COOR^2$,

$R^2$ denotes hydrogen, $C_1$-$C_6$-alkyl or benzyl;

$X^2$ and $X^3$ which may be identical or different denote a bridge selected from among $-(CH_2)_n-$, $-(CH_2)_nO-$, $-(CH_2)_n-S-$, $-(CH_2)_nNR^3-$ and $-(CH_2)_nN^+R^3R^4-$ where n=1 or 2;

A denotes $C_2$-$C_{12}$-alkylene wherein one or more hydrogen atoms may optionally be replaced by one or more groups selected from among F, $OR^3$ and $COOR^3$, or

A denotes $-(CH_2)_l-D-(CH_2)_m-$, whilst in the alkylene groups $-(CH_2)_l-$ and $-(CH_2)_m-$ one or two hydrogen atoms may optionally be replaced by a methyl group and wherein

D denotes a group selected from among phenyl and cyclohexyl wherein optionally one or more hydrogen atoms may be replaced by one or more groups selected from among F, $R^3$ and $OR^3$
and l and m, which may be identical or different, denote 0, 1, 2 or 3,
or
D denotes -O- and l and m, which may be identical or different, denote 2 or 3;

or

A denotes $-G^1-(CH_2)_r-G^2-$,
if $X^2$ or $X^3$ represents $-(CH_2)_n-$ A also denotes
$-E^1-(CH_2)_r-G^1-$ or $-E^1-(CH_2)_r-E^2-$,
wherein
r denotes the number 0, 1, 2 or 3,

$G^1$ and $G^2$ which may be identical or different denote a single bond, cyclopentyl or cyclohexyl, but if r=0 or r=1 $G^1$ and $G^2$ cannot simultaneously represent a single bond;
$E^1$ and $E^2$ which may be identical or different denote piperidine or piperazine, wherein at least one N-atotn is bound to $X^2$ or $X^3$ = $(CH_2)_n$,

optionally in the form of their racemates, enantiomers, diastereomers, tautomers and mixtures thereof, and optionally the pharmacologically acceptable acid addition salts thereof.

**5.** Compounds of general formula (IA) according to one of claims 1 to 4, wherein

$B^1$ and $B^2$ which may be identical or different denote $-C(=NR^1)-NH_2$, $-CH_2NH_2$ or $-CH_2CH_2NH_2$;

$R^1$ denotes hydrogen, OH, $-COR^2$ or $-COOR^2$,

$R^2$ denotes hydrogen, methyl, ethyl, propyl, butyl, pentyl, hexyl or benzyl;

$X^2$ and $X^3$ $X^4$ which may be identical or different denote a bridge selected from among $-(CH_2)_n-$, $-(CH_2)_nO-$, $-(CH_2)_nNH-$, $-(CH_2)_nNMe-$, $-(CH_2)_nNEt-$, $-(CH_2)_nNprop-$, $-(CH_2)_nNcyclopropy-$, $-(CH_2)_nNBu-$ and $-(CH_2)_nN^+(Me)_2-$ where n=1;

A denotes $C_2$-$C_{12}$-alkylene which may optionally be substituted by a group selected from among OH, COOH and COOMe, or
$-(CH_2)_l-D-(CH_2)_m-$, whilst in the alkylene groups $-(CH_2)_l-$ and $-(CH_2)_m-$ one or two hydrogen atoms may optionally be replaced by methyl and wherein

D denotes phenyl or cyclohexyl which may optionally be substituted by methyl and l and m, which may be identical or different, denote 0, 1, 2 or 3,
or
D denotes -O- and l and m, which may be identical or different, represent 2 or 3;

or

A denotes $-G^1-(CH_2)_r-G^2-$,
if $X^2$ or $X^3$ denotes $-(CH_2)_n-$ A may also denote
$-E^1-(CH_2)_r-G^1-$ or $-E^1-(CH_2)_r-E^2-$,
wherein
r denotes the number 0, 1, 2 or 3,

$G^1$ and $G^2$ which may be identical or different denote a single bond or cyclohexyl, but if r=0 or r=1 $G^1$ and $G^2$ cannot simultaneously represent a single bond;
$E^1$ and $E^2$ which may be identical or different denote piperidine or piperazine, wherein at least one N-atom is bound to $X^2$ or $X^3$ = $(CH_2)_n$,

optionally in the form of their racemates, enantiomers, diastereomers, tautomers and mixtures thereof, and optionally the pharmacologically acceptable acid addition salts thereof.

**6.** Compounds of general formula (IA) according to one of claims 1 to 5, wherein the grouping

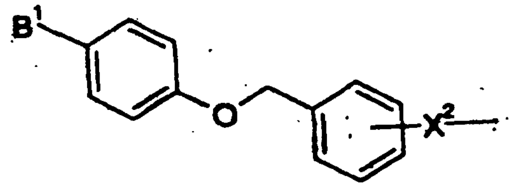

denotes a group selected from among

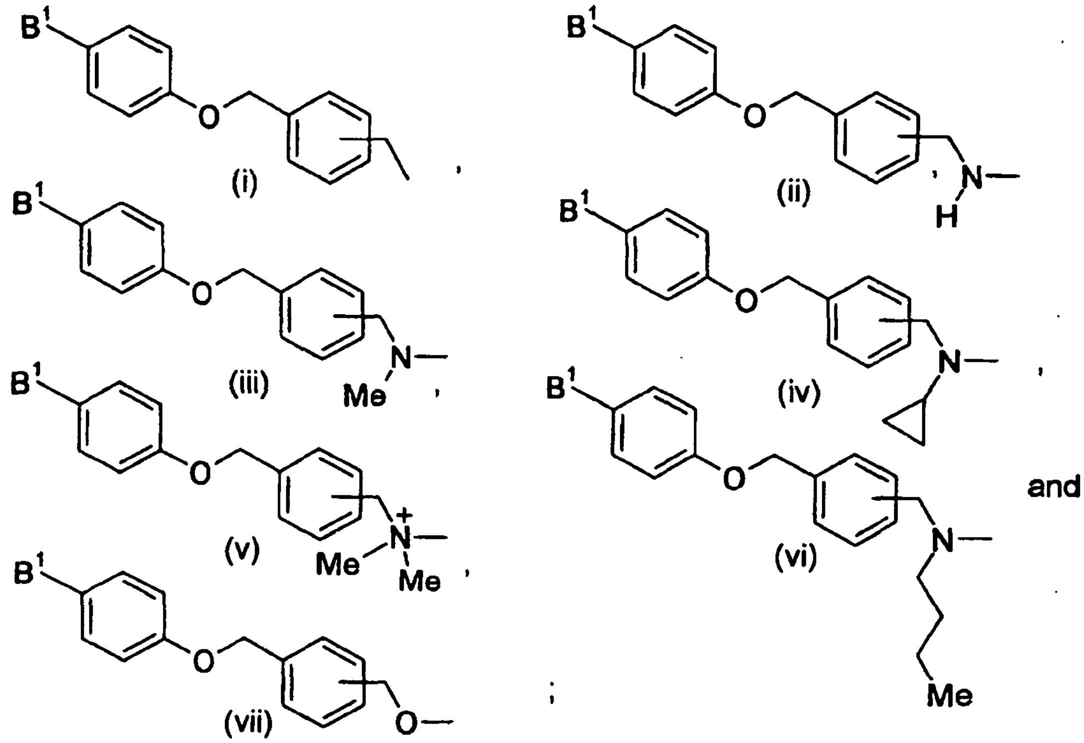

(i), (ii), (iii), (iv), (v), (vi) and (vii);

the grouping

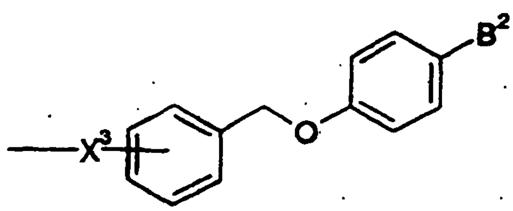

denotes a group selected from among

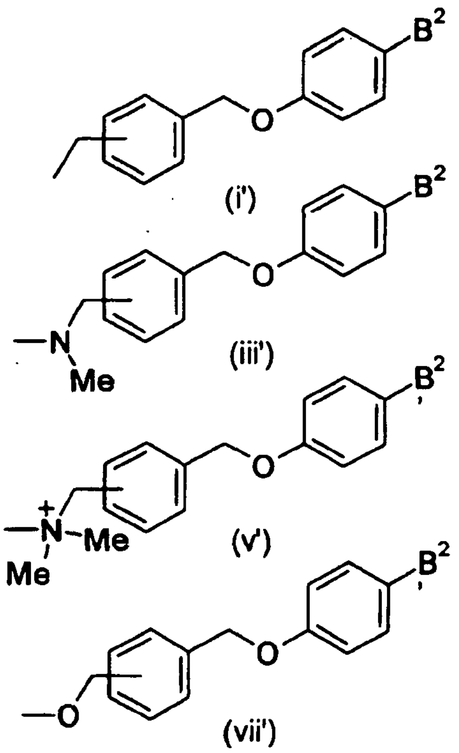

(i') (iii') (v') (vii') ;

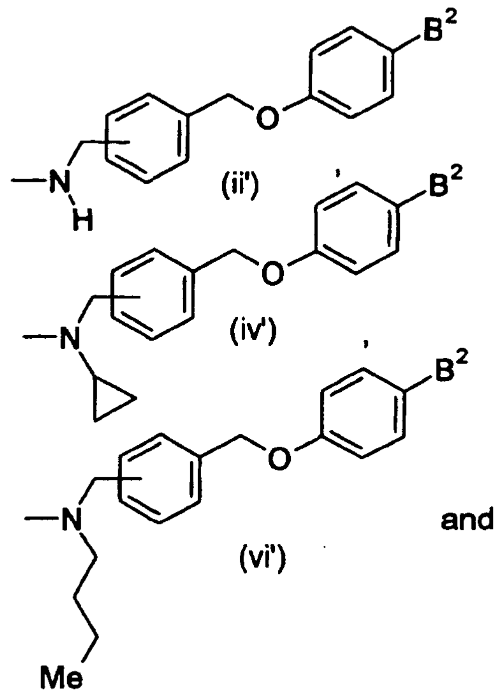

(ii'), (iv'), (vi') and

wherein

B[1] and B[2] which may be identical or different denote $-C(=NR^1)-NR^{1'}H$, $-CH_2NH_2$, $-CH_2CH_2NH_2$ or $-NH-C(=NH)-NH_2$;

$R^1$ and $R^{1'}$ which may be identical or different denote hydrogen or OH;

A denotes a bridging group which is selected from among
(viii) $C_4$-$C_{10}$-alkylene which may optionally be substituted by COOH,

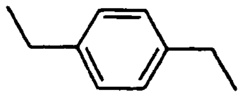,
(ix)

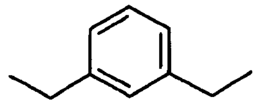,
(x)

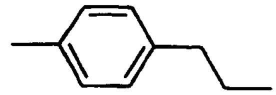,
(xi)

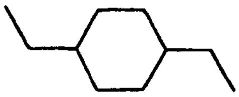,
(xii)

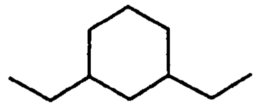,
(xiii)

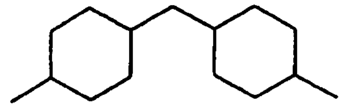,
(xiv)

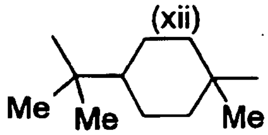,
(xv)

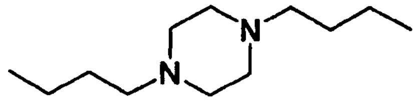
(xvi)

and

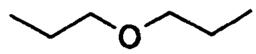;
(xvii)

or

A may also denote

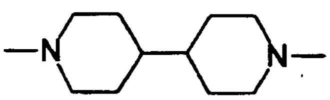
(xviii)

if the grouping

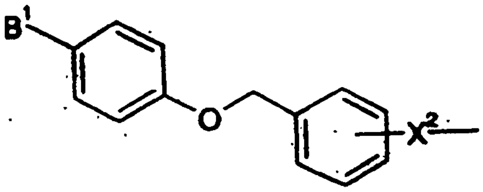

represents the group

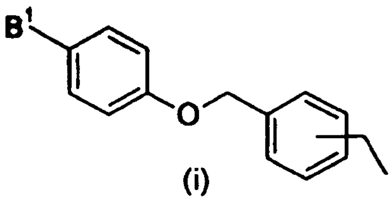
(i)

and the grouping

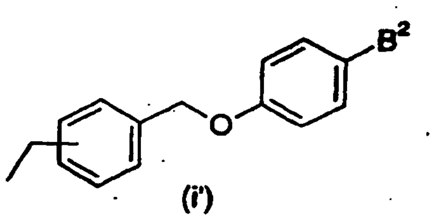

(i')

represents the group

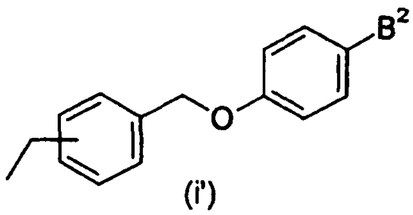

(i')

or

A may also denote

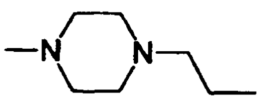

(xix)

if the grouping of the two groups

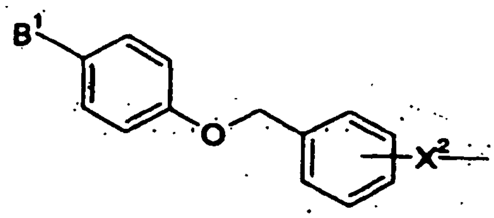

and

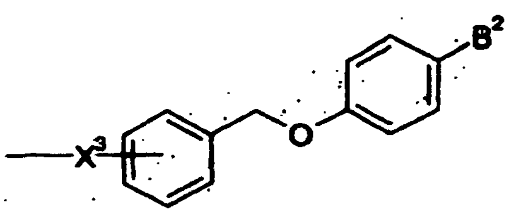

represents the group (i) or (i') which is bound directly to the piperazine-nitrogen of the group A,

optionally in the form of their racemates, enantiomers, diastereomers, tautomers and mixtures thereof, and optionally the pharmacologically acceptable acid addition salts thereof.

**7.** Compounds of general formula (IA) according to one of claims 1 to 6, wherein the grouping

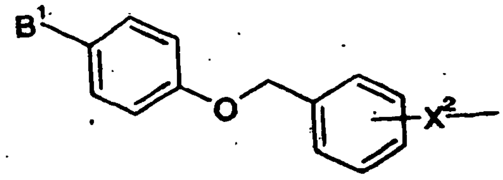

denotes a group selected from among

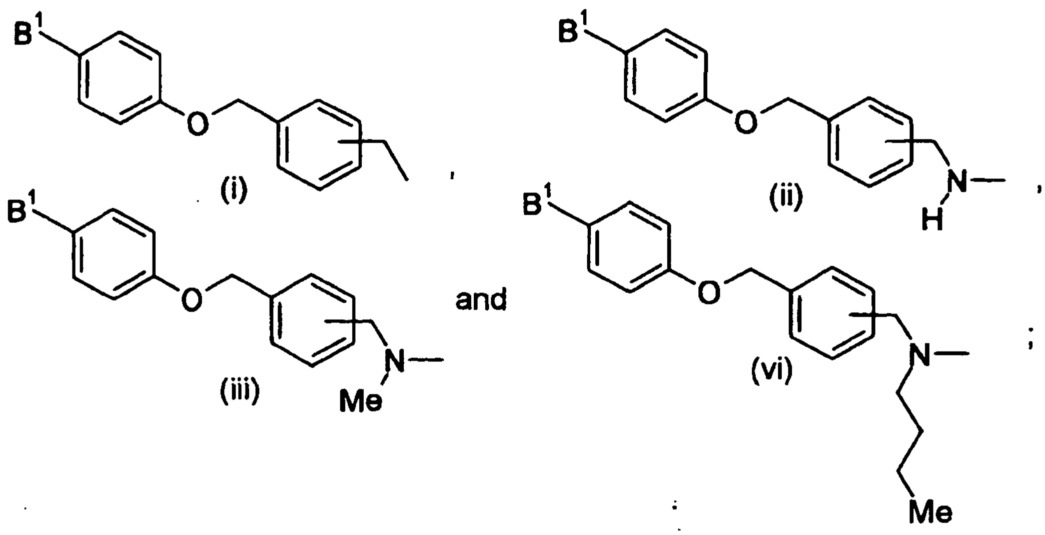

(i) , (ii) ,

(iii) and (vi) ;

the grouping

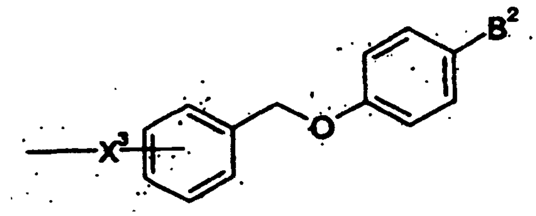

denotes a group selected from among

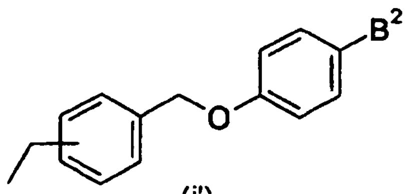

(i')

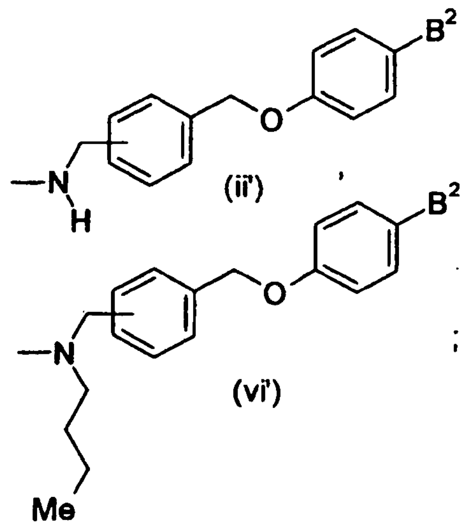

(ii') ,

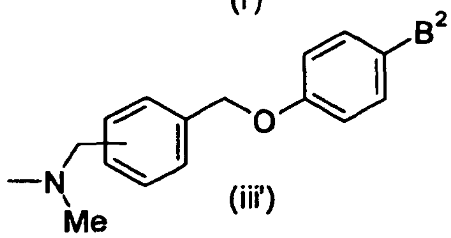

(iii')

and

(vi') ;

wherein

B[1] and B[2] which may be identical or different denote -C(=NR[1])-NR[1']H or $-CH_2NH_2$,

R[1] and R[1'] which may be identical or different denote hydrogen or OH;

A denotes a bridging group which is selected from among
(viii) $C_4$-$C_{10}$-alkylene,

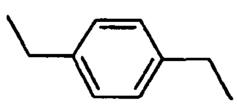

(ix) ,

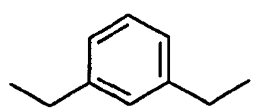

(x) ,

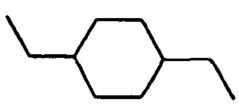

(xii) ,

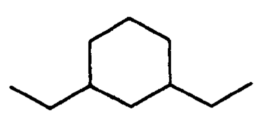

(xiii)

and

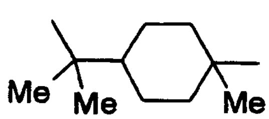

(xv) ;

or

A may also denote

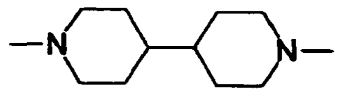

(xviii)

if the grouping

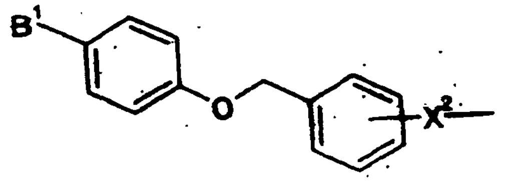

denotes the group (i)

and the grouping

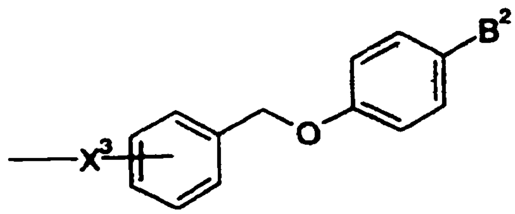

denotes the group (i'),

optionally in the form of their racemates, enantiomers, diastereomers, tautomers and mixtures thereof, and optionally the pharmacologically acceptable acid addition salts thereof.

**8.** Compounds of general formula (IA1)

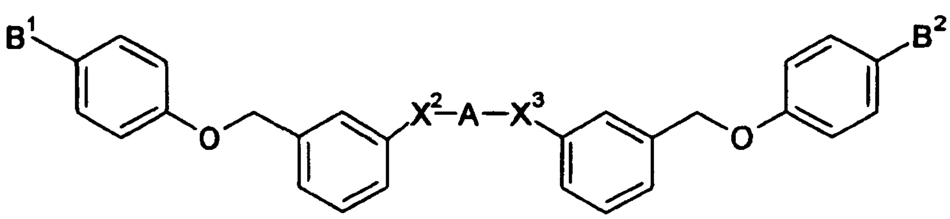

( IA1 )

wherein $B^1$, $B^2$, A, $X^2$ and $X^3$ have the meanings given in claims 1 to 7, optionally in the form of their racemates, enantiomers, diastereomers, tautomers and mixtures thereof, and optionally the pharmacologically acceptable acid addition salts thereof.

**9.** Compounds of general formula (IA) or (IA1) according to one of claims 1 to 8, wherein $-X^2$-A-$X^3$- denotes a group selected from among the formulae

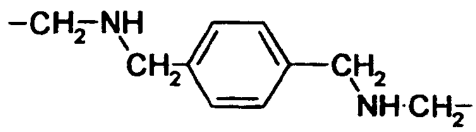

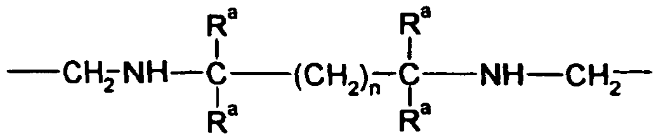

wherein Ra denotes hydrogen or methyl and n is 3, 4, 5 or 6,

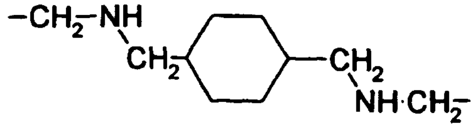

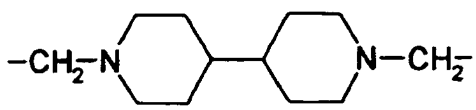

**10.** Compounds of general formula (IA) or (IA1) according to one of claims 1 to 9, selected from among:

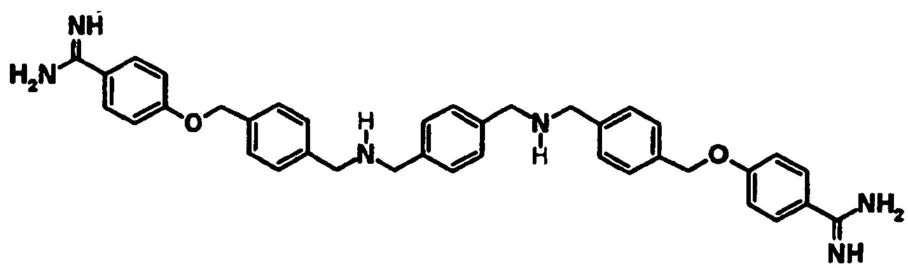

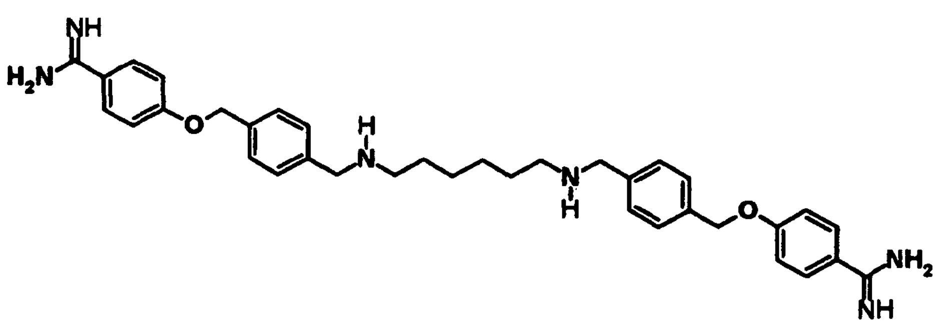

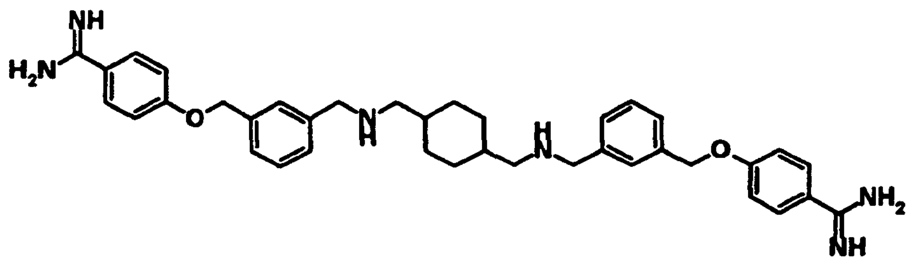

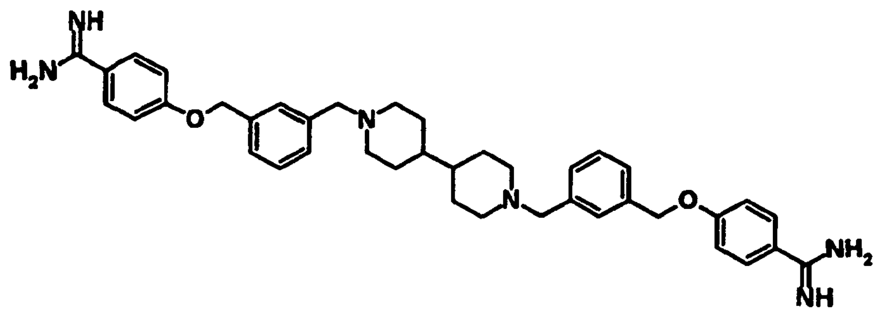

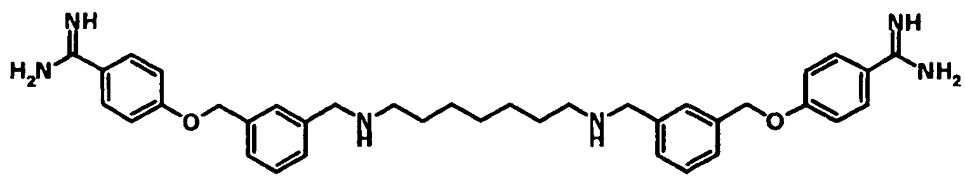

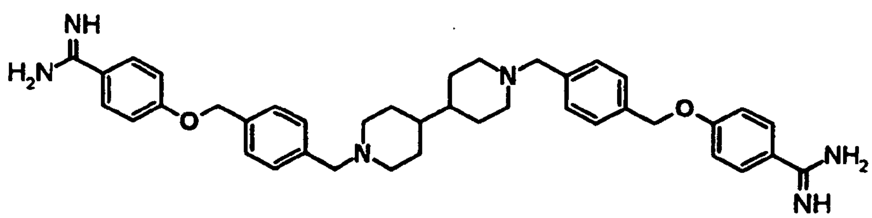

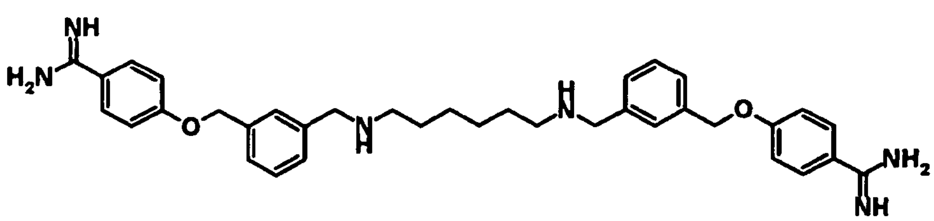

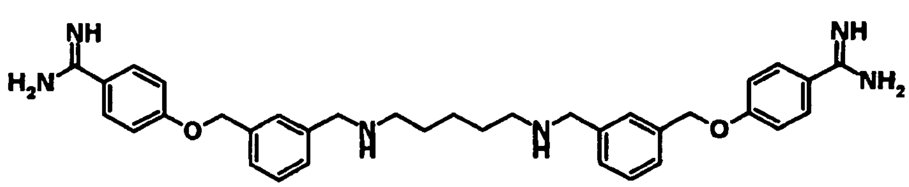

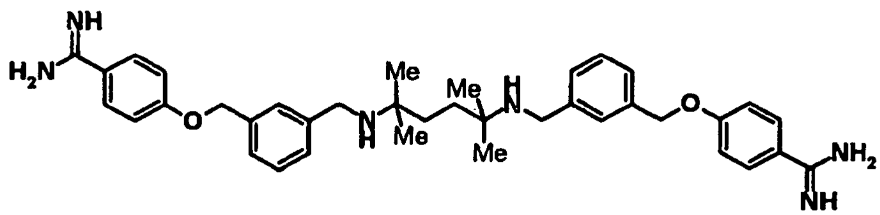

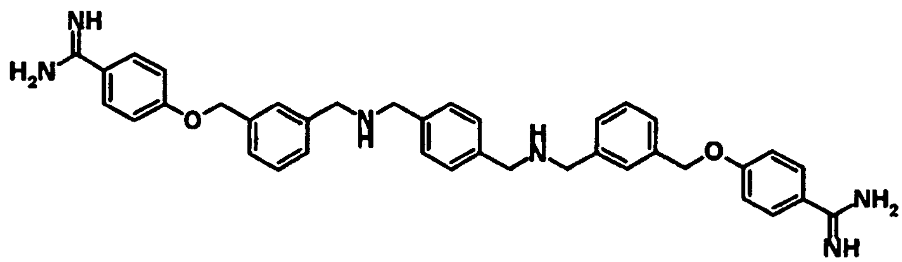

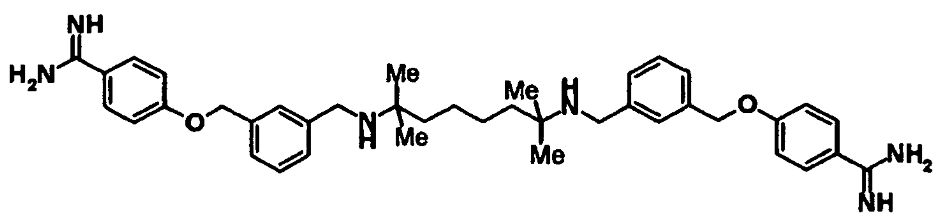

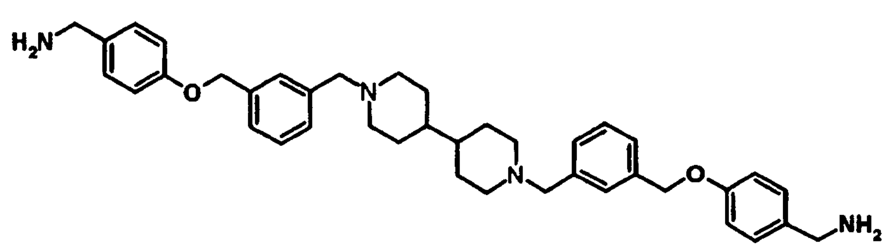

11. Use of compounds of general formula (IA) or (IA1) according to one of claims 1-10 for preparing a pharmaceutical composition for the prevention and/or treatment of diseases in which tryptase inhibitors may have a therapeutic benefit.

12. Pharmaceutical composition, **characterised in that** it contains one or more compounds according to one of claims 1-10.

13. Process for preparing the compounds of general formula (IA)

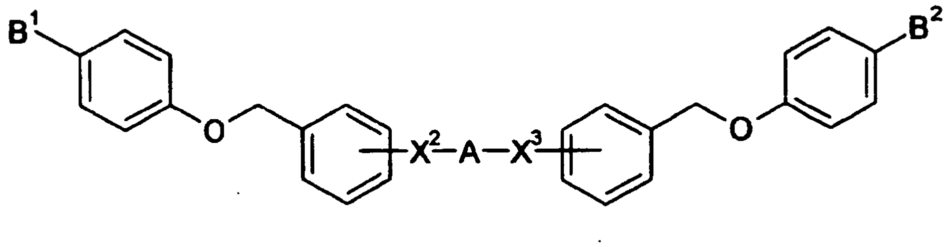

( IA )

wherein $B^1$ and $B^2$ denote -C(=NH)-$NH_2$ and the groups $X^2$, A, $X^3$ may have the meanings given in claims 1 to 10, **characterised in that**

a) imidoesters of general formula (IIA)

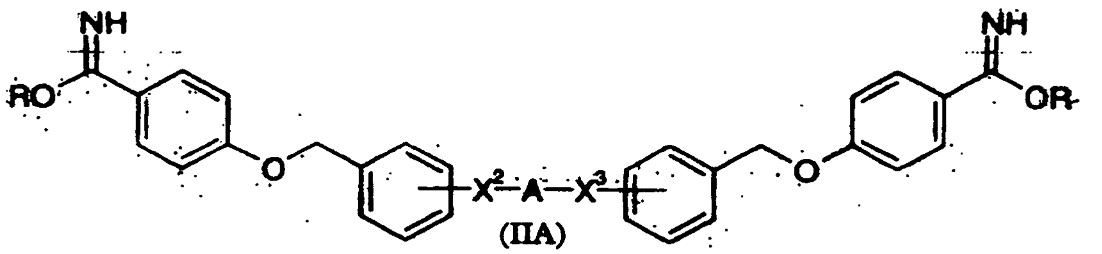

(IIA)

wherein R denotes $C_1$-$C_6$-alkyl and the groups $X^2$, A, $X^3$ may have the meanings given in claims 1 to 10, are reacted with ammonia; or

b) compounds of general formula (IA), wherein $B^1$ and $B^2$ denote -C(=NOH)-$NH_2$ and the groups $X^2$, A, $X^3$ may have the meanings given in claims 1-10, are reduced.

**14.** Process for preparing compounds of general formula (IA)

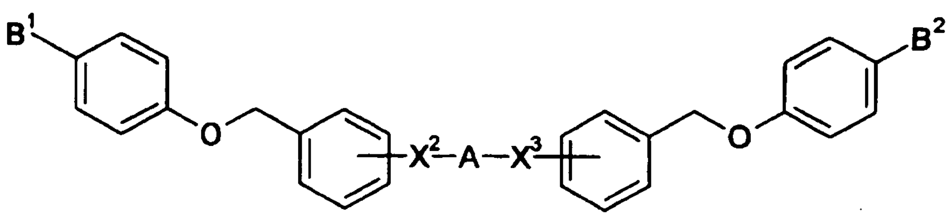

( IA )

wherein $B^1$ and $B^2$ denote -C(=NOH)-$NH_2$ and the groups $X^2$, A, $X^3$ may have the meanings given in claims 1 to 10, **characterised in that** compounds of general formula (IIIA)

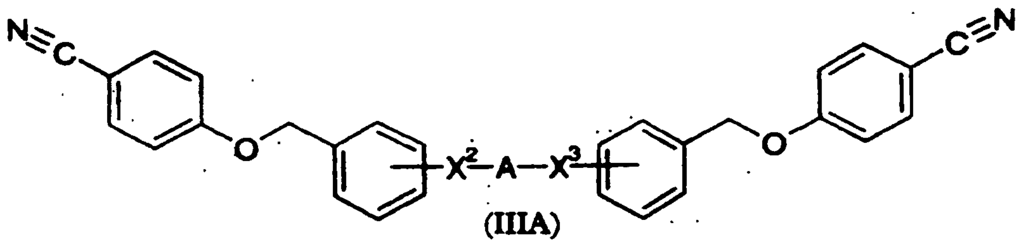

(IIIA)

wherein the groups $X^2$, A, $X^3$ may have the meanings given in claims 1 to 10, are treated with hydroxylamine.

**15.** Process for preparing compounds of general formula (IA)

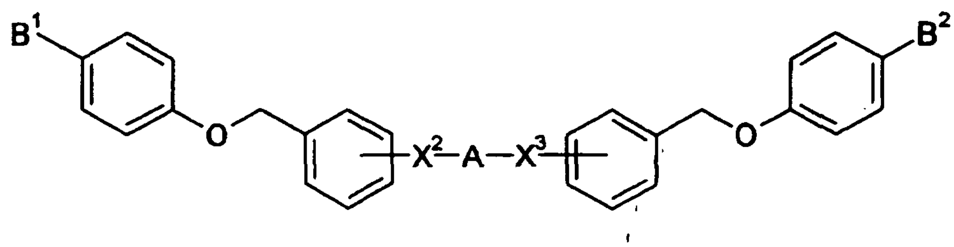

( IA )

wherein $B^1$ and $B^2$ and the groups $X^2$, A, $X^3$ may have the meanings given in claims 1 to 10, **characterised in that**

a) compounds of general formula (IVA) and (VA)

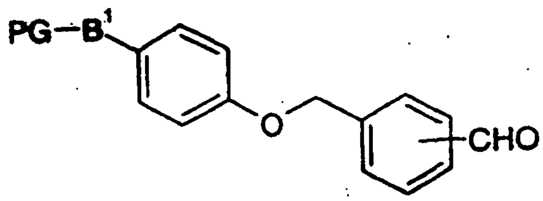

(IVA)

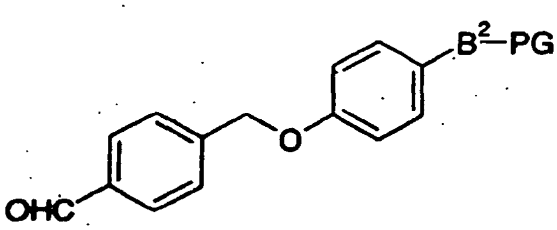

(VA)

wherein PG may denote a protecting group suitable for protecting amines, are reacted with a diamine with subsequent reduction of the C=N- double bonds thus formed and finally the protecting groups PG are cleaved in the usual way; or

b) compounds of general formulae (VIA) and (VIIA)

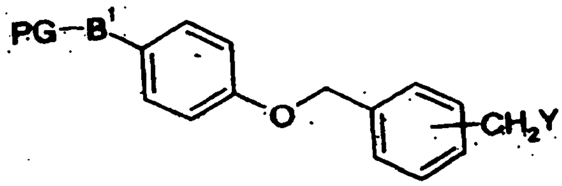

(VIA)

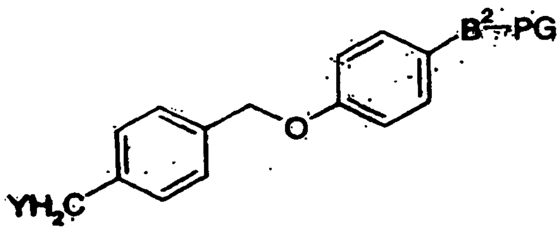

(VIIA)

wherein the groups PG, independently of one another, may in each case denote a protecting group suitable for protecting amines,
and the groups Y, independently of one another, in each case denote a group selected from among fluorine, chlorine, bromine, iodine, $C_1$-$C_4$-alkyl and arylsulphonate,
are reacted with a diamine or a dialcohol and finally the protecting groups PG are cleaved in the usual way.

**16.** Process for preparing the compounds of general formula (IA)

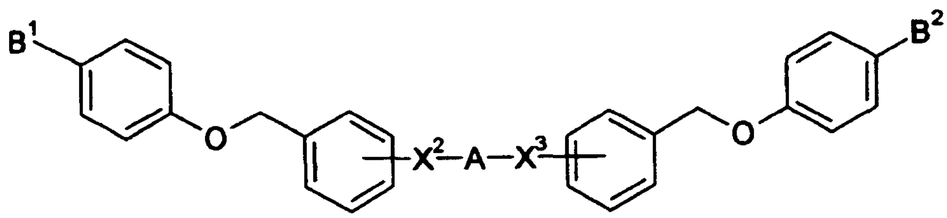

( IA )

wherein $B^1$ and $B^2$ denote -C(=NR$^1$)-NH$_2$ with $R^1 \neq H$ and the groups $X^2$, A, $X^3$ may have the meanings given in claims 1-10, **characterised in that** compounds of general formula (IA) wherein $B^1$ and $B^2$ denote -C(=NH)-NH$_2$ are reacted with chloroformates or acyl halides or corresponding anhydrides.

**17.** Process for preparing the compounds of general formula (IA)

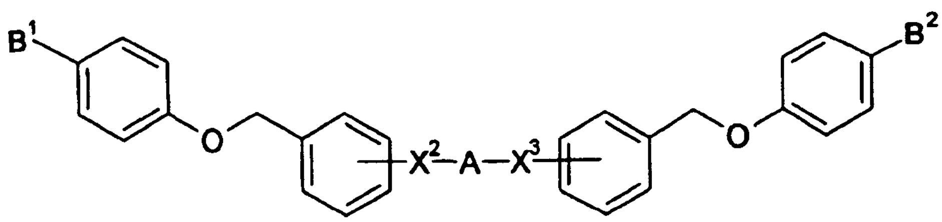

( IA )

wherein $B^1$ and $B^2$ denote $-CH_2-NH_2$ and the groups $X^2$, A, $X^3$ may have the meanings given in claims 1-10, **characterised in that** the corresponding nitrile compounds of general formula (III)

$$NC-Ar^1-X^1-Ar^2-X^2-A-X^3-Ar^3-X^4-Ar^4-CN \quad (III)$$

wherein

| | |
|---|---|
| $Ar^1$, $Ar^2$, $Ar^3$ and $Ar^4$ | denote phenyl; |
| $-X^1-$ | denotes $-O-CH_2-$; |
| $-X^4-$ | denotes $-CH_2-O-$, |

are reduced.

## Revendications

**1.** Composés de formule générale (IA)

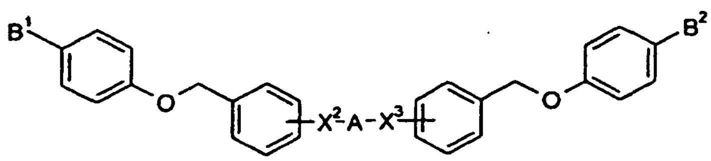

(IA)

où
$B^1$ et $B^2$, identiques ou différents, représentent $-C(=NR^1)-NR^{1'}H$, $-CH_2NH_2$, $-CH_2CH_2NH_2$ ou $-NH-C(=NH)-NH_2$;
$R^1$ et $R^{1'}$, identiques ou différents, représentent l'hydrogène, OH, $-COR^2$ ou $-COOR^2$ ;
$R^2$ représente l'hydrogène, $C_1$-$C_{18}$-alkyle, aryle ou aryl-$C_1$-$C_6$-alkyle ;
$X^2$ et $X^3$
identiques ou différents, représentent un pont choisi dans le groupe $-(CH_2)_n-$, $-(CH_2)_nO-$, $-(CH_2)_n-S-$, $-(CH_2)_nNR^3-$ et $-(CH_2)_nN^+R^3R^4-$ avec n = 1 ou 2 ;
A représente un groupement choisi dans le groupe $C_2$-$C_{16}$-alkylène et $C_2$-$C_{16}$-alcénylène, dans lequel éventuellement un ou plusieurs atomes d'hydrogène peuvent être remplacés par un ou plusieurs groupements choisis dans le groupe F, $R^3$, $OR^3$ et $COOR^3$,
$C_2$-$C_{16}$-alcynylène, ou
A représente $-(CH_2)_l-D-(CH_2)_m-$, où, dans les groupes alkylène $-(CH_2)_l-$ et $-(CH_2)_m-$, un ou deux atomes d'hydrogène peuvent éventuellement être remplacés par $C_1$-$C_6$-alkyle et où
D représente aryle ou $C_3$-$C_{10}$-cycloalkyle, dans lequel éventuellement un ou plusieurs atomes d'hydrogène peuvent être remplacés par un ou plusieurs groupements choisis dans le groupe F, $R^3$ et $OR^3$ et l et m, identiques ou différents, représentent 0, 1, 2, 3 ou 4, ou
D représente -O-, -S- ou $-NR^3-$
et l et m, identiques ou différents, représentent 2, 3 ou 4 ;
ou
A représente $-G^1-(CH_2)_r-G^2-$, si $X^2$ ou $X^3$ représente $-(CH_2)_n-$, également $-E^1-(CH_2)_r-G^1-$ ou $-E^1-(CH_2)_r-E^2-$,
où
r représente un nombre 0, 1, 2, 3, 4, 5 ou 6,
$G^1$ et $G^2$, identiques ou différents, représentent une simple liaison ou $C_3$-$C_{10}$-cycloalkyle, mais, dans le cas de r = 0 ou r = 1, $G^1$ et $G^2$ ne peuvent pas représenter en même temps une simple liaison ;
$E^1$ et $E^2$, identiques ou différents, représentent $C_3$-$C_{10}$-aza-cycloalkyle, qui contient un ou deux atomes d'azote, où au moins un atome N est lié à $X^2$ ou $X^3 = (CH_2)_n$,
éventuellement sous forme de leurs racémates, de leurs énantiomères, de leurs diastéréoisomères, de leurs tautomères et de leurs mélanges, et éventuellement de leurs sels d'addition d'acide pharmacologiquement acceptables.

**2.** Composés de formule générale (IA) selon la revendication 1 où $B^1$ et $B^2$, identiques ou différents, représentent $-C(=NR^1)-NR^{1'}H$, $-CH_2NH_2$, $-CH_2CH_2NH_2$ ou $-NH-C(=NH)-NH_2$ ;
$R^1$ et $R^{1'}$, identiques ou différents, représentent l'hydrogène, OH, $-COR^2$ ou $-COOR^2$ ;
$R^2$ représente l'hydrogène, $C_1$-$C_{14}$-alkyle, aryle ou aryl-$C_1$-$C_6$-alkyle ;
$X^2$ et $X^3$
identiques ou différents, représentent un pont choisi dans le groupe consistant en $-(CH_2)_n-$, $-(CH_2)_nO-$, $-(CH_2)_n-S-$, $-(CH_2)_nNR^3-$ et $-(CH_2)_nN^+R^3R^4-$ avec n = 1 ou 2 ;
A représente un groupement choisi dans le groupe $C_2$-$C_{14}$-alkylène et $C_2$-$C_{10}$-alcénylène, dans lequel éventuellement un ou plusieurs atomes d'hydrogène peuvent être remplacés par un ou plusieurs groupements choisis dans le groupe F, $R^3$, $OR^3$ et $COOR^3$, $C_2$-$C_{10}$-alcynylène, ou
A représente $-(CH_2)_l-D-(CH_2)_m-$, où, dans les groupes alkylène $-(CH_2)_l-$ et $-(CH_2)_m-$, un ou deux atomes d'hydrogène peuvent éventuellement être remplacés par $C_1$-$C_6$-alkyle et où
D représente aryle ou $C_3$-$C_8$-cycloalkyle, dans lequel éventuellement un ou plusieurs atomes d'hydrogène peuvent être remplacés par un ou plusieurs groupements choisis dans le groupe F, $R^3$ et $OR^3$ et l et m, identiques ou différents, représentent 0, 1, 2, 3 ou 4, ou
D représente -O-, -S- ou $-NR^3-$
et l et m, identiques ou différents, représentent 2, 3 ou 4 ;
ou
A représente $-G^1-(CH_2)_r-G^2-$,
si $X^2$ ou $X^3$ représente $-(CH_2)_n-$ également $-E^1-(CH_2)_r-G^1-$ ou $-E^1-(CH_2)_r-E^2-$,
où
r représente un nombre 0, 1, 2, 3, 4, 5 ou 6,
$G^1$ et $G^2$, identiques ou différents, représentent une simple liaison ou $C_3$-$C_8$-cycloalkyle, mais, dans le cas de r = 0 ou r = 1, $G^1$ et $G^2$ ne peuvent pas représenter en même temps une simple liaison ;
$E^1$ et $E^2$, identiques ou différents, représentent $C_3$-$C_8$-aza-cycloalkyle, qui contient un ou deux atomes d'azote, où au moins un atome N est lié à $X^2$ ou $X^3 = (CH_2)_n$,
éventuellement sous forme de leurs racémates, de leurs énantiomères, de leurs diastéréoisomères, de leurs tautomères et de leurs mélanges, et éventuellement de leurs sels d'addition d'acide pharmacologiquement acceptables.

**3.** Composés de formule générale (IA) selon l'une des revendications 1 ou 2 où
$B^1$ et $B^2$, identiques ou différents, représentent $-C(=NR^1)-NR^{1'}H$, $-CH_2NH_2$, $-CH_2CH_2NH_2$ ou $-NH-C(=NH)-NH_2$ ;
$R^1$ et $R^{1'}$, identiques ou différents, représentent l'hydrogène, OH, $-COR^2$ ou $-COOR^2$;
$R^2$ représente l'hydrogène, $C_1$-$C_{10}$-alkyle ou aryl-$C_1$-$C_4$-alkyle ;
$X^2$ et $X^3$
identiques ou différents, représentent un pont choisi dans le groupe consistant en $-(CH_2)_n-$, $-(CH_2)_nO-$, $-(CH_2)_n-S-$, $-(CH_2)_nNR^3-$ et $-(CH_2)_nN^+R^3R^4-$ avec n = 1 ou 2 ;
A représente $C_2$-$C_{12}$-alkylène dans lequel éventuellement un ou plusieurs atomes d'hydrogène peuvent être remplacés par un ou plusieurs groupements choisis dans le groupe F, $OR^3$ et $COOR^3$, ou
A représente $-(CH_2)_l-D-(CH_2)_m-$, où, dans les groupes alkylène $-(CH_2)_l-$ et - $(CH_2)_m-$, un ou deux atomes d'hydrogène peuvent éventuellement être remplacés par un groupe $C_1$-$C_4$-alkyle et où
D représente un groupement choisi dans le groupe consistant en phényle, cyclopentyle et cyclohexyle dans lequel éventuellement un ou plusieurs atomes d'hydrogène peuvent être remplacés par un ou plusieurs groupements choisis dans le groupe F, $R^3$ et $OR^3$ et l et m, identiques ou différents, représentent 0, 1, 2, 3 ou 4, ou
D représente -O- ou $-NR^3-$
et l et m, identiques ou différents, représentent 2, 3 ou 4 ;
ou
A représente $-G^1-(CH_2)_r-G^2-$,
si $X^2$ ou $X^3$ représente $-(CH_2)_n-$ également
$-E^1-(CH_2)_r-G^1-$ ou $-E^1-(CH_2)_r-E^2-$,
où
r représente un nombre 0, 1, 2, 3 ou 4,
$G^1$ et $G^2$, identiques ou différents, représentent une simple liaison, cyclopentyle, cyclohexyle ou cycloheptyle, mais, dans le cas de r = 0 ou r = 1, $G^1$ et $G^2$ ne peuvent pas représenter en même temps une simple liaison ;
$E^1$ et $E^2$, identiques ou différents, représentent un groupement choisi dans le groupe consistant en pyrrolidine, imidazolidine, pipéridine et pipérazine, où au moins un atome N est lié à $X^2$ ou $X^3 = (CH_2)_n$,
éventuellement sous forme de leurs racémates, de leurs énantiomères, de leurs diastéréoisomères, de leurs tautomères et de leurs mélanges, et éventuellement de leurs sels d'addition d'acide pharmacologiquement acceptables.

**4.** Composés de formule générale (IA) selon l'une des revendications 1, 2 ou 3 où
$B^1$ et $B^2$, identiques ou différents, représentent $-C(=NR^1)-NR^{1'}H$, $-CH_2NH_2$, $-CH_2CH_2NH_2$ ou $-NH-C(=NH)-NH_2$ ;
$R^1$ et $R^{1'}$, identiques ou différents, représentent l'hydrogène, OH, $-COR^2$ ou $-COOR^2$ ;
$R^2$ représente l'hydrogène, $C_1$-$C_6$-alkyle ou benzyle ;
$X^2$ et $X^3$
identiques ou différents, représentent un pont choisi dans le groupe consistant en $-(CH_2)_n-$, $-(CH_2)_nO-$, $-(CH_2)_n-S-$, $-(CH_2)_nNR^3-$ et $-(CH_2)_nN^+R^3R^4-$ avec n = 1 ou 2 ;
A représente $C_2$-$C_{12}$-alkylène, dans lequel éventuellement un ou plusieurs atomes d'hydrogène peuvent être remplacés par un ou plusieurs groupements choisis dans le groupe F, $OR^3$ et $COOR^3$, ou
A représente $-(CH_2)_l-D-(CH_2)_m-$, où, dans les groupes alkylène $-(CH_2)_l-$ et
$-(CH_2)_m-$, un ou deux atomes d'hydrogène peuvent éventuellement être remplacés par un groupe méthyle et où
D représente un groupement choisi dans le groupe consistant en phényle et cyclohexyle, dans lequel éventuellement un ou plusieurs atomes d'hydrogène peuvent être remplacés par un ou plusieurs groupements choisis dans le groupe F, $R^3$ et $OR^3$
et l et m, identiques ou différents, représentent 0, 1, 2 ou 3, ou
D représente -O- et l et m, identiques ou différents, représentent 2 ou 3 ;
ou
A représente $-G^1-(CH_2)_r-G^2-$,
si $X^2$ ou $X^3$ représente $-(CH_2)_n-$ également
$-E^1-(CH_2)_r-G^1-$ ou $-E^1-(CH_2)_r-E^2-$,
où
r représente un nombre 0, 1, 2 ou 3,
$G^1$ et $G^2$, identiques ou différents, représentent une simple liaison, cyclopentyle ou cyclohexyle, mais, dans le cas de r = 0 ou r = 1, $G^1$ et $G^2$ ne peuvent pas représenter en même temps une simple liaison ;
$E^1$ et $E^2$, identiques ou différents, représentent pipéridine ou pipérazine, où au moins un atome N est lié à $X^2$ ou $X^3 = (CH_2)_n$,
éventuellement sous forme de leurs racémates, de leurs énantiomères, de leurs diastéréoisomères, de leurs tautomères et de leurs mélanges, et éventuellement de leurs sels d'addition d'acide pharmacologiquement acceptables.

**5.** Composés de formule générale (IA) selon l'une des revendications 1 à 4 où
$B^1$ et $B^2$, identiques ou différents, représentent $-C(=NR^1)-NH_2$, $-CH_2NH_2$ ou $-CH_2CH_2NH_2$ ;
$R^1$ représente l'hydrogène, OH, $-COR^2$ ou $-COOR^2$;
$R^2$ représente l'hydrogène, méthyle, éthyle, propyle, butyle, pentyle, hexyle ou benzyle ;
$X^2$ et $X^3$ $X^4$ identiques ou différents, représentent un pont choisi dans le groupe consistant en $-(CH_2)_n-$, $-(CH_2)_nO-$, $-(CH_2)_nNH-$, $-(CH_2)_nNMe-$, $-(CH_2)_nNEt-$, $-(CH_2)_nNProp-$, $-(CH_2)_nNCyclopropy-$, $-(CH_2)_nNBu-$ et $-(CH_2)_nN^+(Me)_2-$, avec n = 1 ;
A représente $C_2$-$C_{12}$-alkylène qui peut éventuellement être substitué par un groupement choisi dans le groupe OH, COOH et COOMe, ou
$-(CH_2)_l-D-(CH_2)_m-$, où, dans les groupes alkylène $-(CH_2)_l-$ et $-(CH_2)_m-$, un ou deux atomes d'hydrogène peuvent éventuellement être remplacés par méthyle et où
D représente phényle ou cyclohexyle, qui peut éventuellement être substitué par méthyle et l et m, identiques ou différents, représentent 0, 1, 2 ou 3,
ou
D représente -O- et l et m, identiques ou différents, représentent 2 ou 3 ;
ou
A représente $-G^1-(CH_2)_r-G^2-$,
si $X^2$ ou $X^3$ représente $-(CH_2)_n-$ également
$-E^1-(CH_2)_r-G^1-$ ou $-E^1-(CH_2)_r-E^2-$,
où
r représente un nombre 0, 1, 2 ou 3,
$G^1$ et $G^2$ identiques ou différents, représentent une simple liaison ou cyclohexyle, mais, dans le cas de r = 0 ou r = 1, $G^1$ et $G^2$ ne peuvent pas représenter en même temps une simple liaison ;
$E^1$ et $E^2$, identiques ou différents, représentent pipéridine ou pipérazine, où au moins un atome N est lié à $X^2$ ou $X^3 = (CH_2)_n$,
éventuellement sous forme de leurs racémates, de leurs énantiomères, de leurs diastéréoisomères, de leurs tautomères et de leurs mélanges, et éventuellement de leurs sels d'addition d'acide pharmacologiquement acceptables.

**6.** Composés de formule générale (IA) selon l'une des revendications 1 à 5, où

le groupement

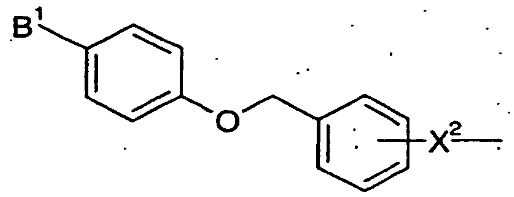

représente un groupement choisi dans le groupe consistant en

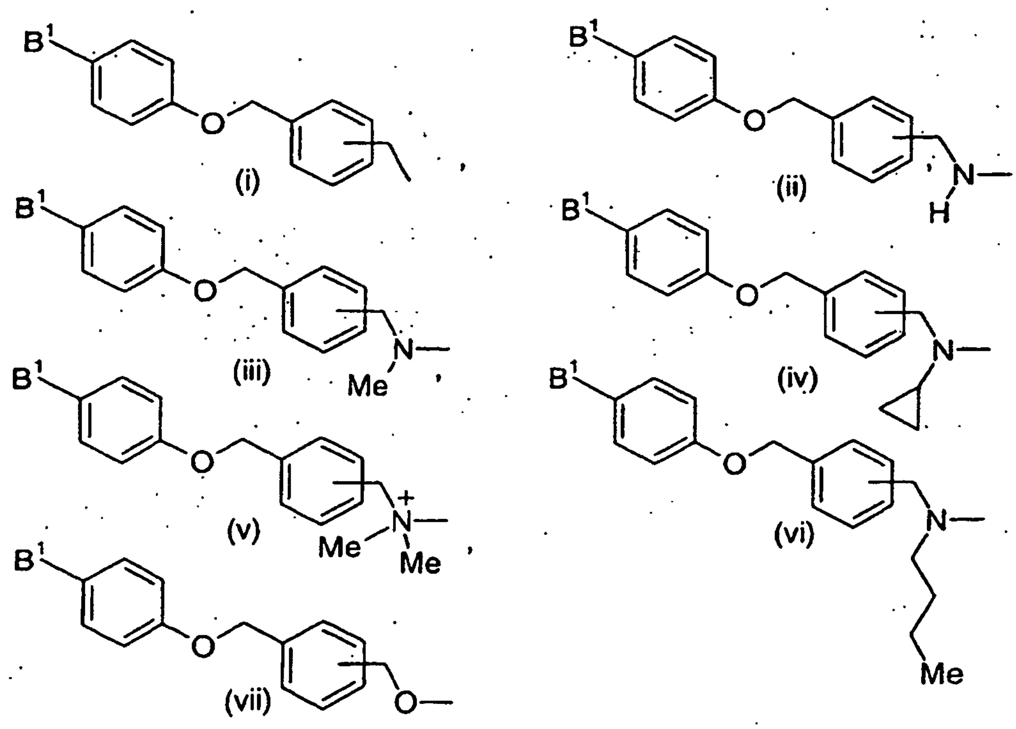

(i), (ii), (iii), (iv), (v), (vi) et (vii)

le groupement

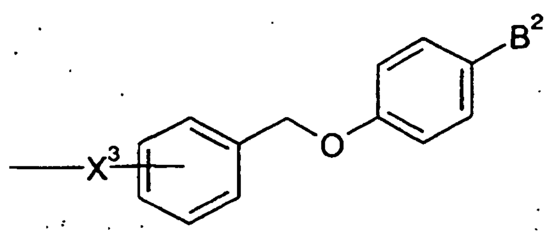

représente un groupement choisi dans le groupe consistant en

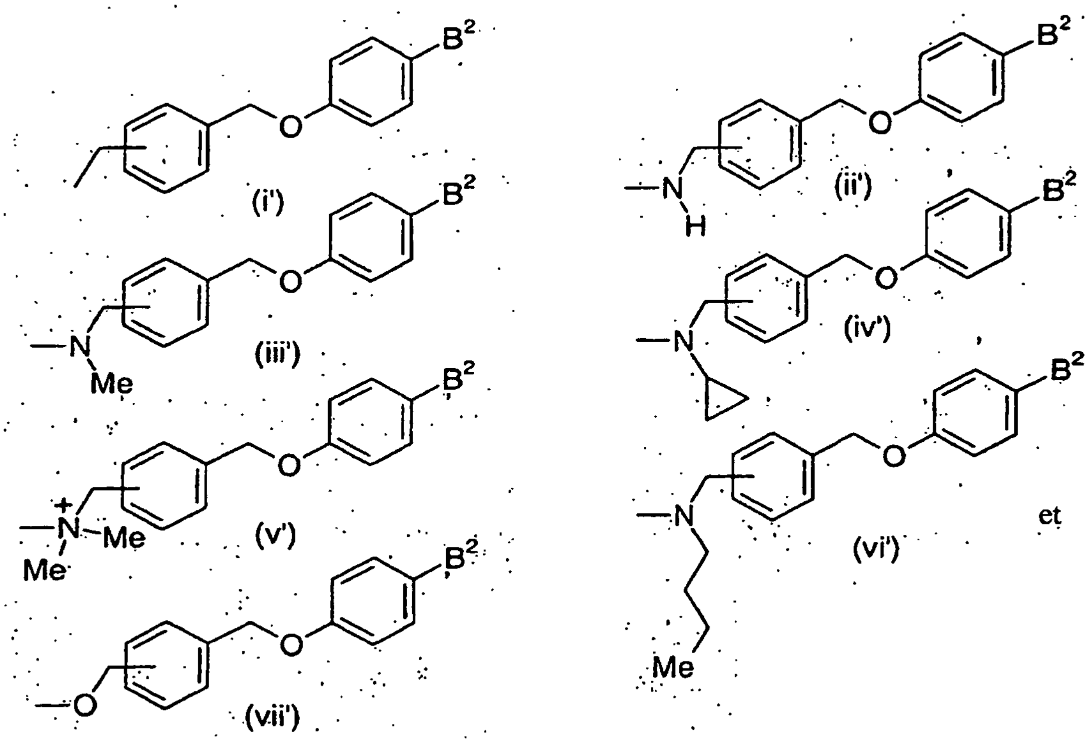

(i') (ii') , (iii') (iv') , (v') (vi') et (vii')

où

$B^1$ et $B^2$, identiques ou différents, représentent $-C(=NR^1)-NR^{1'}H$, $-CH_2NH_2$, $-CH_2CH_2NH_2$ ou $-NH-C(=NH)-NH_2$;
$R^1$ et $R^{1'}$, identiques ou différents, représentent l'hydrogène ou OH ;
A représente un groupement de pontage qui est choisi dans le groupe consistant en (viii) $C_4$-$C_{10}$-alkylène, qui peut éventuellement être substitué par COOH,

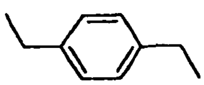
(ix),

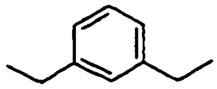
(x),

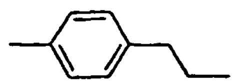
(xi),

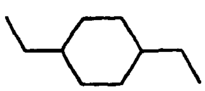
(xii),

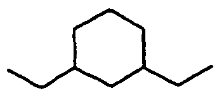
(xiii),

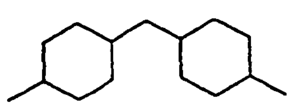
(xiv),

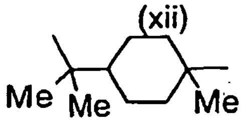
(xv),

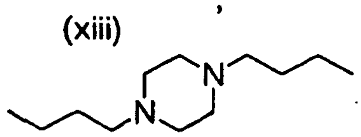
(xvi) et

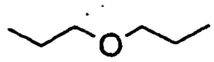
(xvii) ;

ou
A représente en outre

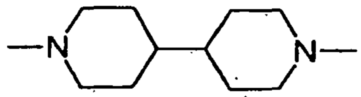

(xviii)

quand le groupement

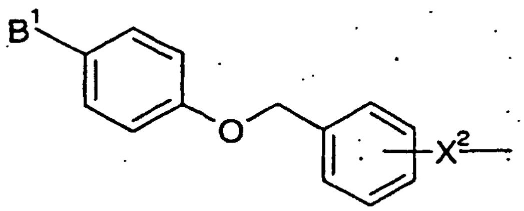

représente le groupe

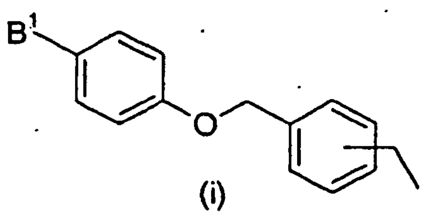

(i)

et le groupement

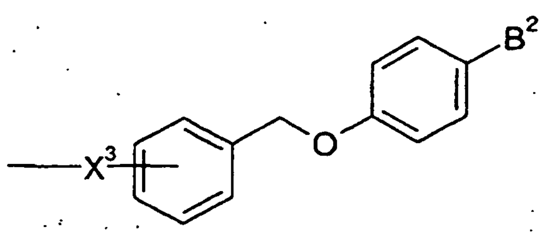

représente le groupement

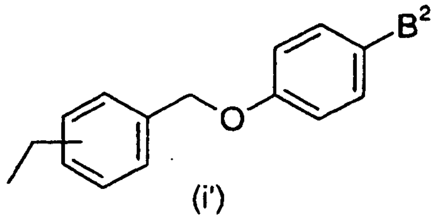

(i')

ou

A peut être en outre

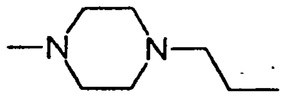

(xix)

quand le groupement des deux groupements

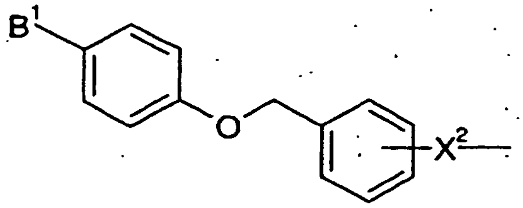

et

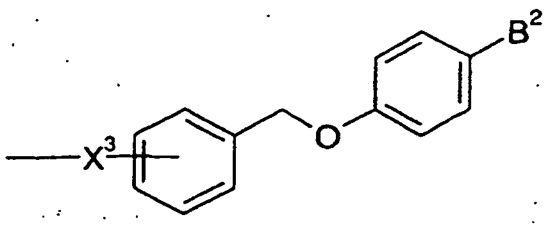

représente le groupement (i) ou (i'), qui est lié directement à l'azote de pipérazine du groupe A, éventuellement sous forme de leurs racémates, de leurs énantiomères, de leurs diastéréoisomères, de leurs tautomères et de leurs mélanges, et éventuellement de leurs sels d'addition d'acide phannacologiquement acceptables.

**7.** Composés de formule générale (IA) selon l'une des revendications 1 à 6, où le groupement

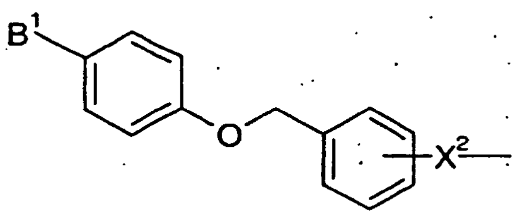

représente un groupement choisi dans le groupe consistant en

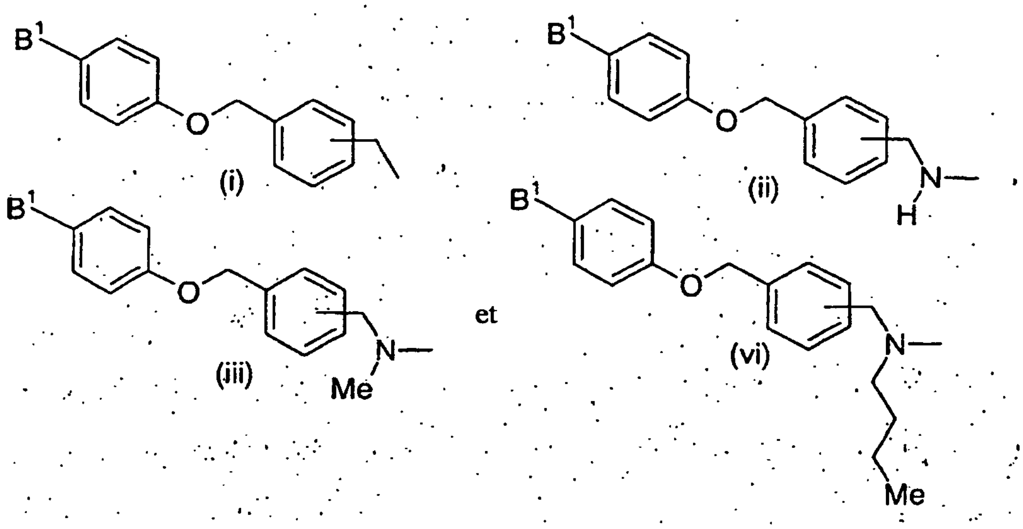

(i) (ii) (iii) et (vi)

le groupement

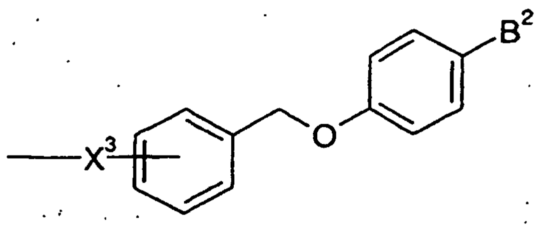

représente un groupement choisi dans le groupe consistant en

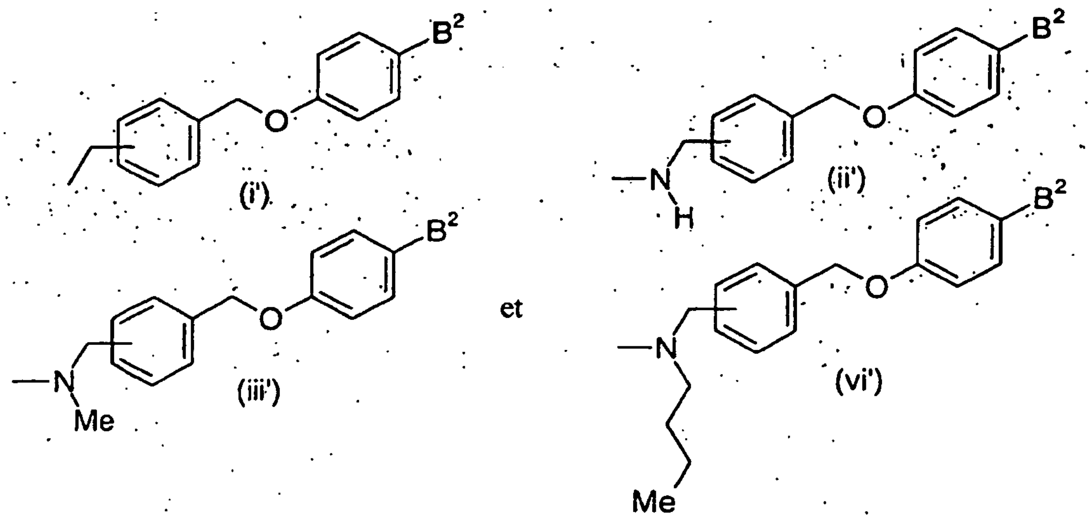

(i') (ii') (iii') et (vi')

où
$B^1$ et $B^2$, identiques ou différents, représentent $-C(=NR^1)-NR^{1'}H$ ou $-CH_2NH_2$;
$R^1$ et $R^{1'}$, identiques ou différents, représentent l'hydrogène ou OH ;
A représente un groupement de pontage qui est choisi dans le groupe consistant en (viii) $C_4$-$C_{10}$-alkylène,

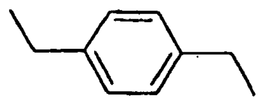
(ix)
,
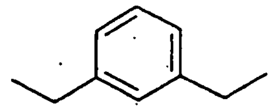
(x)
,

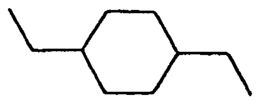
(xii)
,
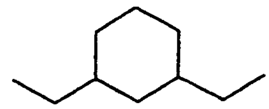
(xiii)

et

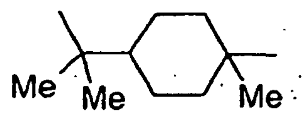
(xv)

ou
A représente en outre

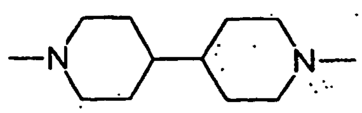
(xviii)

quand le groupement

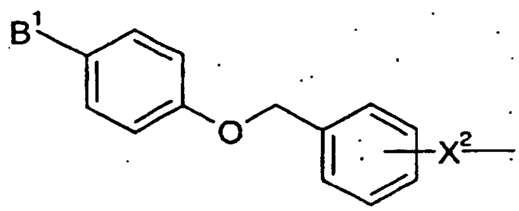

représente le groupe (i)
et le groupement

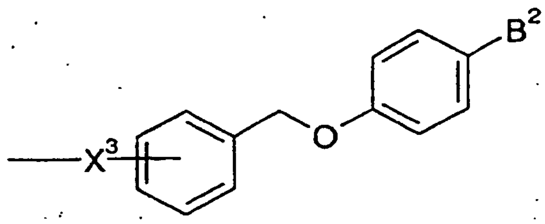

représente le groupement (i') éventuellement sous forme de leurs racémates, de leurs énantiomères, de leurs diastéréoisomères, de leurs tautomères et de leurs mélanges, et éventuellement de leurs sels d'addition d'acide pharmacologiquement acceptables.

**8.** Composés de formule générale (IA1)

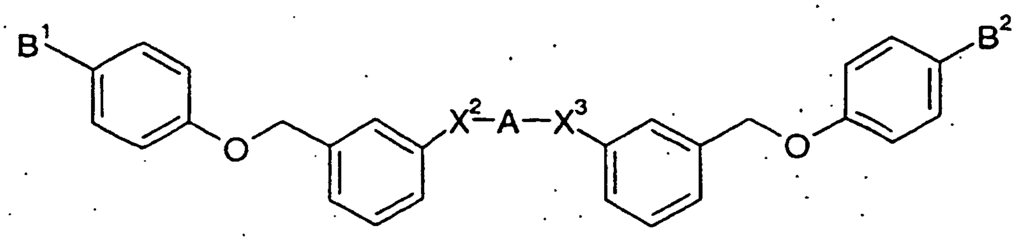

(IA1)

où $B^1$, $B^2$, A, $X^2$ et $X^3$ présentent les significations indiquées dans les revendications 1 à 7, éventuellement sous forme de leurs racémates, de leurs énantiomères, de leurs diastéréoisomères, de leurs tautomères et de leurs mélanges, et éventuellement de leurs sels d'addition d'acide pharmacologiquement acceptables.

**9.** Composés de formule générale (IA) ou (IA1) selon l'une des revendications 1 à 8, où $-X^2-A-X^3-$ représente un groupe choisi parmi les formules

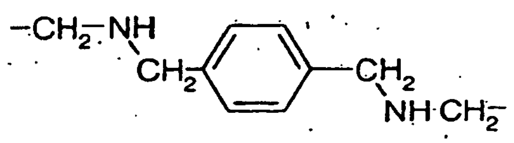

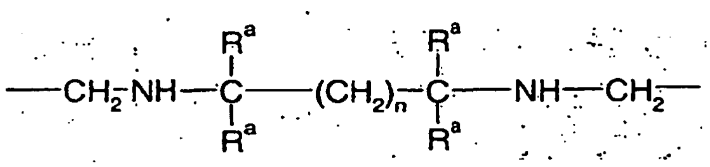

où Ra représente l'hydrogène ou méthyle et n est 3, 4, 5 ou 6,

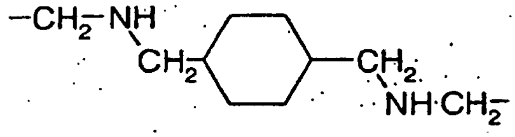

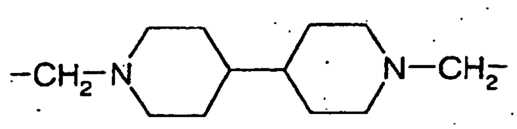

**10.** Composés de formule générale (IA) ou (IA1) selon l'une des revendications 1 à 9 choisis dans le groupe consistant en :

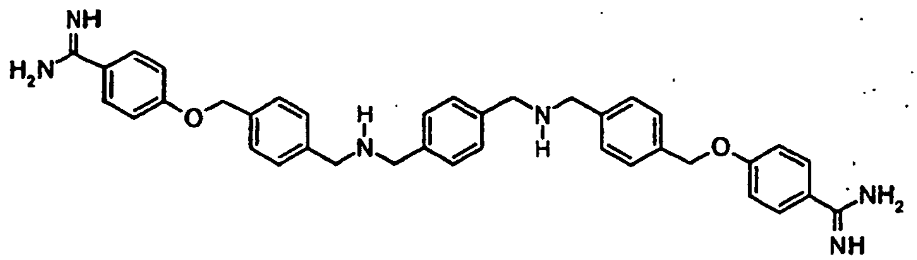

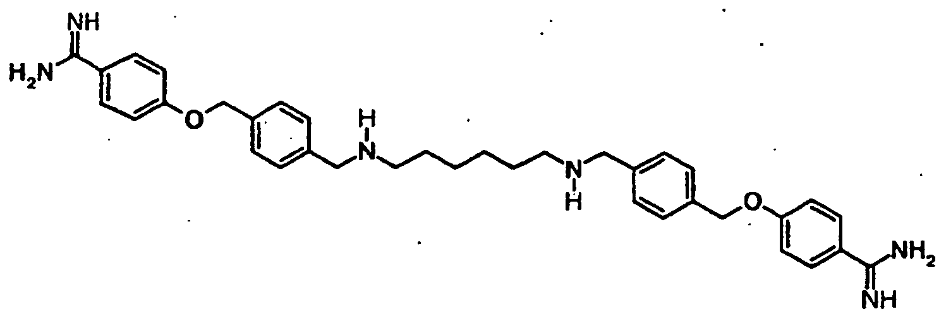

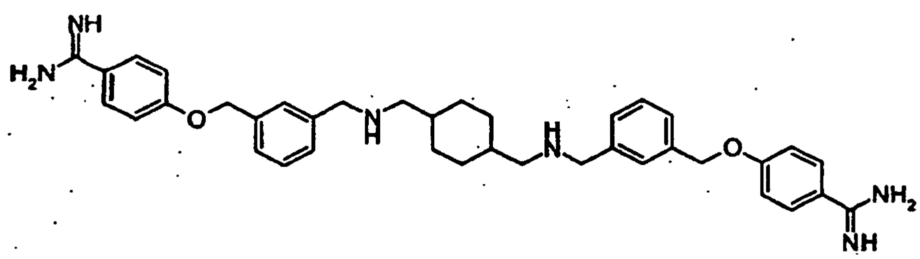

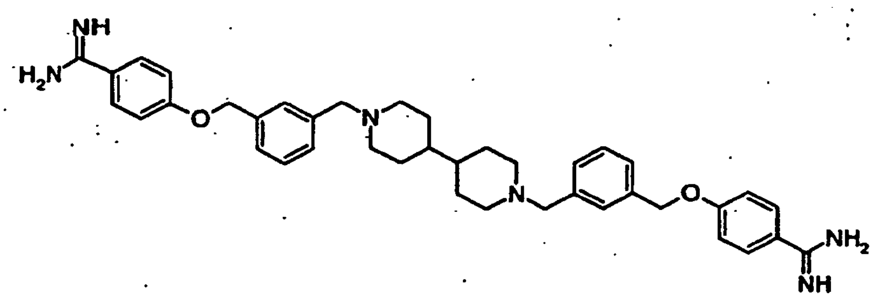

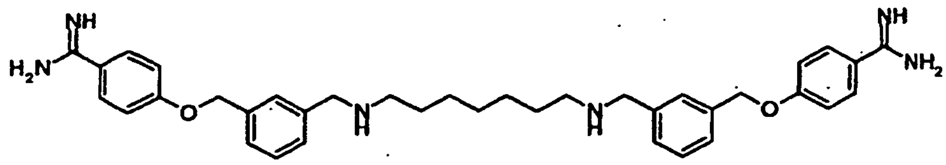

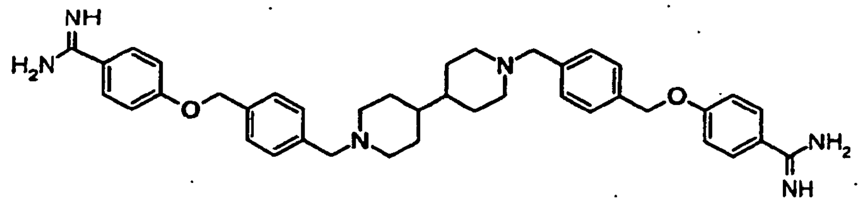

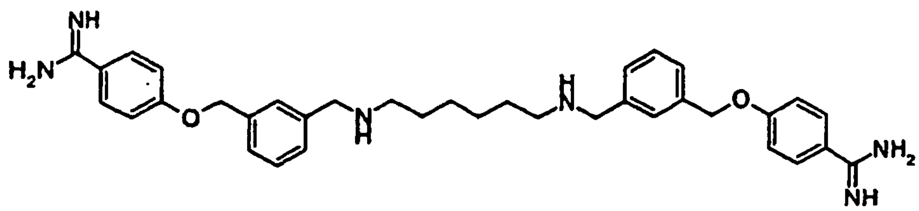

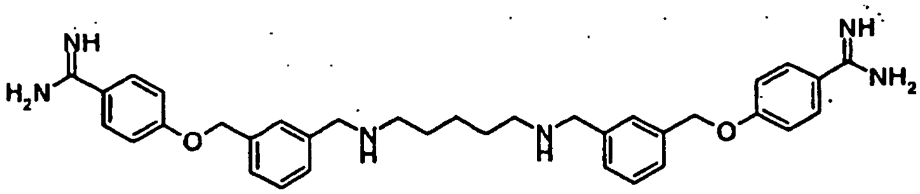

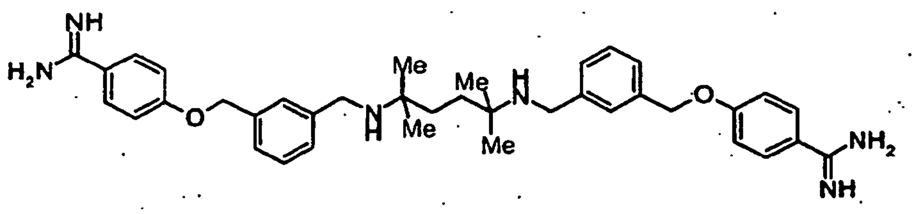

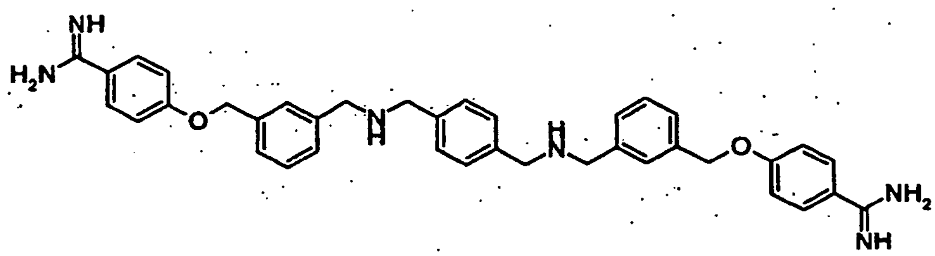

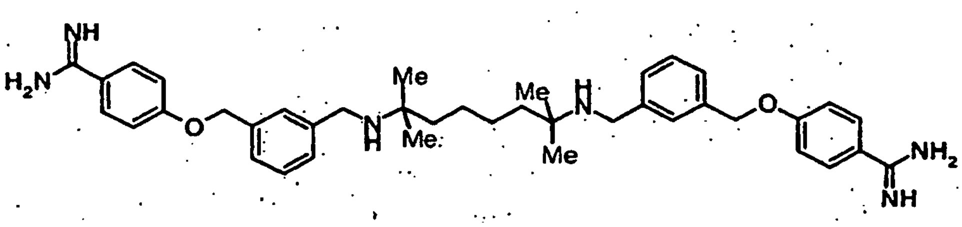

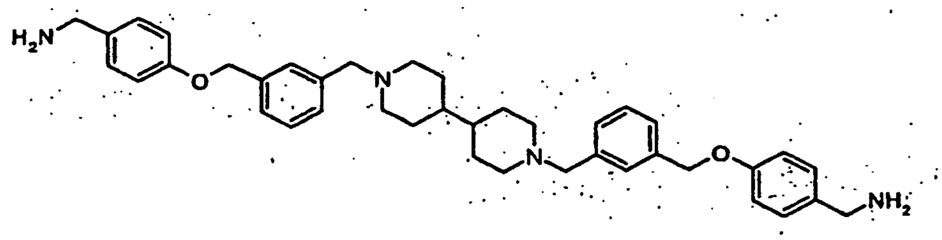

**11.** Utilisation de composés de formule générale (IA) ou (IA1) selon l'une des revendications 1-10 pour la production d'un médicament pour la prévention et/ou le traitement des maladies dans lesquelles les inhibiteurs de tryptase peuvent présenter une utilité thérapeutique.

**12.** Composition pharmaceutique **caractérisée par** une teneur en un ou plusieurs composés selon l'une des revendications 1-10.

**13.** Procédé de production des composés de formule générale (IA)

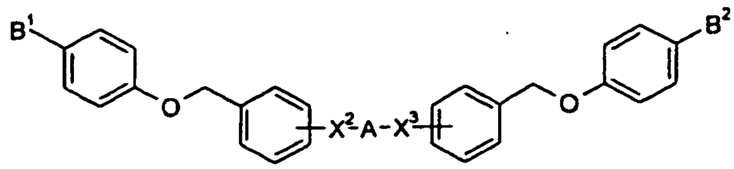

(IA)

dans lesquels $B^1$ et $B^2$ représentent -C(=NH)-$NH_2$ et les groupes $X^2$, A, $X^3$ peuvent présenter les significations citées dans les revendications 1-10, **caractérisé**

(a) en ce que l'on fait réagir avec l'ammoniac des imidoesters de formule générale (IIA)

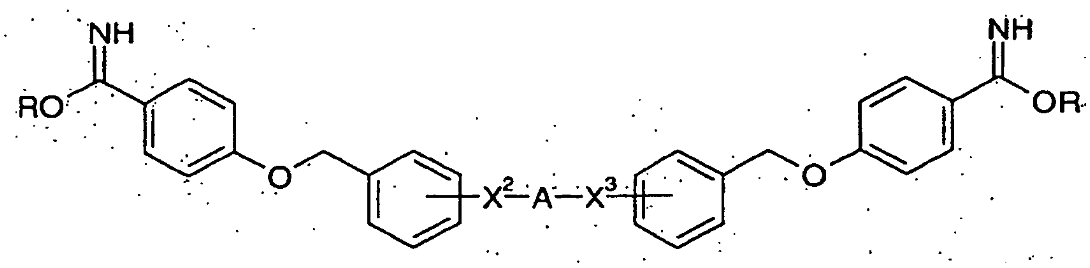

(IIA)

dans lesquels R représente $C_1$-$C_6$-alkyle et les groupes $X^2$, A, $X^3$ peuvent présenter les significations citées dans les revendications 1-10 ; ou
(b) en ce que l'on réduit des composés de formule générale (IA) dans lesquels $B^1$ et $B^2$ représentent -C(=NOH)-$NH_2$ et les groupes $X^2$, A, $X^3$ peuvent présenter les significations citées dans les revendications 1-10.

**14.** Procédé de production de composés de formule générale (IA)

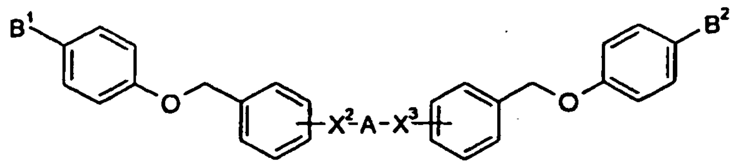

( IA )

dans lesquels $B^1$ et $B^2$ représentent -C(=NOH)-$NH_2$ et les groupes $X^2$, A, $X^3$ peuvent présenter les significations citées dans les revendications 1-10, **caractérisé en ce que** des composés de formule générale (IIIA)

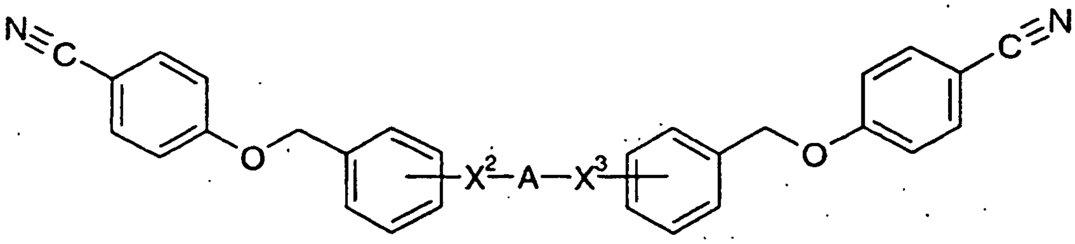

( IIIA )

dans lesquels les groupes $X^2$, A, $X^3$ peuvent présenter les significations citées dans les revendications 1-10, sont traités avec l'hydroxylamine.

**15.** Procédé de production des composés de formule générale (IA)

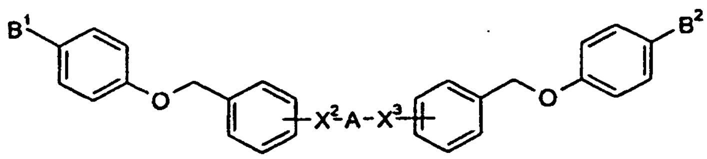

( IA )

(a) dans lesquels $B^1$ et $B^2$ et les groupes $X^2$, A, $X^3$ peuvent avoir les significations citées dans les revendications 1-10, **caractérisé en ce que** l'on fait réagir des composés des formules générales (IVA) et (VA)

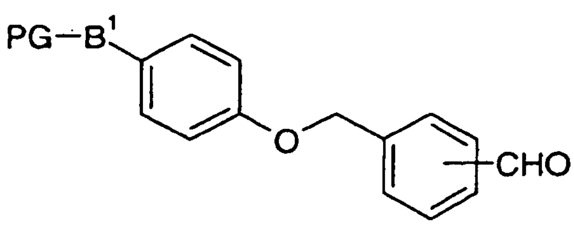

( IVA )

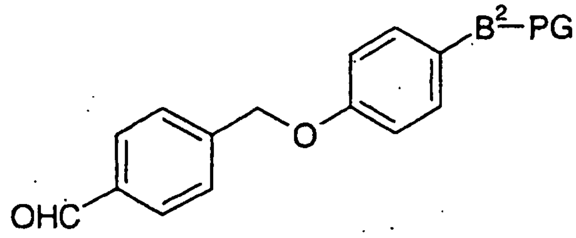

( VA )

dans lesquels PG peut représenter un groupe protecteur approprié pour la protection d'amines, avec une diamine avec déduction subséquente des doubles liaisons C=N- ainsi formées et enfin on clive les groupes protecteurs PG de la manière habituelle ; ou

(b) **en ce que** l'on fait réagir avec une diamine ou un dialcool des composés des formules générales (VIA) et (VIIA)

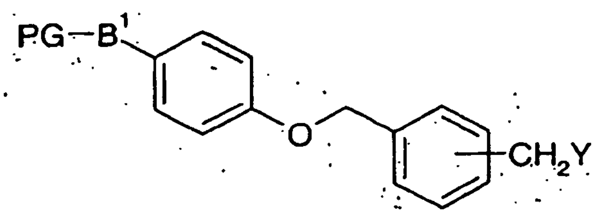

( VIA )

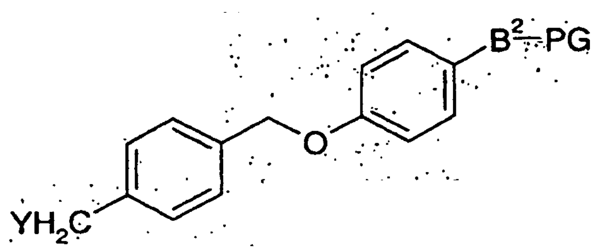

( VIIA )

dans lesquels PG peut représenter à chaque fois indépendamment de l'autre un groupe protecteur approprié pour la protection d'amines et Y représente à chaque fois indépendamment de l'autre un groupement choisi dans le groupe consistant en le fluor, le chlore, le brome, l'iode, $C_1$-$C_4$-alkyle et arylsulfonate et enfin on clive les groupes protecteurs PG de la manière habituelle.

**16.** Procédé de production des composés de formule générale (IA)

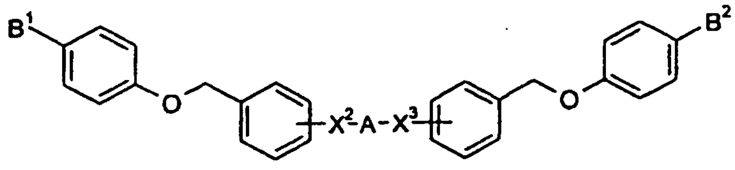

(IA)

dans lesquels $B^1$ et $B^2$ représentent -C(=NR$^1$)-NH$_2$ avec $R^1 \neq H$ et les groupes $X^2$, A, $X^3$ peuvent avoir les significations citées dans les revendications 1-10, **caractérisé en ce que** l'on fait réagir des composés de formule générale (IA) dans lesquels $B^1$ et $B^2$ représentent -C(=NH)-NH$_2$ avec des esters de l'acide chloroformique ou des halogénures d'acyle ou les anhydrides correspondants.

**17.** Procédé de production des composés de formule générale (IA)

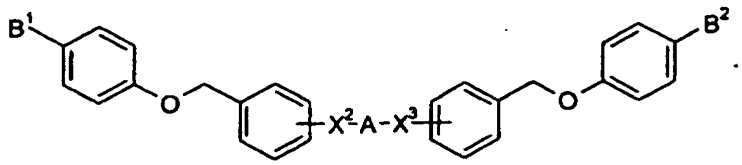

(IA)

dans lesquels $B^1$ et $B^2$ représentent -CH$_2$-NH$_2$ et les groupes $X^2$, A, $X^3$ peuvent avoir les significations citées dans les revendications 1-10, **caractérisé en ce que** l'on réduit les composés nitriles correspondants de formule générale (III)

$$NC\text{-}Ar^1\text{-}X^1\text{-}Ar^2\text{-}X^2A\text{-}X^3\text{-}Ar^3\text{-}X^4\text{-}Ar^4\text{-}CN \qquad \text{(III)}$$

où
$Ar^1$, $Ar^2$, $Ar^3$ et $Ar^4$ représentent phényle ;
-$X^1$- représente -O-CH$_2$- ;
-$X^4$- représente -CH$_2$-O-.